(19) Europäisches Patentamt
European Patent Office
Office européen des brevets

(11) EP 4 212 524 A1

(12) EUROPEAN PATENT APPLICATION
published in accordance with Art. 153(4) EPC

(43) Date of publication:
**19.07.2023 Bulletin 2023/29**

(21) Application number: **21865940.7**

(22) Date of filing: **06.09.2021**

(51) International Patent Classification (IPC):
***C07D 403/04*** (2006.01) ***A61K 31/513*** (2006.01)
***A61P 35/00*** (2006.01)

(52) Cooperative Patent Classification (CPC):
**A61K 31/513; A61P 35/00; C07D 403/04**

(86) International application number:
**PCT/CN2021/116696**

(87) International publication number:
**WO 2022/052886 (17.03.2022 Gazette 2022/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.09.2020 CN 202010934821**
**03.12.2020 CN 202011396364**
**08.02.2021 CN 202110172897**

(71) Applicant: **Betta Pharmaceuticals Co., Ltd**
**Yuhang**
**Hangzhou**
**Zhejiang 311100 (CN)**

(72) Inventors:
- **WU, Hao**
  **Hangzhou, Zhejiang 311100 (CN)**
- **YANG, Xiaofeng**
  **Hangzhou, Zhejiang 311100 (CN)**
- **LIU, Qisheng**
  **Hangzhou, Zhejiang 311100 (CN)**
- **HAN, Han**
  **Hangzhou, Zhejiang 311100 (CN)**
- **LI, Jinhua**
  **Hangzhou, Zhejiang 311100 (CN)**
- **LI, Yang**
  **Hangzhou, Zhejiang 311100 (CN)**
- **JIANG, Feng**
  **Hangzhou, Zhejiang 311100 (CN)**
- **KUANG, Cuiwen**
  **Hangzhou, Zhejiang 311100 (CN)**
- **XIA, Hongfeng**
  **Hangzhou, Zhejiang 311100 (CN)**
- **ZHANG, Hongbo**
  **Hangzhou, Zhejiang 311100 (CN)**
- **LAN, Hong**
  **Beijing 100176 (CN)**
- **WANG, Jiabing**
  **Beijing 100176 (CN)**
- **DING, Lieming**
  **Hangzhou, Zhejiang 311100 (CN)**

(74) Representative: **Graf von Stosch Patentanwaltsgesellschaft mbH**
**Prinzregentenstraße 22**
**80538 München (DE)**

(54) **CD73 INHIBITOR AND APPLICATION THEREOF IN MEDICINE**

(57) The present invention relates to a novel compound, which has cancer therapeutic activity. The present invention also relates to a preparation method for the compound and a pharmaceutical composition comprising the compound.

EP 4 212 524 A1

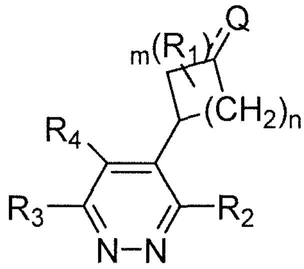

(I)

## Description

### Technical field

The present invention relates to a CD73 inhibitor, which has cancer therapeutic activity. The invention also relates to a method for preparing these compounds and a pharmaceutical composition comprising them.

### Background technique

Ecto-5'-nucleotidase (CD73) is a cytoplasmic glycoprotein that is present on the surface of cell membranes of various cell types, including endothelial cells, lymphocytes, stromal cells, and tumor cells. CD73 can induce extracellular 5'-AMP (5'-AMP) to produce adenosine, which induces immunosuppressive effects and promotes tumor proliferation and/or metastasis. In addition, CD73 also promotes tumorigenesis through non-immune-related mechanisms, such as tumor angiogenesis and tumor cell adhesion to extracellular matrix proteins. Clinically, high levels of CD73 expression are associated with lymph node metastasis and poor prognosis of multiple cancer types, and CD73 has been found to be an independent prognostic factor in patients with prostate cancer and triple-negative breast cancer.

Drugs targeting CD73 have become one of the hot areas of drug research and development, and some varieties have entered the clinical stage. The varieties under development include both large - molecule antibodies and small - molecule drugs. The invention provides a novel structure of small molecule CD73 inhibitor with good antitumor activity.

### Summary

The present invention provides a compound having formula (I), its tautomer, deuterated compound,or pharmaceutically acceptable salt thereof, wherein;

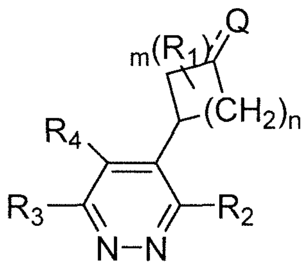

(I)

Q is chain or cyclic unsaturated group, Q is $=CH_2$, $C_{2-6}$ alkenyl, $C_{2-6}$ alkynyl, or ring A; the $=CH_2$ is optionally substituted by one or more $R_5$; the $C_{2-6}$ alkenyl or $C_{2-6}$ alkynyl is optionally substituted by 1 or more $R_5$ or ring A;
Ring A is $C_{6-14}$ aryl or 5-14 heteraryl; the $C_{6-14}$ aryl or the 5-14 heteraryl is optionally substituted by one or more $R_6$;
$R_1$ is independently H, halogen, cyano, $C_{1-3}$ alkyl, $C_{1-3}$ alkoxy or $C_{1-3}$ haloalkyl, preferably H;
$R_2$ is H, halogen, cyano, substituted or unsubstituted $C_{1-3}$ alkyl, substituted or unsubstituted $C_{1-3}$ alkoxy, substituted or unsubstituted $C_{2-4}$ alkenyl, substituted or unsubstituted $C_{2-4}$ alkynyl;
$R_3$ is

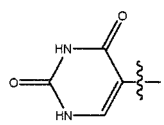

;

$R_4$ is H, halogen, or methyl;
$R_5$ is independently H, cyano, halogen, $C_{1-6}$ alkyl, $C_{1-6}$ haloalkyl, $-C_{0-6}$ alkylene $-OR_a$, $-C_{0-6}$ alkylene $-OC(O)N(R_a)_2$, $-C_{0-6}$ alkylene $-N(R_a)_2$, $-C_{0-6}$ alkylene $-NR_aC(O)R_a$, $-C_{0-6}$ alkylene - $NR_aC(O)R_a$, $-C_{0-6}$ alkylene $-NR_aC(O)N(R_a)_2$, $-C_{0-6}$ alkylene $-NR_aS(O)R_a$, $-C_{0-6}$ alkylene - $NR_aS(O)_2R_a$, $-C_{0-6}$ alkylene $-S(=O)R_a$,- $C_{0-6}$ alkylene $-S(=O)_2R_a$, - $C_{0-6}$ alkylene $-SR_a$, - $C_{0-6}$ alkylene $-S(R_a)_5$, - $C_{0-6}$ alkylene $-C(=O)R_a$, - $C_{0-6}$ alkylene $-C(=O)OR_a$, - $C_{0-6}$ alkylene $-C(=O)N(R_a)_2$ , $-C_{0-6}$ alkylene $-C_{3-14}$ cycloalkyl or $-C_{0-6}$ alkylene -(3-14) heterocyclic, the $C_{1-6}$ alkyl, - $C_{0-6}$ alkylene $-C_{3-14}$ cycloalkyl or $-C_{0-6}$ alkylene -(3-14 heterocyclic) optionally substituted by one or more $R_a$;

$R_6$ is H, cyano, halogen, $C_{1-6}$ alkyl, $C_{1-6}$ halogen alkyl, -$C_{0-6}$ alkylene-$OR_a$, -$C_{0-6}$ alkylene-OC(O)N $(R_a)_2$, -$C_{0-6}$ alkylene-N $(R_a)_2$, -$C_{0-6}$ alkylene-$NR_aC(O)R_a$, -$C_{0-6}$ alkylene-$NR_aC(O)N$ $(R_a)_2$, -$C_{0-6}$ alkylene-$NR_aS(O)R_a$, -$C_{0-6}$ alkylene-$NR_aS(O)_2R_a$, -$C_{0-6}$ alkylene-S(=O)$R_a$, -$C_{0-6}$ alkylene-S(=O)$_2R_a$, -$C_{0-6}$ alkylene-$SR_a$, -$C_{0-6}$ alkylene-S$(R_a)_5$, -$C_{0-6}$ alkylene-C(=O)$R_a$, -$C_{0-6}$ alkylene-C(=O)$OR_a$, -$C_{0-6}$ alkylene-C(=O)N $(R_a)_2$, $C_{2-6}$ alkenyl, $C_{2-6}$ alkynyl, -$C_{0-6}$ alkylene-$C_{3-14}$ cycloalkyl, -$C_{0-6}$ alkylene(3-14 heterocyclic) , -$C_{0-6}$ alkylene-$C_{6-14}$ aryl or -$C_{0-6}$ alkylene(5-14 heteroaryl) , the $C_{1-6}$ alkyl, $C_{2-6}$ alkenyl, $C_{2-6}$ alkynyl, -$C_{0-6}$ alkylene-$C_{3-14}$ cycloalkyl, -$C_{0-6}$ alkylene(3-14 heterocyclic) , -$C_{0-6}$ alkylene-$C_{6-14}$ aryl or -$C_{0-6}$ alkylene(5-14 heteroaryl) optionally substituted by one or more $R_a$;

Each $R_a$ is independently H, halogen, hydroxyl, amino, oxo, nitro, cyano, carboxyl, $C_{1-6}$ alkyl, $C_{1-6}$ hydroxyalkyl, $C_{1-6}$ aminoalkyl, $C_{1-6}$ haloalkyl, $C_{1-6}$ alkoxy, $C_{1-6}$ haloalkoxy, $C_{1-6}$ heteroalkyl, $C_{3-8}$ cycloalkyl, 3-8 heterocyclic, $C_{6-14}$ aryl or 5-14 heteroaryl;

m is 0, 1, 2, 3, or 4;

n is 0 or 1;

------- is single bond or double bond.

The present invention provides a compound having formula (IA) or (IA-1) , it's tautomer, deuterated compound,or pharmaceutically acceptable salt thereof,

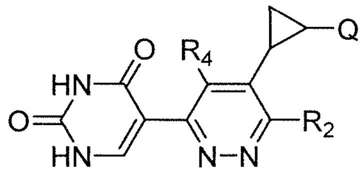

(IA)

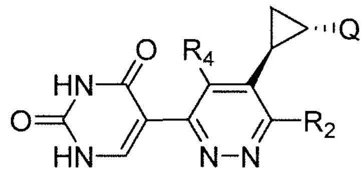

(IA-1)

wherein;

Q is

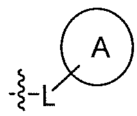

;

L is bond, $C_{2-6}$ alkenyl or $C_{2-6}$ alkynyl; the $C_{2-6}$ alkenyl or $C_{2-6}$ alkynyl is optionally substituted by one or more $R_5$;

$R_5$ is independently H, cyano, halogen, $C_{1-6}$ alkyl, $C_{1-6}$ haloalkyl, -$C_{0-6}$ alkylene -$OR_a$, -$C_{0-6}$ alkylene -OC(O)N $(R_a)_2$, -$C_{0-6}$ alkylene -N $(R_a)_2$, -$C_{0-6}$ alkylene -$NR_aC(O)R_a$, -$C_{0-6}$ alkylene - $NR_aC(O)N(R_a)_2$, -$C_{0-6}$ alkylene -$NR_aS(O)R_a$, -$C_{0-6}$ alkylene -$NR_aS(O)_2R_a$, -$C_{0-6}$ alkylene -S (=O)$R_a$,- $C_{0-6}$ alkylene -S (= O)$_2R_a$, - $C_{0-6}$ alkylene -$SR_a$, - $C_{0-6}$ alkylene -S$(R_a)_5$, - $C_{0-6}$ alkylene - C(=O)$R_a$, - $C_{0-6}$ alkylene -C(=O)$OR_a$, - $C_{0-6}$ alkylene -C(=O)N $(R_a)_2$ , -$C_{0-6}$ alkylene -$C_{3-14}$ cycloalkyl or -$C_{0-6}$ alkylene -(3-14 heterocyclic), the $C_{1-6}$ alkylene, -$C_{0-6}$ alkylene -$C_{3-14}$ cycloalkyl or -$C_{0-6}$ alkylene -(3-14 heterocyclic) optionally substituted by one or more $R_a$;

Ring A is $C_{6-14}$ aryl or 5-14 heteroaryl, and the $C_{6-14}$ aryl or 5-14 heteroaryl is optionally substituted by one or more $R_6$;

$R_6$ is H, cyano, halogen, $C_{1-6}$ alkyl, $C_{1-6}$ halogen alkyl, -$C_{0-6}$ alkylene-$OR_a$,-$C_{0-6}$ alkylene-OC(O)N $(R_a)_2$,-$C_{0-6}$ alkylene-N $(R_a)_2$,-$C_{0-6}$ alkylene-$NR_aC(O)R_a$,-$C_{0-6}$ alkylene-$NR_aC(O)N$ $(R_a)_2$,-$C_{0-6}$ alkylene-$NR_aS(O)R_a$,-$C_{0-6}$ alkylene-$NR_aS(O)_2R_a$,-$C_{0-6}$ alkylene-S(=O)$R_a$,-$C_{0-6}$ alkylene-S(=O)$_2R_a$,-$C_{0-6}$ alkylene-$SR_a$,-$C_{0-6}$ alkylene-S$(R_a)_5$,-$C_{0-6}$ alkylene-C(=O)$R_a$,-$C_{0-6}$ alkylene-C(=O)$OR_a$,-$C_{0-6}$ alkylene-C(=O)N $(R_a)_2$, $C_{2-6}$ alkenyl ,$C_{2-6}$ alkynyl, -$C_{0-6}$ alkylene-$C_{3-14}$ cycloalkyl,-$C_{0-6}$ alkylene(3-14 heterocyclic),-$C_{0-6}$ alkylene-$C_{6-14}$ aryl or -$C_{0-6}$ alkylene(5-14 heteroaryl),the $C_{1-6}$ alkyl, $C_{2-6}$ alkenyl ,$C_{2-6}$ alkynyl, -$C_{0-6}$ alkylene-$C_{3-14}$ cycloalkyl,-$C_{0-6}$ alkylene(3-14 heterocyclic),-$C_{0-6}$ alkylene-$C_{6-14}$ aryl or -$C_{0-6}$ alkylene(5-14 heteroaryl) optionally substituted by one or more $R_a$;

Each $R_a$ is independently H, halogen, hydroxyl, amino, oxo, nitro, cyano, carboxyl, $C_{1-6}$ alkyl, $C_{1-6}$ hydroxyalkyl, $C_{1-6}$ aminoalkyl, $C_{1-6}$ haloalkyl, $C_{1-6}$ alkoxy, $C_{1-6}$ haloalkoxy, $C_{1-6}$ heteroalkyl, $C_{3-8}$ cycloalkyl, 3-8 heterocycle, $C_{6-14}$ aryl, or 5-14 heteroaryl;

$R_2$ is H, halogen, cyano, $C_{1-3}$ alkyl, $C_{1-3}$ alkoxy, $C_{1-3}$ haloalkoxy, $C_{2-4}$ alkenyl or $C_{2-4}$ alkynyl;

$R_4$ is H, halogen, or methyl.

In certain embodiments, wherein; L is bond, ethylene or acetylene, the ethylene or acetylene is optionally

substituted by one or more Rs;preferably L is bond, ethylene or acetylene, and preferably L is a bond.

In certain embodiments, wherein; $R_5$ is independently H, cyano, halogen, $C_{1-6}$ alkyl, $C_{1-6}$ haloalkyl, $-OR_a$, $-OC(O)N(R_a)_2$, $-N(R_a)_2$, $-NR_aC(O)R_a$, $-NR_aC(O)N(R_a)_2$, $-NR_aS(O)R_a$, $-NR_aS(O)_2R_a$, $-S(=O)R_a$, $-S(=O)_2R_a$, $-SR_a$, $-S(R_a)_5$, $-C(=O)R_a$, $-C(=O)OR_a$, $-C(=O)N(R_a)_2$, $-C_{3-14}$ cycloalkyl or -(3-14) heterocyclic, each $R_a$ is independently H, $C_{1-6}$ alkyl, $C_{1-6}$ haloalkyl, $C_{1-6}$ heteroalkyl, $C_{3-8}$ cycloalkyl, 3-8 heterocyclic, $C_{6-14}$ aryl or 5-14 heteroaryl; preferably $R_5$ is independently H, halogen, cyano, amino, hydroxyl, $C_{1-6}$ alkyl, $C_{1-6}$ alkoxy, $C_{1-6}$ haloalkyl, $C_{1-6}$ haloalkoxy, $-S-C_{1-6}$ alkyl, $C_{3-8}$ cycloalkyl or 3-8 heterocyclic.

In certain embodiments, wherein; Ring A is

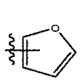, 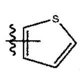, 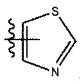, 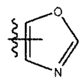, 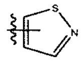,

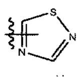, 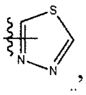, 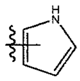, 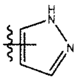, 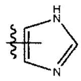, 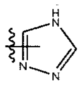, 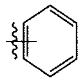, 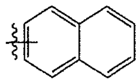, 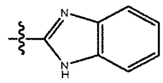, 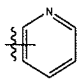, 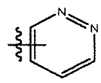,

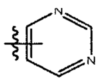 or 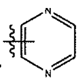,

Ring A optionally substituted by one or more $R_6$, Preferably ring A is phenyl or pyridine.

In certain embodiments, wherein; $R_6$ is H, cyano, halogen, $C_{1-6}$ alkyl, $C_{1-6}$ halogen alkyl, - ORa, -OC(O)N(Ra)$_2$, -N(Ra)$_2$, $-NR_aC(O)R_a$, $-NR_aC(O)N(R_a)_2$, $-NR_aS(O)R_a$, $-NR_aS(O)_2R_a$, $-S(=O)R_a$, -S(=O)2R$_a$, $-SR_a$, $-S(R_a)_5$, $-C(=O)R_a$, $-C(=O)OR_a$, $-C(=O)N(R_a)_2$, $C_{2-6}$ alkenyl, $C_{2-6}$ alkynyl, $-C_{3-14}$ cycloalkyl, 3-14 heterocyclic, $-C_{6-14}$ aryl or - 5-14 heteroaryl, each $R_a$ is independently H, $C_{1-6}$ alkyl, $C_{1-6}$ heteroalkyl, $C_{3-8}$ cycloalkyl, 3-8 heterocyclic, $C_{6-14}$ aryl or 5-14 heteraryl; Preferably $R_6$ is H, halogen, cyano, $C_{1-3}$ alkyl, $C_{1-3}$ alkoxy, $C_{1-3}$ haloalkyl or -CHO, more preferably $R_6$ is H, fluorine, chlorine, cyano, methoxy or methyl.

The invention provides a compound, its tautomer, deuterium substitute or pharmaceutical salt, which is selected from the compound of formula (IB) or formula (IB-1):

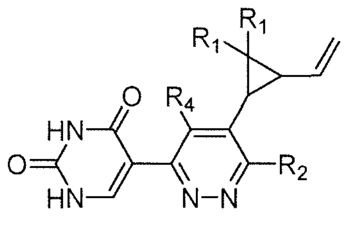

(IB)

or

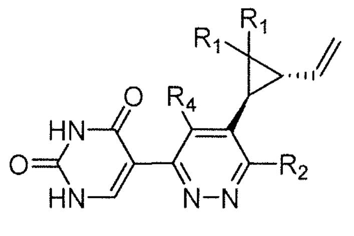

(IB-1)

wherein;

$R_1$, $R_2$ and $R_4$ are defined in formula(I).

In certain embodiments, wherein; $R_2$ is H, halogen, cyano, $C_{1-3}$ alkyl, $C_{1-3}$ alkoxy or ethylene; preferably $R_2$ is H, chlorine, cyano, methyl or methoxy or ethylene ; more preferably $R_2$ is chlorine, cyano, methyl or methoxy .

In certain embodiments, wherein; $R_4$ is H.

A compound, it's tautomer, deuterated compound or pharmaceutically acceptable salt, wherein the compound is

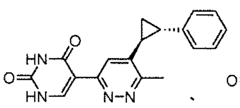 、 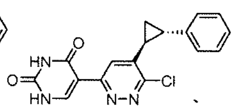 、 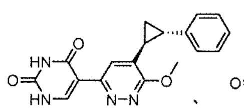 、 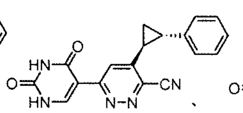 、 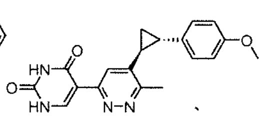 、

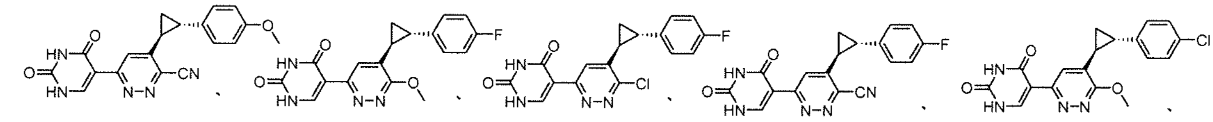
、 、 、 、 、

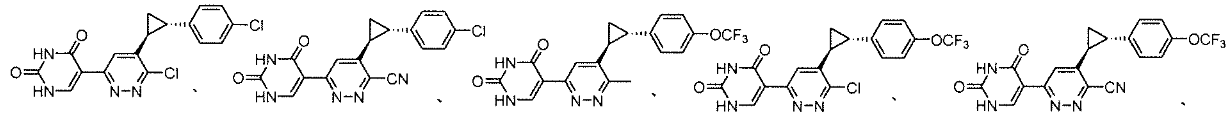
、 、 、 、 、

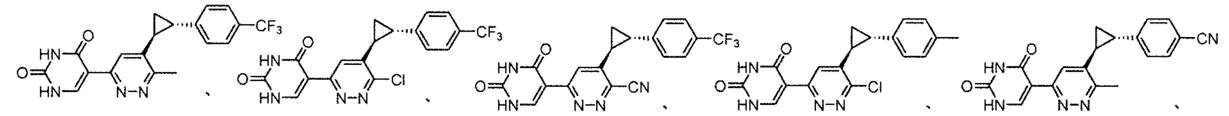
、 、 、 、 、

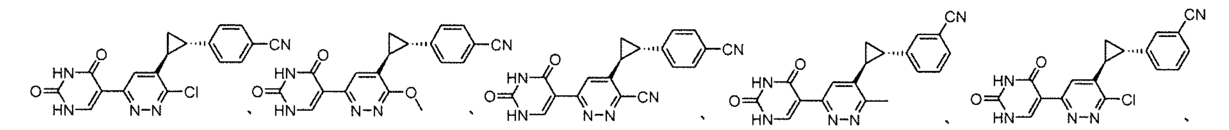
、 、 、 、 、

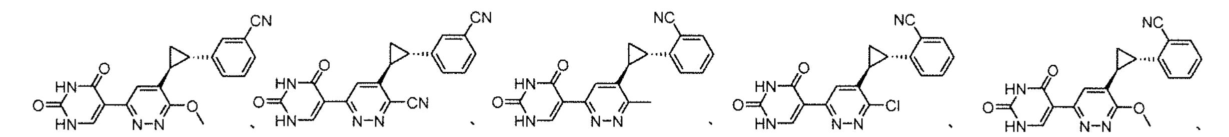
、 、 、 、 、

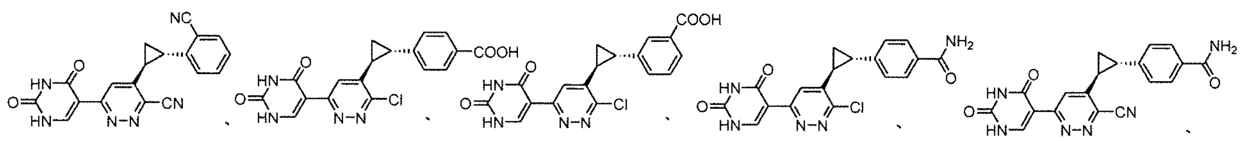
、 、 、 、 、

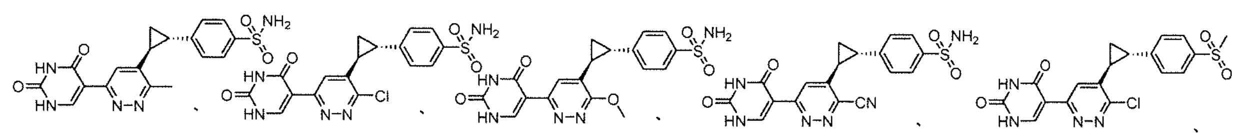
、 、 、 、 、

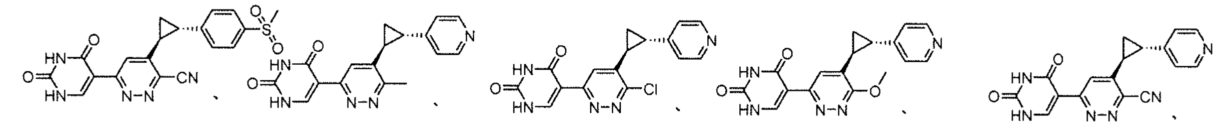
、 、 、 、 、

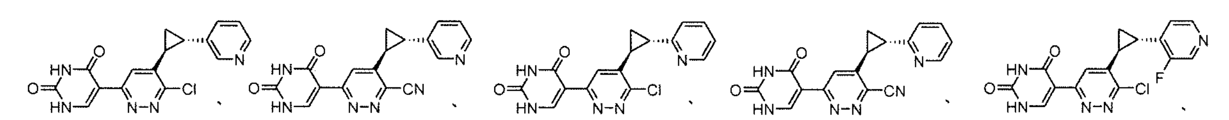
、 、 、 、 、

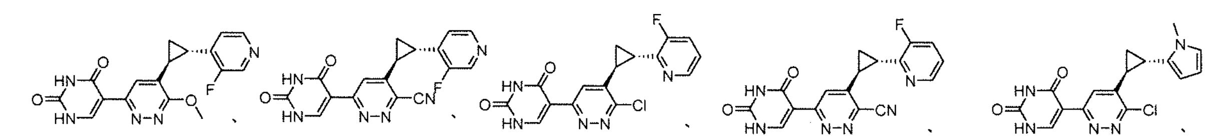
、 、 、 、 、

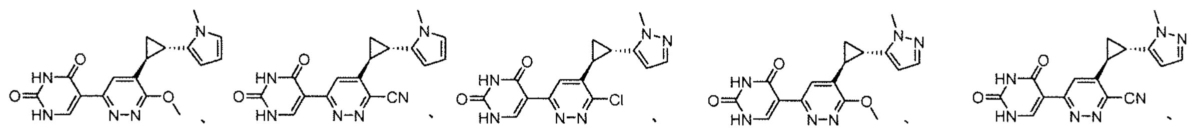 、 、 、 、 、

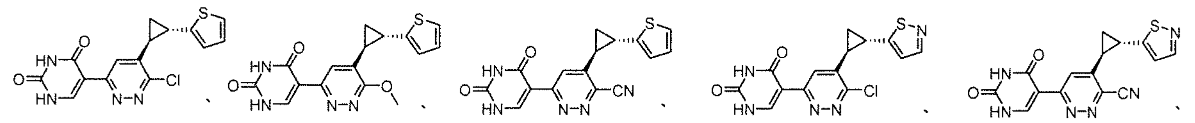 、 、 、 、 、

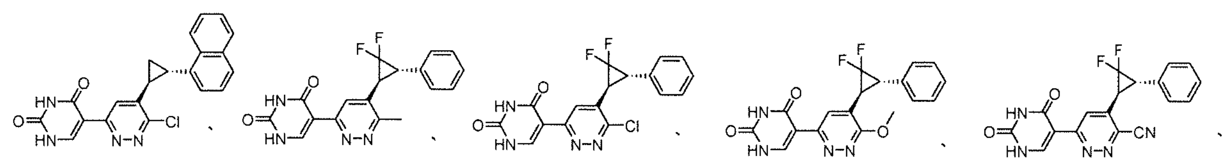 、 、 、 、 、

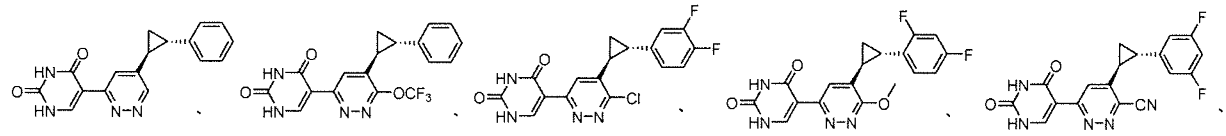 、 、 、 、 、

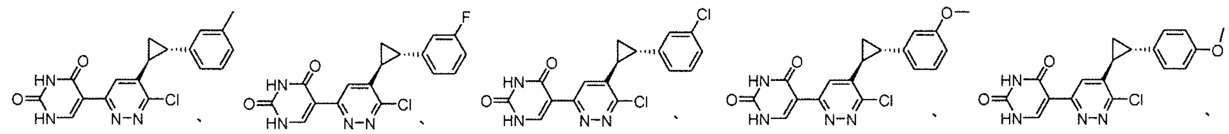 、 、 、 、 、

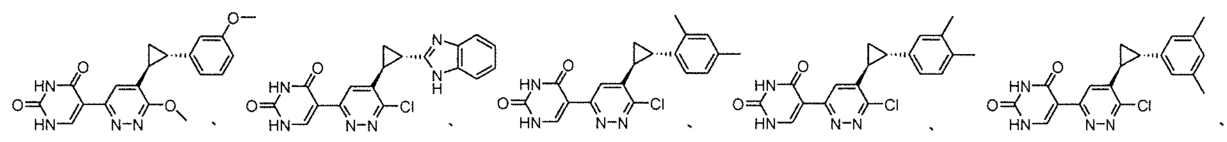 、 、 、 、 、

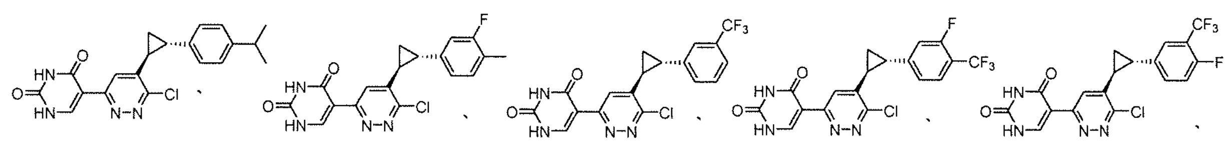 、 、 、 、 、

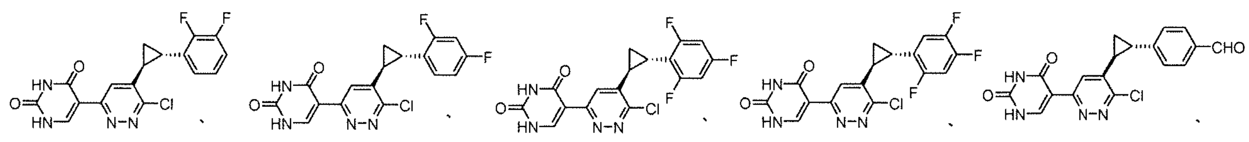 、 、 、 、 、

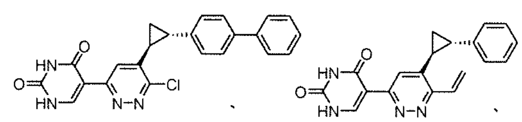 、 、

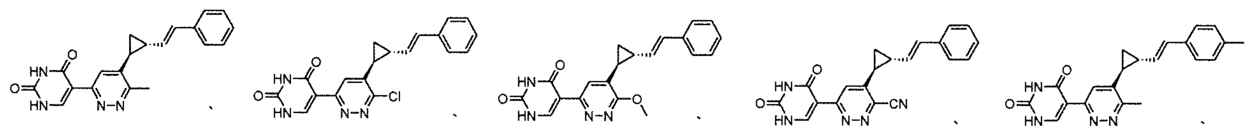 、 、 、 、 、

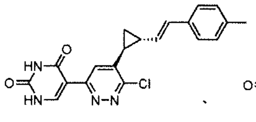,
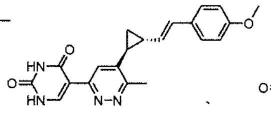,
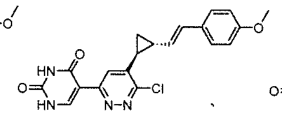,
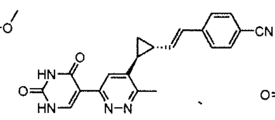,
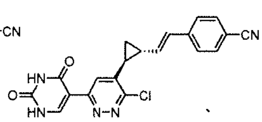,
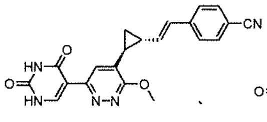,
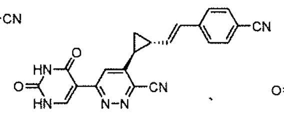,
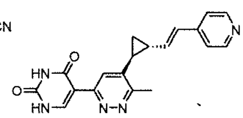,
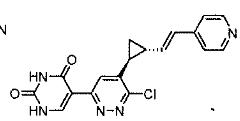,
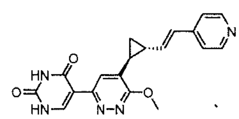,
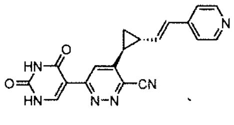,
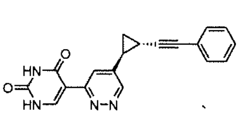,
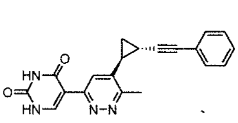,
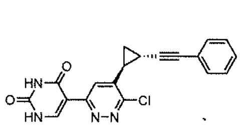,
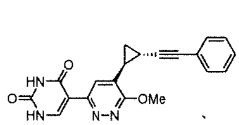,
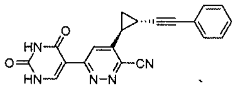,
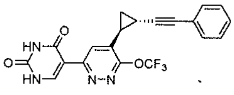,
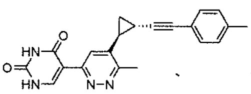,
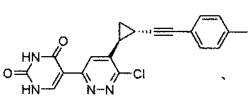,
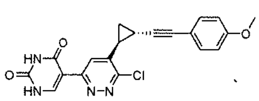,
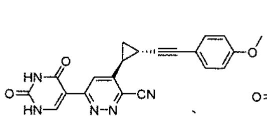,
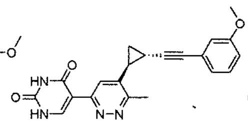,
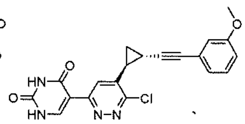,
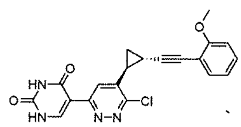,
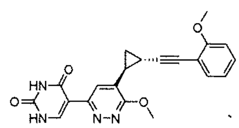,
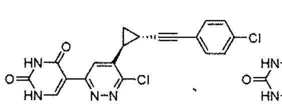,
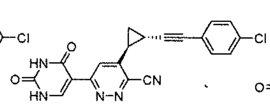,
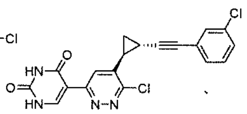,
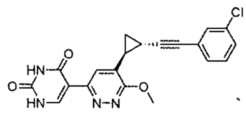,
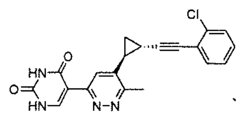,
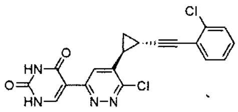,
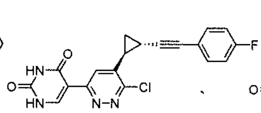,
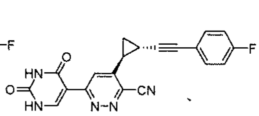,
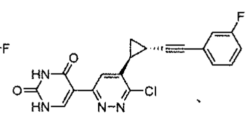,
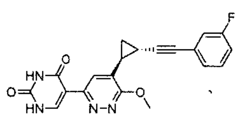,
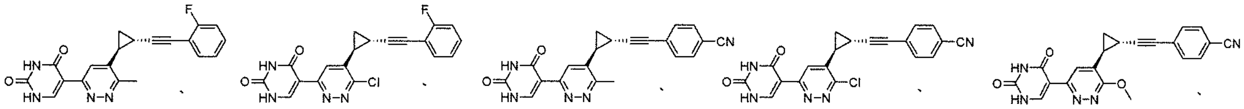
, , , , ,
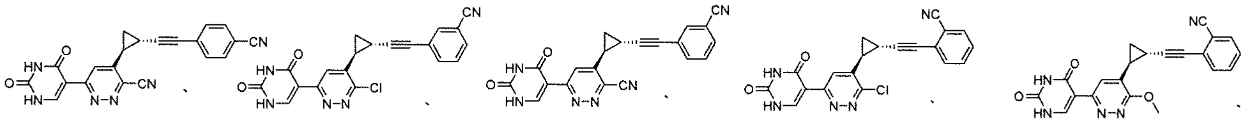
, , , , ,
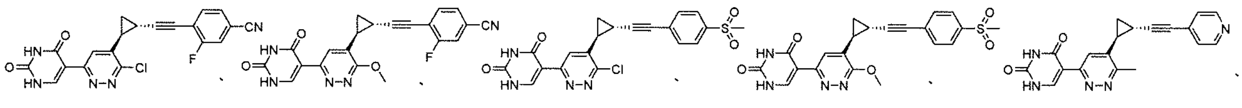
, , , , ,
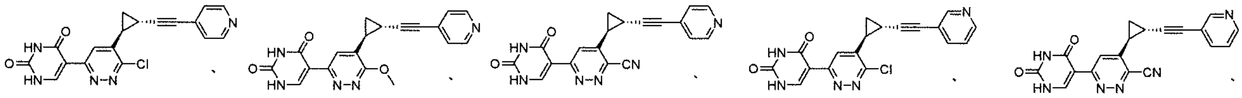
, , , , ,

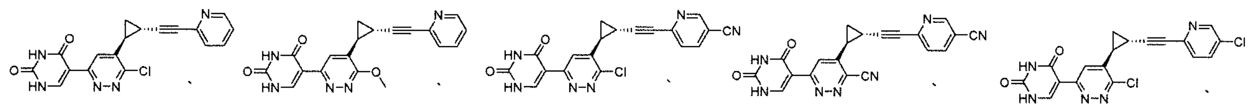

, , , , ,

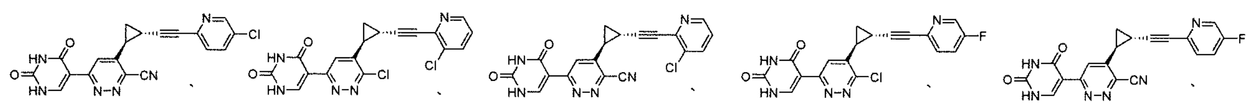

, , , , ,

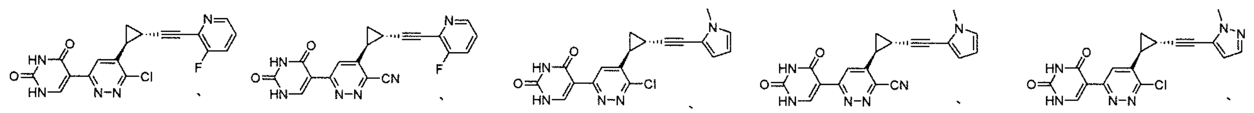

, , , , ,

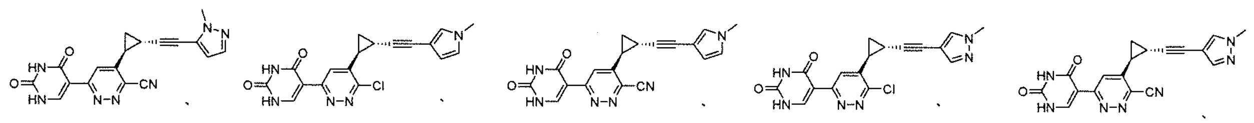

, , , , ,

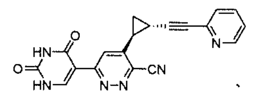

,

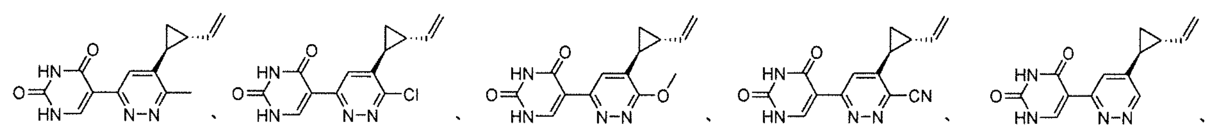

, , , , ,

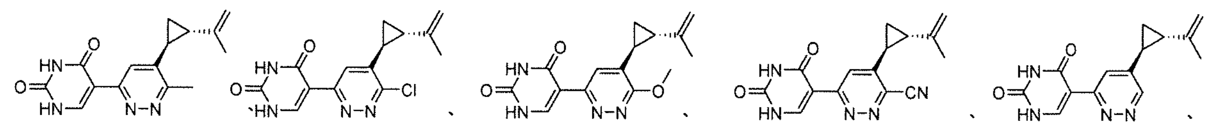

, , , , ,

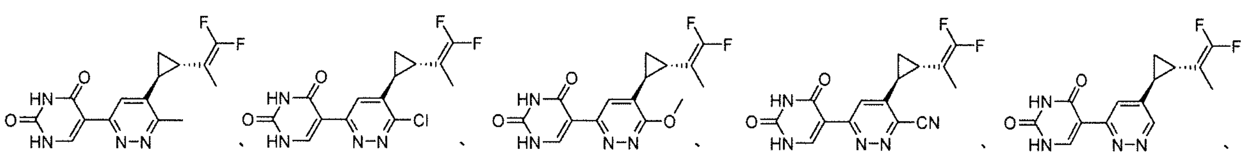

, , , , ,

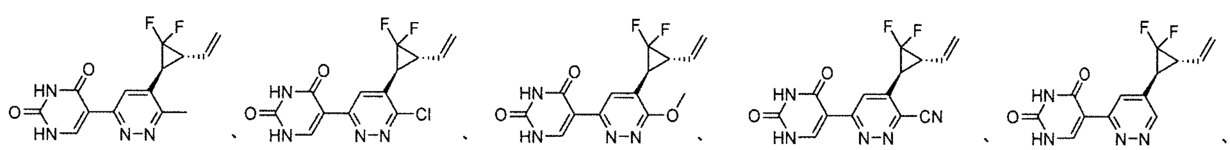

, , , , ,

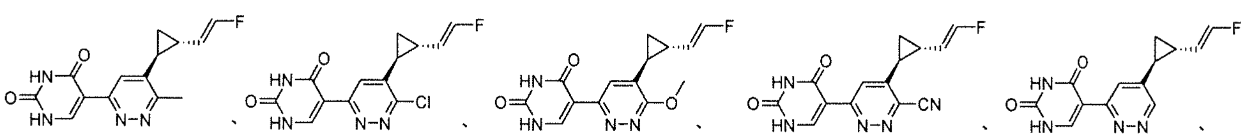

, , , , ,

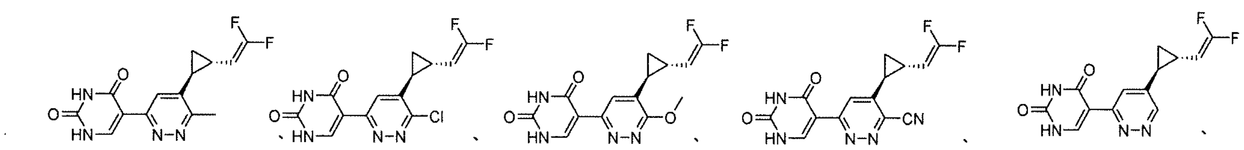

, , , , ,

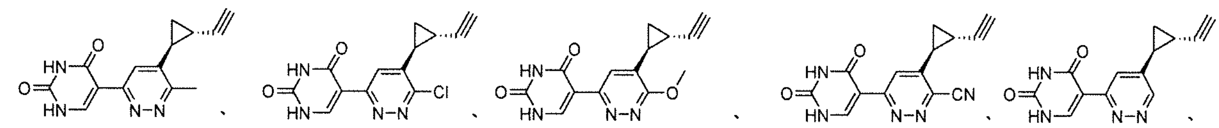

, , , , ,

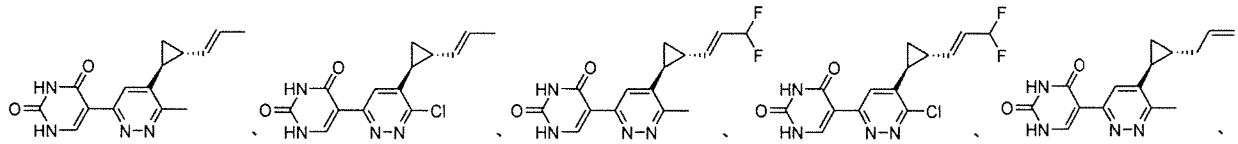

, , , , ,

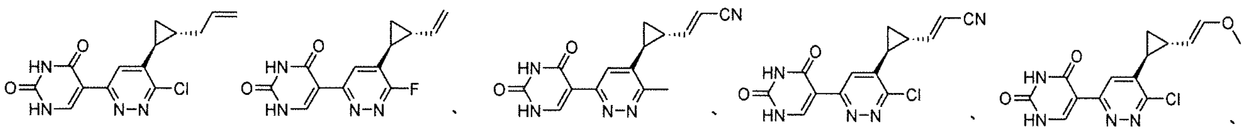

, , , ,

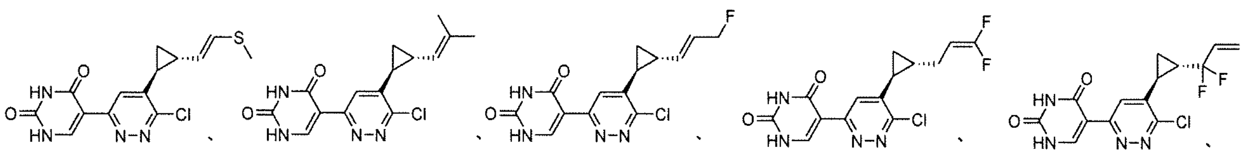

, , , , ,

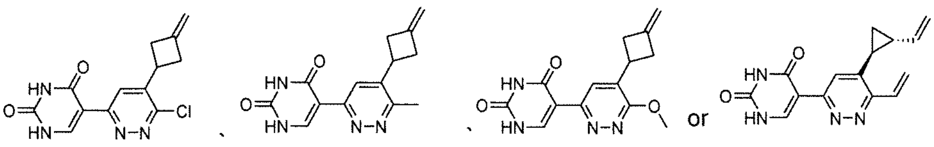

, , or

The present invention provides a pharmaceutical composition, wherein, the pharmaceutical composition contains a compound of Formula (I) of a therapeutic effective quantity, its tautomeric, deuterated compound or pharmaceutically acceptable salt thereof, and at least one pharmaceutically acceptable excipient.

The present invention provides an application of the compound of formula (I), it's tautomeric, deuterated compound or pharmaceutically acceptable salt thereof or its pharmaceutical composition in the preparation of drugs; preferably application of the preparation of cancer therapeutics.

The present invention provides a method for treating and/or preventing disease, including the application to the treated object of a therapeutic effective quantity of the compound of formula(I) or its pharmaceutical composition; preferably for the treatment and/or prevention of cancer.

The present invention provides a combination of drugs, the drug combination contains the compounds of formula(I), its tautomeric, deuterated compound or pharmaceutically acceptable salt, and anti-PD-L (1) antibodies.

The present invention provides a method for preparing compound having formula (ID), its tautomeric, deuterated compound or pharmaceutically acceptable salt, which includes:

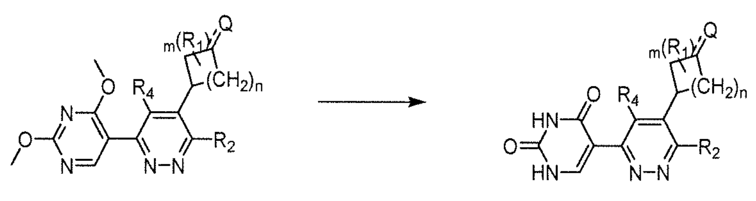

IC ID

The compound having formula (IC) reacts under acidic conditions to obtain the compound having formula (ID), wherein;

$R_1$-$R_2$, $R_4$, Q, m, n are defined in formula (I).

The present invention further provides a preferably technical solution for the application of the compound in the preparation of medicine.

Preferably, the application is for the preparation of a medicament for treating a disease mediated by CD73.

Preferably, the application is in the preparation of a drug for treating and/or preventing cancer.

Preferably, the cancer is breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophagus cancer, melanoma, colorectal cancer, hepatocellular carcinoma, head and neck tumor, hepatobiliary cell carcinoma, myelodysplastic syndrome, malignant glioma, prostate cancer, thyroid cancer, xuwang's cell tumor, lung squamous cell carcinoma, lichenoid keratosis, synovial sarcoma, skin cancer, pancreatic cancer, testicular cancer or liposarcoma.

The present invention also provides a method for treating and/or preventing diseases, comprising administering a therapeutically effective amount of at least any compound represented by structural Formula (I) or a pharmaceutical composition containing the compound to a treatment subj ect.

The present invention also provides a method for treating and/or preventing diseases, including administering to a treatment subject at least any compound shown in formula (I) or a pharmaceutical composition containing the compound in a therapeutically effective amount.

The present invention also provides a method for treating cancer, comprising administering a therapeutically effective amount of at least any compound represented by structural Formula (I) or a pharmaceutical composition containing the compound to a treatment subject.

Unless otherwise specified, the general chemical terms used in the general structural Formula have their usual meanings.

For example, unless otherwise specified, the term "halogen" used in the present invention refers to fluorine, chlorine, bromine or iodine.

In the present invention, unless otherwise specified, "alkyl" will be understood to mean a linear or branched monovalent saturated hydrocarbon group. For example, alkyl includes methyl, ethyl, propyl, isopropyl, n-butyl, isobutyl, sec-butyl, tert-butyl, n-pentyl, 3-(2-methyl)butyl, 2-pentyl, 2-methylbutyl, neopentyl, n-hexyl, 2-hexyl, 2-methylpentyl, etc. Similarly, the "$_{1-6}$" in "$C_{1-6}$ alkyl" refers to a straight-chain or branched group containing 1, 2, 3, 4, 5 or 6 carbon atoms.

"Heteroalkyl" means that one of the carbon atoms on the alkyl group is substituted by a heteroatom, including N, S, or O.

" alkylene" refers to a divalent saturated hydrocarbon group including a straight or branched chainmethylene, for example, methylene, 1, 2-ethylenes, 1, 3-metapropyl or 1, 2-subisopropyl.

Similarly, The "1-6" in "$C_{1-6}$ alkylene" refers to a group that contains one, two, three, four, five, or six carbon atoms arranged in straight or branched chains.

"Alkoxy" refers to the oxyether form of the aforementioned linear or branched alkyl group, which is -O-alkyl.

In the present invention, "a", "an", "the", "at least one" and "one or more" can be used interchangeably. Thus, for example, a composition comprising "a" pharmaceutically acceptable excipient can be interpreted to mean that the composition includes "one or more" pharmaceutically acceptable excipients.

The term "aryl" in the present invention, unless otherwise specified, will be understood to mean an unsubstituted or substituted monocyclic or condensed ring aromatic group including carbon ring atoms. In a further embodiment, the aryl group is a 6 to 10 membered monocyclic or bicyclic aromatic ring group. In a further embodiment, it is phenyl and naphthyl. Most preferably is phenyl. The aryl ring can be fused to a heteroaryl, heterocyclyl, or cycloalkyl, wherein the ring attached to the parent structure is an aryl ring, non-limiting examples including, but not limited to, benzocyclopentyl.

The term " heterocyclyl ", in the present invention, unless otherwise specified, will be understood to mean an unsubstituted or substituted 3-14 member-stable monocyclic system consisting of carbon atoms and 1-3 heterocyclic atoms selected from N, O, or S, which is a saturated or partially unsaturated monocyclic or polycyclic 14-member-ring hydrocarbon substituted group comprising 3 to 14 carbon atoms in which nitrogen or sulfur heterocyclic atoms may be selectively oxidized.And nitrogen hetero atoms can be selectively quaternized.The heterocyclic group can be attached to any heteroatom or carbon atom to form a stable structure.Examples of these heterocyclic groups include, but are not limited to, azocyclic butakyl, pyrroalkyl, piperidine, oxypiperazine, oxypiperidine, tetrahydrofuran, dioxane, tetrahydroimidazolyl, tetrahydrothiazolyl, tetrahydrooxazolyl, tetrahydropyranyl, morpholine, thiomorpholine sulfoxide, thiomorpholine sulfoxide, and tetrahydrooxadiazole.The heterocyclic group may be thickened to an aryl group, heteraryl group or cycloalkyl ring, wherein the ring connected with the parent structure is a heterocyclic group.

The term "heteroaryl", as used herein, unless otherwise specified, will be understood to mean an unsubstituted or substituted stable 5- or 6-membered monocyclic aromatic ring system or an unsubstituted or substituted 9- or 14-membered benzo-fused heteroaromatic ring system or bicyclic heteroaromatic ring system, which consists of carbon atoms and 1-4 heteroatoms selected from N, O or S, and wherein the nitrogen or sulfur heteroatoms can be selectively oxidized. The nitrogen heteroatoms can be selectively quaternized. The heteroaryl group can be attached to any heteroatom or carbon atom to form a stable structure. Examples of heteroaryl groups include, but are not limited to thienyl, furyl, imidazolyl, isoxazolyl, oxazolyl, pyrazolyl, pyrrolyl, thiazolyl, thiadiazolyl, triazolyl, pyridyl, pyridazinyl, indolyl, azaindolyl, indazolyl, benzimidazolyl, benzofuranyl, benzothienyl, benzisoxazolyl, benzothiazolyl, benzothiazolyl, benzene and thiadiazolyl, benzotriazolyl adenine, quinolinyl or isoquinolinyl. The heteroaryl group can be fused to an aryl, heterocyclyl or cycloalkyl ring, wherein, the ring connected with the parent structure is a heteroaryl ring.

The term "cycloalkyl" refers to a cyclic saturated or partially unsaturated monocyclic or polycyclic cyclic hydrocarbon substituent having 3 to 14 carbon atoms, for example, cyclopropyl, cyclobutyl, cyclopentyl or cyclohexyl. The cycloalkyl group can be fused on an aromatic, heterocyclic or heteroaryl ring, wherein the ring connected with the parent structure is a cycloalkyl group.

The term "substituted" will be understood to mean that one or more hydrogen atoms in the group are replaced by the same or different substituents. Typical substituents include, but are not limited to H, cyano, halogen, $C_{1-6}$ alkyl, $C_{1-6}$ haloalkyl, -$C_{0-6}$ alkylene -$OR_a$, -$C_{0-6}$ alkylene - OC(O)N $(R_a)_2$, -$C_{0-6}$ alkylene -N$(R_a)_2$, -$C_{0-6}$ alkylene -$NR_aC(O)R_a$, -$C_{0-6}$ alkylene -$NR_aC(O)N$ $(R_a)_2$, -$C_{0-6}$ alkylene -$NR_aS(O)R_a$, -$C_{0-6}$ alkylene -$NR_aS(O)_2R_a$, -$C_{0-6}$ alkylene -S(=O)$R_a$,-$C_{0-6}$ alkylene -S (= O)$_2R_a$, - $C_{0-6}$ alkylene -$SR_a$, - $C_{0-6}$ alkylene -S$(R_a)_5$, - $C_{0-6}$ alkylene -C(=O)$R_a$, - $C_{0-6}$ alkylene -C(=O)$OR_a$, - $C_{0-6}$ alkylene -C(=O)N $(R_a)_2$, $C_{2-6}$ alkenyl, $C_{2-6}$ alkynyl,-$C_{0-6}$ alkylene - $C_{3-14}$ cycloalkyl , -$C_{0-6}$ alkylene -(3-14) heterocyclic, -$C_{0-6}$ alkylene -$C_{6-14}$ aryl or -$C_{0-6}$ alkylene -(5-14 heteroaryl),the $C_{1-6}$ alkyl, $C_{2-6}$ alkenyl, $C_{2-6}$ alkynyl, -$C_{0-6}$ alkylene -$C_{3-14}$ cycloalkyl ,-$C_{0-6}$ alkylene -(3-14) heterocyclic, -$C_{0-6}$ alkylene -$C_{6-14}$ aryl or -$C_{0-6}$ alkylene -(5-14 heteroaryl) optionally substituted by one or more $R_a$;

Each $R_a$ is independently H, halogen, hydroxyl, amino, oxo, nitro, cyano, carboxyl, $C_{1-6}$ alkyl, $C_{1-6}$ hydroxyalkyl, $C_{1-6}$ aminoalkyl, $C_{1-6}$ haloalkyl, $C_{1-6}$ alkoxy, $C_{1-6}$ haloalkoxy, $C_{1-6}$ heteroalkyl, $C_{3-8}$ cycloalkyl, 3-8 heterocyclic, $C_{6-14}$ aryl, or 5-14 heteroaryl.

In certain embodiments, the substituents are independently -F, -Cl, -Br, -I, -OH, trifluoromethoxy, ethoxy, propoxy, isopropoxy, n-butoxy group, isobutoxy, tert-butoxy, -$SCH_3$, - $SC_2H_5$, formaldehyde, -C($OCH_3$), cyano, nitro, -$CF_3$, -$OCF_3$, amino, dimethylamino, methylthio, sulfonyl or acetyl groups.

Examples of substituted alkyl groups include, but are not limited to, 2,3-dihydroxypropyl, 2-aminoethyl, 2-hydroxyethyl, pentachloroethyl, trifluoromethyl, methoxymethyl, pentafluoroethyl, phenylmethyl, dioxenylmethyl and piperazinylmethyl.

Examples of substituted alkoxy groups include, but are not limited to, 2-hydroxyethoxy, 2-fluoroethoxy, 2,2-difluoroethoxy, 2-methoxyethoxy, 2-aminoethoxy, 2,3-dihydroxypropoxy, cyclopropylmethoxy, aminomethoxy, trifluoromethoxy, 2-diethylaminoethoxy, 2-ethoxycarbonylethoxy, 3- hydroxypropoxy.

The term "pharmaceutically acceptable salt" will be understood to mean a salt prepared from a pharmaceutically acceptable non-toxic base or acid. When the compound provided by the present invention is an acid, its corresponding salt can be conveniently prepared from pharmaceutically acceptable non-toxic bases, including inorganic bases and organic bases. When the compound provided by the present invention is a base, the corresponding salt can be conveniently prepared from pharmaceutically acceptable non-toxic acids, including inorganic acids and organic acids.

Since the compound represented by Formula (I) will be used as a medicine, it is preferable to use a certain purity, for example, at least 60% purity, more suitable purity is at least 75%, and particularly suitable purity is at least 98% (% is weight ratio).

The prodrug of the compound of the present invention is included in the protection scope of the present invention. Generally, the prodrug refers to a functional derivative that is easily converted into a desired compound in the body. For example, any pharmaceutically acceptable salt, ester, salt of ester or other derivative of the compound of the present application can directly or indirectly provide the compound of the present application or its pharmaceutically active metabolite or residues.

The compound of the present invention may contain one or more asymmetric centers, and may produce diastereomers and optical isomers from this. The present invention includes all possible diastereomers and their racemic mixtures, their substantially pure resolved enantiomers, all possible geometric isomers and their pharmaceutically acceptable salts.

When the compound shown in formula (I) has tautomers, unless otherwise stated, the invention includes any possible tautomers, pharmaceutically acceptable salts thereof, and mixtures thereof.

When compounds of Formula (I) which are substituted with heavier isotopes such as deuterium may offer certain therapeutic advantages, this is due to greater metabolic stability, such as increased in vivo half-life or reduced dosage requirements.

When the compound represented by Formula (I) and its pharmaceutically acceptable salt have solvates or polymorphs, the present invention includes any possible solvates and polymorphs. The type of solvent that forms the solvate is not particularly limited, as long as the solvent is pharmaceutically acceptable. For example, water, ethanol, propanol, acetone and similar solvents can be used.

The term "composition", as used herein, will be understood to mean a product comprising a specified amount of each specified ingredient, and any product produced directly or indirectly from a combination of specified amounts of each specified ingredient. Therefore, pharmaceutical compositions containing the compounds of the present invention as active ingredients and methods for preparing the compounds of the present invention are also part of the present invention.

The pharmaceutical composition provided by the present invention includes as an active component a compound

represented by Formula (I) (or a pharmaceutically acceptable salt thereof), a pharmaceutically acceptable excipient and other optional therapeutic components or accessories. Although in any given case, the most suitable way of administering the active ingredient depends on the particular subject to be administered, the nature of the subject and the severity of the disease, the pharmaceutical composition of the present invention includes oral, rectal, topical and a pharmaceutical composition for parenteral administration (including subcutaneous administration, intramuscular injection, and intravenous administration). The pharmaceutical composition of the present invention can be conveniently prepared in a unit dosage form known in the art and prepared by any preparation method known in the pharmaceutical field.

**Examples**

In order to make the above contents more clear and definite, the following examples will be used to further elaborate the technical scheme of the invention. The following examples are only used to explain the specific embodiments of the invention, so that those skilled in the art can understand the invention, but are not used to limit the scope of protection of the invention.

In the specific embodiments of the invention, the technical means or methods not specifically described are conventional technical means or methods in the field.

Unless otherwise specified, all parts and percentages in the present invention are calculated by weight, and all temperatures refer to □.

The following abbreviations have been used:

*n*-BuLi:n-butyl lithium;
Dioxane: dioxane;
DCM: methylene chloride;
DIEA or DIPEA: N, N-diisopropylethylamine;
TFA: trifluoroacetic acid;
TBSCl: tert-butyldimethylchlorosilane;
DIC: N, N-diisopropyl carbodiimide;
PCC: pyridinium chlorochromate;
TBAF: tetrabutyl ammonium fluoride trihydrate;
THF: tetrahydrofuran;
MeOH: methanol;
LC-MS: liquid chromatography-mass spectrometry;
DMAP: 4-dimethylaminopyridine;
DMF: N, N-dimethylformamide;
PdCh(dppf) : [1,1' -bis (diphenylphosphine) ferrocene] palladium dichloride;
$Pd(dppf)Cl_2$-$CH_2Cl_2$:[1,1'-bis(diphenylphosphine) ferrocene] palladium dichloride dichloromethane complex;
PE: petroleum ether;
Xantphos: 4, 5-diphenylphosphine -9, 9-dimethyloxaanthrene;
$Pd(AcO)_2$: palladium acetate;
$Pd_2(dba)_3$: tri (dibenzyl acetone) dipalladium;
Dess-Martin reagent: Dess - Martin's reagent;
h or hrs: hours;
min: minutes.

**Preparation of intermediate M1**

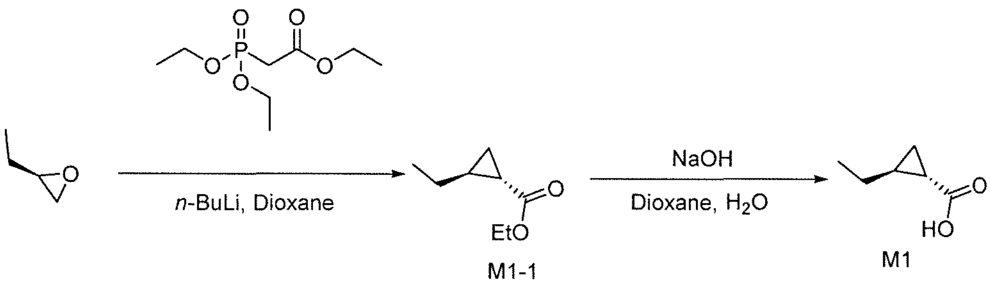


M1-1

M1

Step 1: Synthesis of compound M1-1

Triethyl phosphoacylacetate (46.64g) was dissolved in anhydrous 1, 4-dioxane (60 mL), cooled to 0°C, slowly added n-BuLi(83.2 mL) under nitrogen protection, and then the reaction mixture was stirred at room temperature for 0.5h. (R) -2-ethyl ethylene oxide (15.00g) was added, and the reaction mixture was stirred at room temperature for 10min. Then the reaction mixture was transfered to a tank, heat up to 150°C reaction overnight. The reaction solution was cooled to room temperature, stirred with water, extracted with methyl tert-butyl ether for three times. The organic phase was washed with saturated aqueous NaCl, dried and concentrated to produce yellow oil, which was compound M1-1 (24.00g,81.13%yield).

Step 2: Synthesis of compound M1

Compound M1-1(24.00 g) was dissolved in 1, 4-dioxane (150 mL) and NaOH(47.26 g) was dissolved in water (150 mL), they were then poured into the reaction system and the reaction was heated to 100 °C overnight. The reaction solution was cooled to room temperature, extracted with methyl tert-butyl ether for three times, the aqueous phase was collected, and the pH of the aqueous phase was adjusted to 1-2. The aqueous phase was extracted for three times with methyl tert-butyl ether, and the organic phase was collected. The organic phase was washed with saturated aqueous NaCl, dried and concentrated to obtain a yellow oil (14.50 g, 75.27% yield), which was compound M1.

LCMS: $[M+H]^+$= 114.1

Preparation of intermediate M2

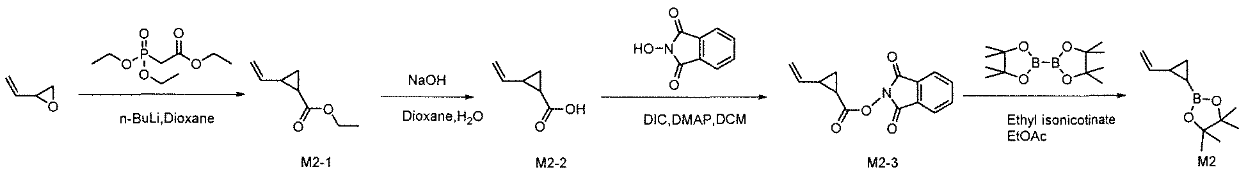


Step 1: Synthesis of compound M2-1

Triethyl phosphoacylacetate (34.20 g) was dissolved in anhydrous 1, 4-dioxane (30 mL), cooled to 0°C, slowly added n-BuLi(61.0 mL) under nitrogen protection, and the reaction mixture was stirred at room temperature for 0.5h. Epoxy butene (10.69g) was added, and the reaction mixture was stirred at room temperature for 10min.Then the reaction mixture was transferred to a tank, heated up to 150 °C reaction overnight. The reaction solution was cooled to room temperature, stirred with water, extracted with methyl tert-butyl ether for three times, the organic phase was collected, washed with saturated aqueous NaCl, dried, and concentrated to yield a yellow oil (20.00g,93.54% yield), which was compound M2-1.

Step 2: Synthesis of compound M2-2

The compound M2-1(10.00g) was dissolved in 1, 4-dioxane (50mL), and NaOH(9.00g) was dissolved in water (50mL), then they were poured into the reaction system, the reaction was heated to 100 ° C overnight. After completion of the reaction, the reaction solution was cooled to room temperature, extracted with methyl tert-butyl ether for three times, the aqueous phase was collected, the pH of the aqueous phase was adjusted to 1-2. The aqueous phase was extracted for three times by methyl tert-butyl ether, and the organic phase was collected. The organic phase was washed with saturated aqueous NaCl, dried and concentrated to produce yellow oil (7.20 g,90.01% yield), which was compound M2-2.

Step 3: Synthesis of compound M2-3

The compounds M2-2(7.20 g), N-hydroxyphthalimide (10.47 g) and DMAP(784 mg) were dissolved in dichloromethylane (80 mL), cooled to 0°C, slowly added DIC(8.10 g), and then the reaction mixture was stirred at room temperature for 2h.The reaction solution was filtered through the sand core funnel covered with silica gel, collectted the filtrate. The filtrate was dried by rotary evaporation to obtain crude product, the crude product was purified by column chromatography to a white solid , which was compound M2-3 (7.50 g, 45.40% yield) .

Step 4: Synthesis of compound M2

The compounds 3(7.50 g), bis(pinacolato)diboron (11.10 g) and ethyl isonicotinate (660 mg) were dissolved in ethyl acetate (80 mL), and the reaction mixture was stirred at 85 °C for 24 h under nitrogen protection. After completion of the reaction, the reaction solution was dried by rotary evaporation to obtain the crude product, the crude product was purified by column chromatography to a colorless oil form, which was compound M2 (130 mg, 2.29% yield).
LCMS: $[M+H]^+$= 194.2

**Preparation of intermediate M3**

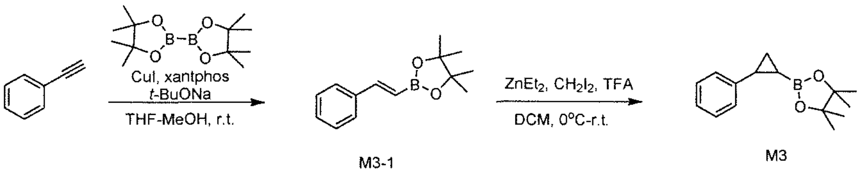


M3-1 M3

Step 1: Synthesis of compound M3-1

Bis(pinacolato)diboron (29.8g), CuI (1.86g), xantphos (5.67g) were added to a 1L three -necked flask, dissolved in THF (250mL), the reaction mixture was stirred at room temperature for 10 min under $N_2$ protection, and then phenylacetylene (10.0g) was added to the reaction solution, the reaction mixture was stirred continuely for 5 min. Sodium tert-butanol (18.81 g) was added to the reaction solution under the ice bath. Finally, methanol (50 mL) was added to the reaction solution, and the reaction was carried out for 2 hours at room temperature. The reaction solution was directly evaporated to obtain the crude product. The crude product was separated by column chromatography (PE:EA=10:1) to obtain 19.8 g of light yellow oil, which was compound M3-1.

Step 2: Synthesis of compound M3

Dry DCM (50 mL) was added into a 500 mL three-necked flask in an ice bath, then 1M diethylzinc (146 mL) was added, and then TFA (9.76 mL) was added very slowly to the reaction solution. The reaction mixture was stirred in an ice bath for 30 minutes under nitrogen protection. Then the diiodomethane (39.1g) was dropped into the reaction solution. The reaction mixture was stirred under the ice bath for 30 minutes, then compound M3-1 (16.8g) was dropped into the reaction solution. The reaction solution slowly rised to room temperature, and the reaction lasts for 48 hours. The reaction solution was quenched by adding saturated ammonium chloride solution, extracted by EA for three times, and the organic phase was collected. The organic phase was washed with water and saturated aqueous NaCl, and evaporated to dryness to obtain the crude product. The crude product was separated by column chromatography (PE:EA=10:1) to obtain 9.8g of milky white oil, which was compound M3.
LCMS: $[M+H]^+$= 244.2

**Preparation of intermediate M4**

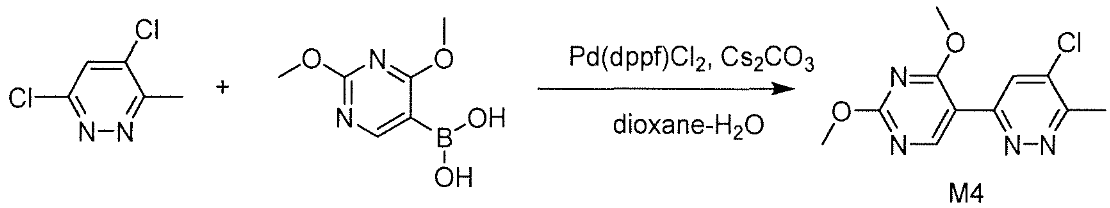


M4

Step 1:4, 6-dichloro-3-methyldapazine (1.3 g), 2, 4-dimethoxy-5-pyrimidine boric acid (1.6 g), $Pd(dppf)Cl_2-CH_2Cl_2$ (325.64 mg)and $Cs_2CO_3$(3.75 g)were mixed with dioxane (25 mL) and water (5 mL).Under nitrogen protection, the reaction mixture was stirred at 70°C for two hours . After completion of the reaction ,the reaction solution was mixed in appropriate amount of water, extracted with ethyl acetate for three times. The organic phase was collected,

which was washed with saturated aqueous NaCl, dried, and concentrated to yield a crude product. The crude product was purified by column chromatography (PE: EA = 1:1) to obtain 1.1 g of white solid, which was compound M4([M+H]$^+$:267, 269).

**Preparation of intermediate compound M5**

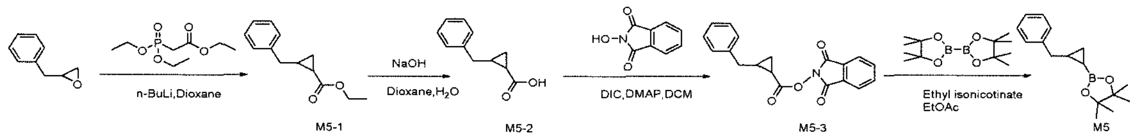


M5-1 M5-2 M5-3 M5

Step 1: Synthesis of compound M5-1

Triethyl phosphonoacetate (8.35g) was dissolved in anhydrous 1, 4-dioxane (20mL) and cooled to 0°C. n-BuLi(14.9mL) was slowly added under nitrogen protection. The reaction mixture was stirred at room temperature for 0.5h. 2-benzyl ethylene oxide (5.00g) was added and the reaction mixture was stirred at room temperature for 10min. The reaction solution was then transferred to a stuffy tank, heated up to 150°C to react overnight. The reaction solution was cooled to room temperature, the reaction system was stirred with water, extracted with methyl tert-butyl ether for three times. The organic phase was collected, washed with saturated aqueous NaCl, dried, and concentrated to obtain a yellow oil (7.00g,91.96% yield), which was compound M5-1.

Step 2: Synthesis of compound M5-2

The compound M5-1(7.00g) was dissolved in 1, 4-dioxane (20mL), and NaOH(6.85g) was dissolved in water (50mL), then they were poured into the reaction system, and the reaction was heated to 100 ° C overnight. After completion of the reaction, the reaction solution was cooled to room temperature, extracted with methyl tert-butyl ether for three times, and the aqueous phase was collected. The pH of the aqueous phase was adjusted to 1-2, extracted with methyl tert-butyl ether for three times, and the organic phase was collected washed with saturated aqueous NaCl, dried and concentrated to obtain a yellow oil (4.80 g,79.49% yield), which was compound M5-2.

Step 3: Synthesis of compound M5-3

The compounds M5-2(4.80 g), N-hydroxyphthalimide (4.88 g) and DMAP(1.6g) were dissolved in methylene chloride (80 mL), cooled to 0°C, and DIC(3.40 g) was slowly added, and then the reaction mixture was stirred at room temperature for 2h.After completion of the reaction, the reaction solution was filtered through the sand core funnel covered with silica gel, the filtrate was collected and dried by rotary evaporation to obtain the crude product. The crude product was purified by column chromatography to a white solid (3.50 g, 39.98% yield), which was compound M5-3.

Step 4: Synthesis of compound M5

The compound M5-3(3.50g), 2,2' -bibenzo [d][1,3,2] dioxazolborane (5.18g) were dissolved in DMF(110mL) and the reaction mixture was stirred for 14h under nitrogen protection with blue light.Then, triethylamine (40mL) solution of pinacol (5.18g) was added to the reaction solution and the reaction mixture was stirred at room temperature for 2h. After completion of the reaction, water was added to the reaction solution. The mixed solution was extracted with ether for three times. The organic phase was collected, and the organic phase was washed with saturated aqueous NaCl, dried, and concentrated to obtain the crude product. The crude product was purified by column chromatography to a colorless oil form (131 mg, 4.66% yield), which was compound M5.
LCMS: [M+H]$^+$=258.2.

**Preparation of intermediate M6**

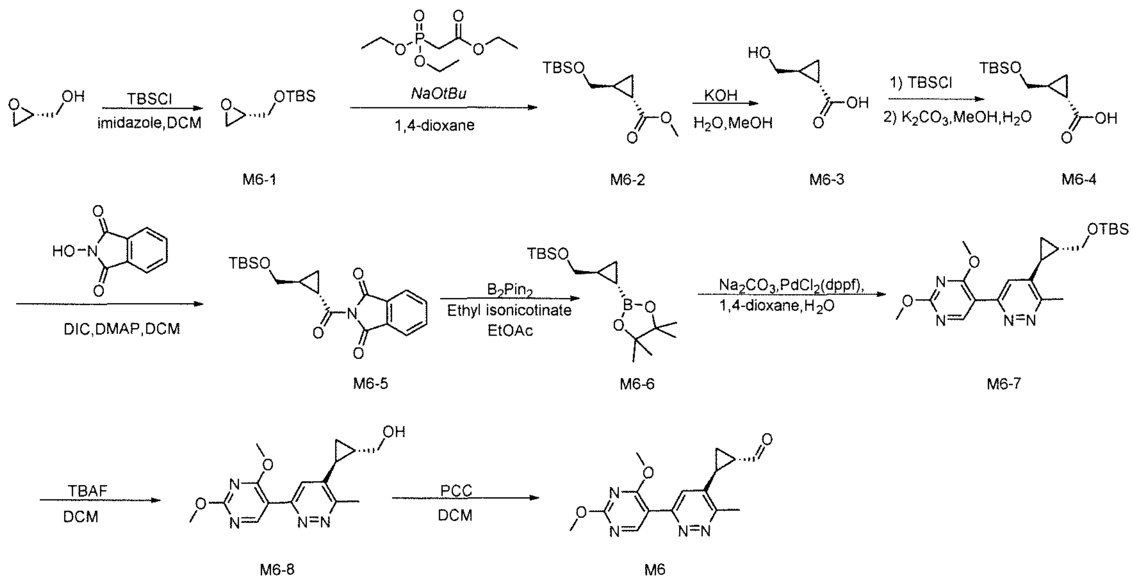


M6-1 M6-2 M6-3 M6-4 M6-5 M6-6 M6-7 M6-8 M6

Step 1: Synthesis of compound M6-1

(R) -Ethylene oxide 2-yl methanol (50g) and imidazole (69g) were dissolved in anhydrous methylene chloride (500mL), cooled to 0°C, and TBSCl (152g) was added in batches under nitrogen protection, then the reaction mixture was stirred at room temperature for 1h. After completion of the reaction, the reaction solution was filtered, the filter residue was washed with methylene chloride. The filtrates were combined, and concentrated to obtain the crude product, the crude product was purified by column chromatography (PE:EA=80:1) to give a colorless liquid (85g, 66.86% yield), which was compound M6-1.

Step 2: Synthesis of compound M6-2

Triethyl phosphoacylacetate (77g) and sodium tert-butanol (33g) were dissolved in anhydrous 1, 4-dioxane (100mL), cooled to 0°C, the reaction mixture was stirred at room temperature for 0.5h under nitrogen protection.Compound M6-1(65g) was added and the reaction mixture was stirred at room temperature for 10min. Then the reaction mixture was transferred to a tank, heat up to 150°C to reactovernight. After completion of the reaction, the reaction system was stirred with water and then extracted with methyl tert-butyl ether for three times. The organic phase was collected, washed with saturated aqueous NaCl, dried, and concentrated to obtain a yellow oil (80g,89.88% yield), which was compound M6-2.

Step 3: Synthesis of compound M6-3

Compound M6-2(65g) was dissolved in methanol (500mL), NaOH(40g) was dissolved in water (50mL), and then they were poured into the reaction system. The reaction mixture was stirred at 60 °C overnight. After completion of the reaction, the reaction solution was cooled to room temperature. The methanol in the reaction solution was evaporated,then the aqueous phase was extracted with methyl tert-butyl ether three times, the aqueous phase was collected, the pH of the aqueous phase was adjusted to 1-2, extracted with ethyl acetate for three times, and the organic phase was collected washed with saturated aqueous NaCl, dried and concentrated to obtain a yellow oil (15 g, 51.36% yield), which was compound M6-3.

Step 4: Synthesis of compound M6-4

Compounds M6-3(30g) and imidazole (37g) were dissolved in methylene chloride (300mL), cooled to 0°C, TBSCl(82g) was added in batches, and the reaction mixture was stirred at room temperature for 1h. After completion of the reaction, the reaction solution was filtered through the sand core funnel covered with silica gel, and the filtrate was dried by rotary evaporation to obtain an oil substance. The oil substance was dissolved in methanol (200mL), and the potassium carbonate (71g) of water (200mL) solution was added. The reaction mixture was stirred at room temperature for 3h. The methanol was removed by rotary evaporation, and the aqueous layer was extracted for three times with ethyl acetate adjusted pH to 5 with 10% aqueous citric acid, and then extracted for three times with ethyl acetate. The organic

layer was collected and dried by rotary evaporation to give a yellow oil (18g, 30.24% yield), which was compound M6-4.

Step 5: Synthesis of compound M6-5

The compound M6-4(18 g), N-hydroxyphthalimide (15 g) and DMAP(0.95g) were dissolved in methylene chloride (200 mL), cooled to 0°C, and DIC(11 g) was slowly added, and then the reaction mixture was stirred at room temperature for 2 h. After completion of the reaction, the reaction solution was filtered through the sand core funnel covered with silica gel, the filtrate was dried by rotary evaporation to obtain the crude product, the crude product was purified by column chromatography to a yellow liquid (22 g, 72.28% yield), which was compound M6-5.

Step 6: Synthesis of compound M6-6

Compound M6-5 (22 g), 2,2 '- Biphenyl [d] [1,3,2] dioxazole borane (29 g) and ethyl isonicotinate (4.3 g) were dissolved in ethyl acetate (220 mL), and the reaction mixture was stirred at 85°C for 16h under the protection of nitrogen. After completion of the reaction, the reaction solution was dried by rotary evaporation to obtain the crude product, the crude product was purified by column chromatography (PE:EA=50:1) to obtain a colorless liquid (10 g, 56.82% yield), which was compound M6-6.

Step 7: Synthesis of compound M6-7

Compound M4(3.0 g), M6-6 (7.0 g), potassium phosphate (9.5 g), dichlorodi-tert-butyl-(4-dimethylaminophenyl) phosphopalladium (2.0 g), dioxane (30 mL) and water (15 mL) were added into a 100 mL three-necked flask under $N_2$ protection. The reaction mixture was stirred at 90°C for 2 h, After completion of the reaction, the reaction solution was poured into water, extracted by ethyl acetate, the organic phase was collected, extracted by ethyl acetate and washed with saturated aqueous NaCl. The organic phase was dried and concentrated to obtain the crude product, and the crude product was separated by column chromatography (PE: EA = 1:1) to obtain the yellow oil (3.9g, 82.97% yield), which was compound M6-7.

Step 8: Synthesis of compound M6-8

Compound M6-7(3.9g), THF of TBAF (1M ,12 mL) and THF(30 mL) were added into 100 mL single-mouth flask and the reaction mixture was stirred at 60°C for 1 h. After completion of the reaction, the saturated ammonium chloride aqueous solution was added to the reaction solution, the mixed solution was extracted by ethyl acetate, and the organic layer was concentrated to obtain the yellow oily crude product (4.9g), which was compound M6-8.

Step 9: Synthesis of compound M6

Compound M6-7(0.25g) and anhydrous methylene chloride (5mL) were added into a 50mL three-necked flask, and stirred under the protection of $N_2$ in an ice bath, PCC(0.35g) was added under the ice bath, and the reaction mixture was stirred at room temperature overnight. After completion of the reaction, the reaction solution was filtered by diatomite, washed by methylene chloride, the organic layer was collected , dried and concentrated to obtain crude product, the crude product was purified by column chromatography (DCM:MeOH=20:1) to obtain brown liquid, which was compound M6 (0.20g,80.53%yield).

LCMS: $[M+H]^+$= 301.3

**Preparation of intermediate compound M7**

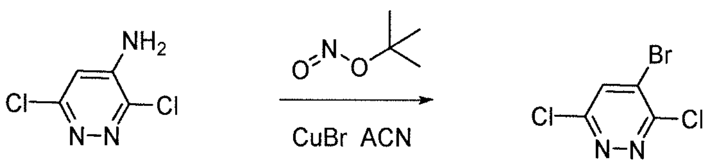

Synthesis of compound M7

3, 6-dichloropyridazine 4-amine (30 g), cuprous bromide (34 g) and acetonitrile (300 mL) were added into a 500 mL three-necked flask, stirred and dissolved, then tert-butyl nitrite (23 g) was slowly added in an ice bath, the reaction mixture was transferred to 60°C and reacted for 4 h. After completion of the reaction, the reaction solution was filtered by diatomite, the filtrate was collected, and the filtrate was dried to obtain the crude product. The crude product was purified by column chromatography (PE: EA = 10:1) to obtain the white solid (21 g, 50.38% yield), which was compound M7.

LCMS: $[M+H]^+$= 228.2

**Preparation of intermediate compound M8**

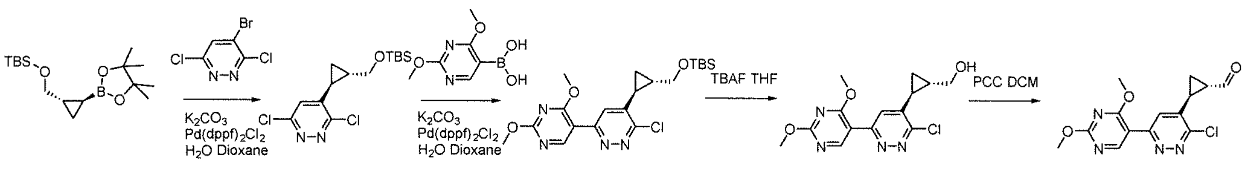


M8-1 M8-2 M8-3 M8-4 M8

Step 1: Synthesis of compound M8-2

Compound M7(8.0 g), M8-1 (13.1 g), potassium carbonate (7.3 g), [1,1' bis (diphenylphosphine) ferrocene] palladium dichloride (2.8 g), dioxane (80 mL) and water (20 mL)were added to 100 mL three-way flask, the reaction mixture was stirred at 90°C for 2 h under $N_2$ protection. After completion of the reaction, the reaction solution was poured into water, extracted by ethyl acetate, and the organic phase was collected. The organic phase was washed with saturated aqueous NaCl, dried and concentrated to obtain the crude product. The crude product was separated by column chromatography (PE: EA = 20:1) to obtain the yellow solid (3.9 g, 34.18% yield), which was compound M8-2.

Step 2: Synthesis of compound M8-3

Compound M8-2(2.0 g), (2,4-Dimethoxypyrimidin-5-yl) boric acid (1.2 g), potassium carboate (1.1 g), [1,1' -bis (diphenylphosphino) ferrocene] palladium dichloride (0.2 g), dioxane hexacyclode (25 mL) and water (1.5 mL) were added in three-necked flask, the reaction mixture was stirred at 60°C for 0.5h under $N_2$ protection. After completion of the reaction, the reaction solution was poured into water, extracted with ethyl acetate, the organic phase was collected, washed with saturated aqueous NaCl, dried and concentrated to obtain the crude product, and the crude product was separated by column chromatography (PE: EA = 1:1) to obtain the yellow liquid (1.0 g, 38.13% yield), which was compound M8-3.

Step 3: Synthesis of compound M8-4

Compound M8-3 (1.1 g), THF(4 mL) of 1M TBAF, and THF(10 mL) were added into 100 mL three-necked flask and the reaction mixture was stirred at 60°Cfor 1 h. After completion of the reaction, the mixed solution was extracted with ethyl acetate, the organic phase was collected, concentrated to obtain the crude product, the crude product was purified by column chromatography to give a white solid (0.2 g), which was compound M8-4.

Step 4: Synthesis of compound M8

Compound M8-4(0.20g) and anhydrous methylene chloride (5mL) were added into a 50mL three-necked flask, stirred under ice bath and $N_2$ protection, PCC(0.26g) was added and then the reaction mixture was stirred at room temperature overnight. After completion of the reaction, the crude product was dried and concentrated to give the crude product, the crude product was purified by column chromatography (DCM:MeOH=20:1) to give a brown liquid (0.17 g, 80.53% yield),which was compound M8.

LCMS: $[M+H]^+$= 321.2

**Preparation of intermediate compound M9**

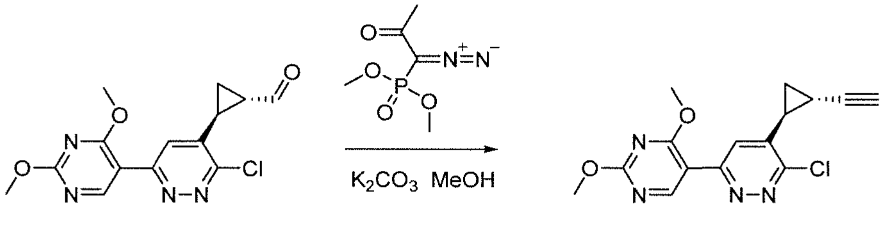


M8 M9

Step 1: Synthesis of compound M9

Compound M8(2.0g), potassium carbonate (1.6g), methanol (20mL) were dissolved in a 100mL single-mouth flask, and (1-diazo-2-oxpropyl) dimethyl phosphonate (2.2g) was added under ice bath, the reaction mixture was stirred at room temperature for 1h. After completion of the reaction, the reaction solution was quenched with saturated ammonium chloride aqueous solution, extracted with ethyl acetate, the organic phase was collected, the organic phase was washed with saturated aqueous NaCl, dried and concentrated to obtain the crude product. The crude product was separated by column chromatography (PE: EA = 1:1) to obtain a white solid (1.1 g, 55.69% yield), which was compound M9.
LCMS: $[M+H]^+$= 317.2

**Preparation of intermediate compound M10**

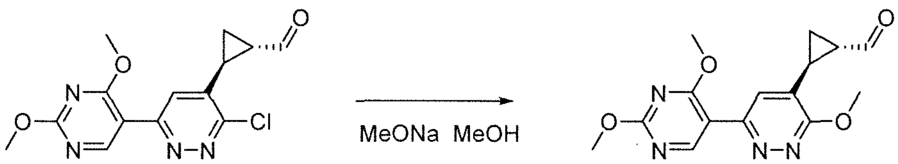


M8 M10

Step 1: Synthesis of compound M10

Compound M8(0.2g), sodium methanol (0.4g) and methanol (10mL) were added to a 100mL single-mouth flask The reaction mixture was stirred for 6h under tube seal at 60 °C. After completion of the reaction, the reaction solution was evaporated to dryness, then water was added, the mixed solution was extracted with ethyl acetate, and the organic phase was collected. The organic phase was washed in saturated aqueous NaCl, dried, and concentrated to a colorless oil (0.18 g, 90.59% yield), which was compound M10.
LCMS: $[M+H]^+$= 317.2

**Preparation of intermediate compound M11**

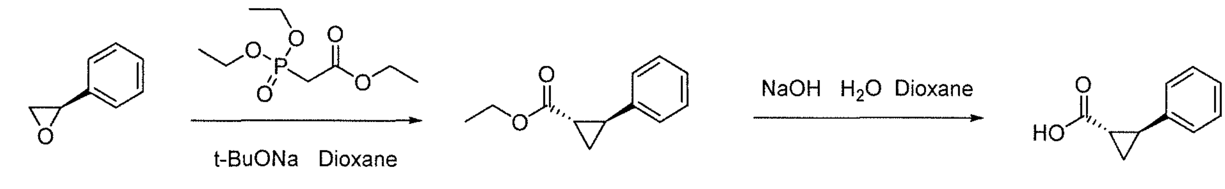


M11-1 M11-2 M11-3

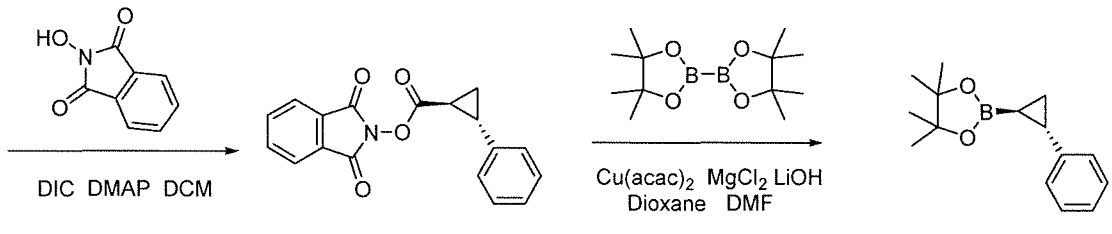


M11-4 M11

Step 1: Synthesis of compound M11-2

Triethyl phosphonoacetate (62g) and sodium tert-butanol (26g) were dissolved in anhydrous 4-dioxane (70mL), cooled to 0°C, the reaction mixture was stirred at room temperature for 0.5h under nitrogen protection. Compound M11-1(30g) was added and the reaction mixture was stirred at room temperature for 10min. Then the reaction mixture was transferred to a tank, heated up to 150°C to react overnight. After completion of the reaction, the reaction solution was cooled to room temperature, stirred with water, the mixed solution was extracted with methyl tert-butyl ether for three times, and the organic phase was collected. The organic phase was washed with saturated aqueous NaCl, dried and concentrated to obtain a yellow oil (40g, 84.21% yield),which was compound M11-2.

Step 2: Synthesis of compound M11-3

The compound M11-2(110g) was dissolved in dioxane (300mL) and NaOH(80g) was dissolved in water (300mL), then they were poured into the reaction system, the reaction mixture was stirred at 100°Covernight. After completion of the reaction, the reaction solution was cooled to room temperature, extracted for three times with methyl tert-butyl ether, and the aqueous phase was collected. The pH of the aqueous phase was adjusted to 1-2, the aqueous phase was extracted for three times with methyl tert-butyl ether, and the organic phase was collected. The organic phase was washed in saturated aqueous NaCl, dried, and concentrated to obtain a yellow oil (90g, 95.97% yield), which was compound M11-3.

Step 3: Synthesis of compound M11-4

Compound M11-3(100 g), N-hydroxyphthalimide (121 g) and DMAP(22 g) were dissolved in methylene chloride (1000 mL), cooled to 0°C, DIC(93 g) was slowly added, and then the reaction mixture was stirred at room temperature for 2h. After completion of the reaction, the reaction solution was filtered by diatomite, and the filtrate was concentrated to obtain crude product. The crude product was purified by column chromatography to a white solid (100 g, 52.78% yield), which was compound M11-4.

Step 4: Synthesis of compound M11

The compounds M11-4(1.00g), bis(pinacolato)diboron (2.48g), anhydrous magnesium chloride (0.46g), lithium hydroxide monohydrate (2.06g) and Copper(II) Acetylacetonate (0.26g) were dissolved in a mixture of methyl tert-butyl ether (24mL) and DMF(4mL) , the reaction mixture was stirred under nitrogen protection at room temperature for 10min. After completion of the reaction, the reaction solution was filtered by diatomite, the filtrate was extracted by EA ,the organic phase was collected, washed with saturated aqueous NaCl, dried with anhydrous sodium sulfate, slurried with petroleum ether, filtered, and the filtrate was collected. The filtrate was purified by column chromatography (PE:EA=100:1) to obtain a colorless liquid (0.25 g, 31.47% yield), which was compound M11.

LCMS: $[M+H]^+$= 245.2

**Example 1: Synthesis of compound 1 5-(5-((*1S,2S*)-2-ethylcyclopropyl)-6-vinylpyridazin-3-yl)pyrimidine-2,4(*1H,3H*)-dione**

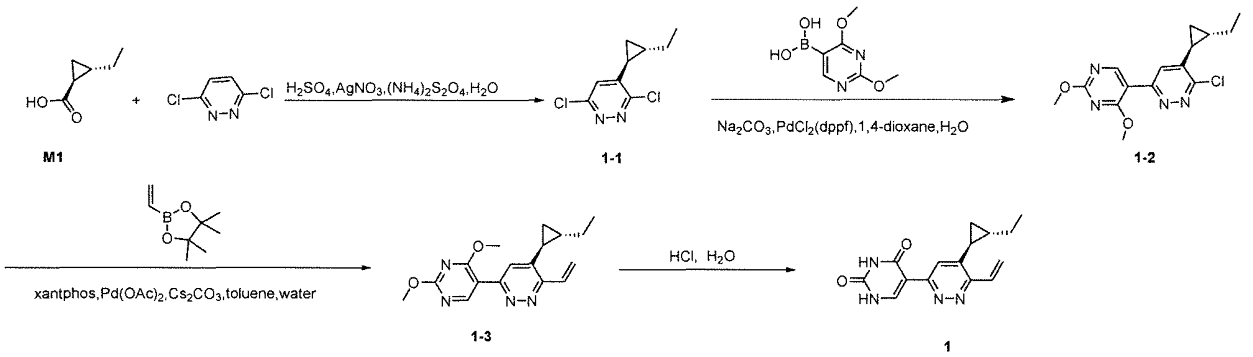


Step 1: Synthesis of compounds 1-1

Compound M1(2.7g) and 2, 5-dichloradazine (3.2g) were added into a 1L three-necked flask, dissolved in water (200mL), and concentrated sulfuric acid (5mL) was added and mixed evenly. Silver nitrate (0.73mg) dissolved with water (200mL) and ammonium disulfite (9.55g) aqueous solution (200mL) were added under $N_2$ protection at 70°C, the reaction mixture was stirred for 1h at 70 °C. The reaction solution was coolled to room temperature, ammonia water was added to the reaction solution under the ice bath, and the pH of the reaction solution was adjusted to 9, the mixed solution was extracted with ethyl acetate, and the organic phase was dried by anhydrous sodium sulfate and concentrated in vacuo.The crude product was separated by column chromatography (PE:EA=10:1) to give 2.6 g of yellow solid, which was compound 1-1.

Step 2: Synthesis of compounds 1-2

Compound 1-1 (2.0 g) and 2, 4-dimethoxy-pyrimidine-5-boric acid (1.7g) were dissolved in 1, 4 dioxane (6mL) and water (2mL) in a 50mL single-mouth flask. $Na_2CO_3$ (1.5g) and $PdCl_2$ (dppf)(540 mg) were added, and the reaction mixture was stirred at 40°C for 3h under $N_2$ protection. The reaction solution was cooled to room temperature, saturated aqueous NaCl (50mL) was added into the reaction solution. The mixed solution was extracted with ethyl acetate, and the organic phase was dried by anhydrous sodium sulfate and concentrated in vacuo. The crude product was separated by column chromatography (PE:Acetone:DCM=35:9:6) to give 650mg of yellow solid, which was compounds 1-2.

Step 3: Synthesis of compounds 1-3

Compound 1-2 (50mg) and vinylboronic acid pinacol cyclic ester (96mg) were added to a 50mL single-mouth flask, dissolved in toluene (1.5mL) and water (0.3mL), cesium carbonate (102mg) was added, and finally Xantphos (4.51mg) and $Pd(AcO)_2$(1.4mg) were added, the reaction mixture was stirred at 100°C for 1h under $N_2$ protection. The reaction solution was cooled to room temperature, saturated aqueous NaCl (10mL) was added extracted with ethyl acetate, and the organic phase was dried by aqueous sodium sulfate and separated by column chromatography (PE:Acetone:DCM=35:9:6) to give 32 mg of white solid, which was compounds 1-3.

Step 4: Synthesis of Compound 1

Compounds 1-3 (32mg) were added to 25mL single-mouth flask and dissolved in 1mol/L hydrochloric acid (2mL), the reaction mixture was stirred at 70°C for 3h. cool to room temperature, ethanol (10mL) was added. The reaction solution was dried by rotary evaporation and separated by reverse phase chromatography ($CH_3CN/H_2O$=30%-55%) to obtain 4.04mg white solid, which was compound 1.
LCMS: $[M+H]^+$= 285.1

**Example 2: Synthesis of compound 2**

**5-(5-((*1S,2S*)-2-ethylcyclopropyl)-6-(prop-1-en-2-yl)pyridazin-3-yl)pyrimidine-2,4(*1H,3H*)**-dione

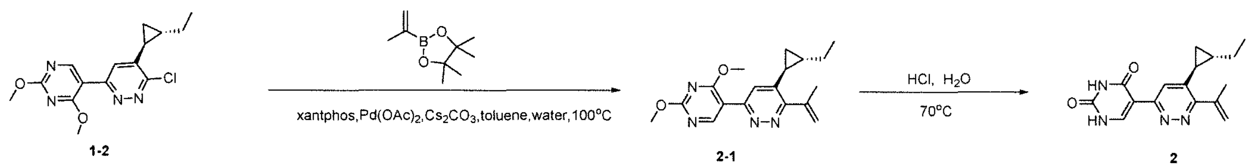


1-2 2-1 2

Step 1: Synthesis of compound 2-1

Compound 1-2 (80mg) and 4,4,5, 5-tetramethyl-2 - (prop-1-ene-2-yl) -1,3, 2-dioxabiorane (168mg) were dissolved in toluene (1.5mL) and water (0.3mL) in a 50mL single-mouth flask, cesium carbonate (162mg) was added, then Xantphos (11.54mg) and $Pd(AcO)_2$(4.48mg) were added, the reaction mixture was stirred at 100°C for 1h under $N_2$ protection, cooled to room temperature, and saturated and salt water (10mL) was added extracted with ethyl acetate, and the organic phase was dried with anhydrous sodium sulfate and separated by column chromatography (PE:Acetone:DCM=35:9:6) to give 40 mg of white solid, which was compound 2-1.

Step 2: Synthesis of Compound 2

Compound 2-1 (40mg) was added to a 25mL single-mouth flask, dissolved in 1mol/L hydrochloric acid (2mL), and the reaction mixture was stirred at 70°C for 3h, cooled to room temperature, ethanol (10mL) was added.The reaction solution was dried by rotary evaporation and separated by reversed-phase chromatography column ($CH_3CN/H_2O$=30%-55%) to give 9.13mg of white solid, which was compound 2 (purity 99.13%).

LCMS: $[M+H]^+$=299.2

**Example 3: Synthesis of compound 3 5-(6-cyclopropyl-5-((*1S,2S*)-2-ethylcyclopropyl)pyridazin-3-yl)pyrimidine-2,4(*1H,3H*)-dione**

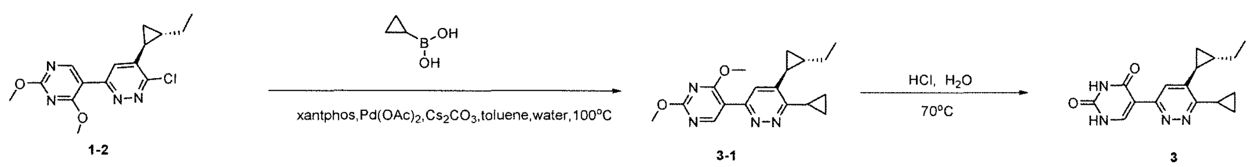


1-2 3-1 3

Step 1: Synthesis of compound 3-1

Compound 1-2 (60mg) and cyclopropylboronic acid (80mg) were added to a 50mL single-mouth bottle, dissolved in toluene (2mL) and water (0.5mL), cesium carbonate (122mg) was added, and finally Xantphos (5.41mg) and $Pd(AcO)_2$ (1.68mg) were added, the reaction mixture was stirred at 100°C for 1h under $N_2$ protection, cooled to room temperature, saturated aqueous NaCl (10mL) was added. The mixed solution was extracted with ethyl acetate, and the organic phase was dried with anhydrous sodium sulfate and separated by column chromatography (PE:Acetone:DCM=35:9:6) to give 38 mg of white solid, which was compound 3-1.

Step 2: Synthesis of compound 3

Compound 3-1 (38mg) was added to a 25mL single-mouth flask and dissolved in 1mol/L hydrochloric acid (2mL), the reaction mixture was stirred at 70 °C for 3h, cooled to room temperature, ethanol (10mL) was added.The reaction solution was dried by rotary evaporation and separated by reversed-phase chromatography column ($CH_3CN/H_2O$=30%-55%) to obtain 18.47mg of white solid, which was compound 3 (purity 97.50%).

LCMS: $[M+H]^+$= 299.2

Example 4: **Synthesis of compound 4**

**5-(6-chloro-5-(3-methylenecyclobutyl) pyridazin-3-yl)pyrimidine-2,4(*1H,3H*)-dione**

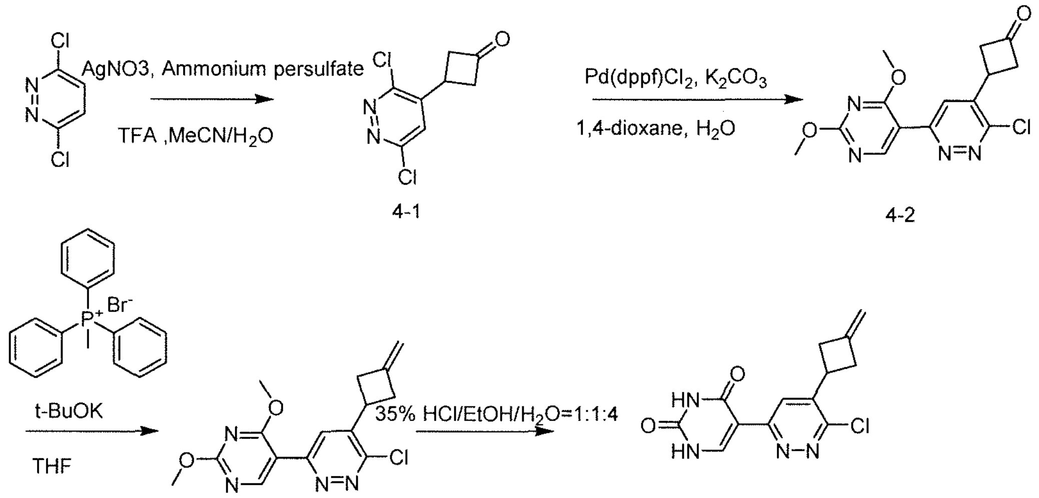


4-1

4-2

4-3

4

Step 1: Synthesis of compound 4-1

In a 250mL three-necked flask, 3-oxycyclobutanyl carboxylic acid (11.5g) and 2, 5-dichloroprazine (5.0g) were added, dissolved in water (70mL) and acetonitrile (70mL). Silver nitrate (5.7g) and ammonium disulfite (15.4g) were added by drops under $N_2$ protection and oil bath (70°C), and finally trifluoroacetic acid (0.76g) was added, the reaction mixture was stirred at 70°C for 1h, cooled to room temperature, ammonia water was added to the reaction solution under the ice bath, the pH was adjusted to 9, extracted with ethyl acetate and the organic phase was collected. The organic phase was dried with anhydrous sodium sulfate and separated by column chromatography (PE:EA=5:1) to obtain 2.5 g white solid, which was compound 4-1.

Step 2: Synthesis of compound 4-2

Compound 4-1 (2.0g) and 2, 4-dimethoxy-pyrimidine-5-boric acid (2.7g) were dissolved in 1, 4-dioxane (50 mL) and water (10 mL) in a 100mL single-mouth flask. $K_2CO_3$( (2.5g) and $PdCl_2$(dppf) (672 mg) were added, the reaction mixture was stirred at 70°C for 1h under $N_2$ protection, cooled to room temperature, then saturated aqueous NaCl (50 mL) was added, the mixed solution was extracted with ethyl acetate, and the organic phase was collected. The organic phase was dried with anhydrous sodium sulfate and separated by column chromatography (EA: DCM=1:5) to obtain 1.2 g white solid, which was compound 4-2.

Step 3: Synthesis of compound 4-3

Methyl triphenyl phosphine bromide (0.4g) was added into a 50mL three-necked flask, dissolved in anhydrous tetrahydrofuran (5mL), and potassium tert-butanol (0.13g) was added, the reaction mixture was stirred at 0°C for 1h under $N_2$ protection. At 0°C, compound 4-2(0.2g) was added, and the reaction was carried out at room temperature for 1h. Saturated aqueous NaCl (10mL) was added into the reaction solution, extracted with ethyl acetate, and the organic phase was collected. The organic phase was dried with anhydrous sodium sulfate and separated by column chromatography (PE:EA=1:1) to obtain 52 mg light yellow solid, which was compound 4-3..

Step 4: Synthesis of Compound 4

Compound 4-3 (52 mg) was added to a 10mL single-mouth flask and dissolved in ethanol (1 mL) and water (4 mL). Concentrated hydrochloric acid (1 ml) was added and the reaction mixture was stirred for 48h at room temperature. The reaction solution was evaporated by rotation at low temperature to remove the solvent and separated by reverse

phase column chromatography ($CH_3CN/H_2O$=25%-68%) to obtain 12.8 mg of white solid, which was compound 4.

$^1H$ NMR (500 MHz, DMSO-$d_6$) $\delta$11.54 (s, 2H), 8.38 (s, 1H), 8.36 (s, 1H), 4.89 - 4.88 (m, 2H), 3.73 - 3.69 (m, 1H), 3.17 - 3.12 (m, 2H), 2.85 - 2.82 (m, 2H).

**Embodiment 6: Synthesis of compound 6 5-(6-methyl-5-(2-vinylcyclopropyl)pyridazin-3-yl)pyrimidine-2,4(*1H,3H*)-dione**

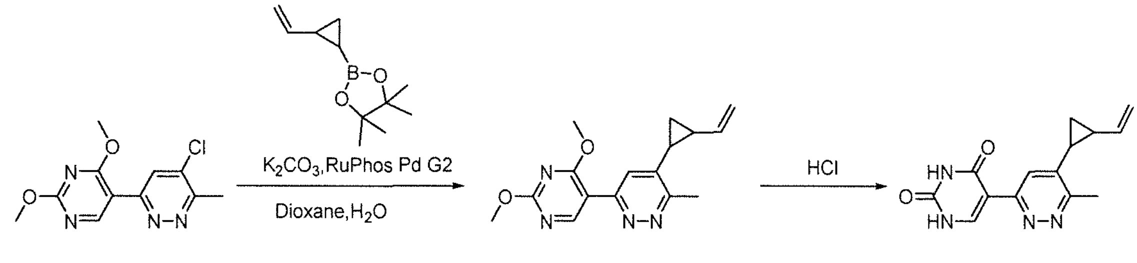


M4 6-1 6

Step 1: Synthesis of compound 6-1

Compound M4(100 mg), compound M2(130 mg), RuPhos Pd G2(10 mg) and $K_2CO_3$ (179.66 mg) were mixed with dioxane (5 mL) and water (1 mL) under the protection of nitrogen. The reaction mixture was stirred at 70°C. Two hours later, TLC monitoring showed a complete conversion. The reaction solution was mixed in appropriate amount of water, extracted with ethyl acetate for three times, and the organic phase was collected, washed by saturated aqueous NaCl, dried and concentrated to obtain the crude product. The crude product was purified by preparation TLC (PE:EA=1:1)to obtain 25mg white solid, which was compound 6-1 ([M+H]+:299.2).

Step 2: Synthesis of compound 6

Compound 6-1(25 mg) was dissolved in 1M hydrochloric acid solution (2 mL) and heated to 70°C for reaction. After 5h, LC-MS showed complete response. The reaction solution was purified by preparative liquid chromatography to obtain 3.9mg white solid, which was compound 6.

($[M+H]^+$:271.2).

$^1H$ NMR (500 MHz, $CDCl_3$) $\delta$ 8.45 (s, 1H), 7.95 (s, 1H), 5.64 - 5.54 (m, 1H), 5.21 (d, J= 17.0 Hz, 1H), 5.05 (d, *J*= 10.3 Hz, 1H), 2.76 (s, 3H), 1.97 - 1.91 (m, 1H), 1.80 - 1.73 (m, 1H), 1.33 - 1.24 (m, 2H).

**Example** 7: **Synthesis of compound 7 5-(6-methyl-5-((*1S*,*2R*)-2-vinylcyclopropyl)pyridazin-3-yl)pyrimidine-2,4(*1H,3H*)-dione**

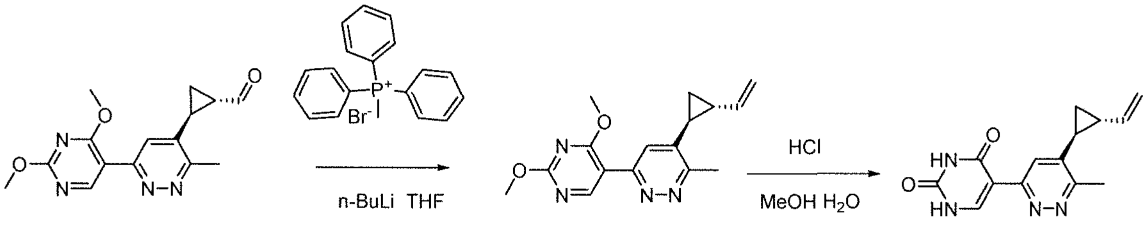


M6 7-1 7

Step 1: Synthesis of compound 7-1

The triphenyl methyl phosphine bromide was dissolved in THF (2mL) and cooled to 0 °C, N-butyl lithium (0.2mL,1.6 Minhexane) was slowly added. After stirring for 5min, compound M6 (50mg) was added, and the reaction mixture was raised to room temperature for 3h. After completion of the reaction, saturated ammonium chloride aqueous

solution was added to quench the reaction. The reaction solution was extracted by ethyl acetate for three times, and the organic phase was collected and washed by saturated aqueous NaCl, dried with anhydrous sodium sulfate, and separated by Pre-TLC (DCM:MeOH=25:1) to obtain 26mg white solid product, which was compound 7-1.

Step 2: Synthesis of compound 7

Compound 7-1 (26mg) was dissolved in 1M HCl (1mL) and methanol (1mL), the reaction mixture was stirred at 30°C for 3d. After completion the reaction, the pH of the reaction solution was adjusted to neutral with sodium bicarbonate, extractwith EA for three times, and the organic phase was collectedand concentrated to obtain the crude product, then separated by preparative liquid chromatography to obtain 6.3 mg white solid product, which was compound 7.

LCMS: $[M+H]^+$= 271.1

$^1H$ NMR (500 MHz, DMSO-$d_6$) $\delta$ 11.43 (s, 2H), 8.23 (s, 1H), 7.76 (s, 1H), 5.64 (ddd, *J*= 17.2, 10.2, 8.7 Hz, 1H), 5.21 (dd, J= 17.1, 1.3 Hz, 1H), 4.99 (dd, J= 10.3, 1.5 Hz, 1H), 2.66 (s, 3H), 2.15 - 2.04 (m, 1H), 1.74 - 1.65 (m, 1H), 1.31-1.20 (m, 2H).

**Embodiment 8: Synthesis of compound 8 5-(6-methyl-5-((*1S,2R*)-2-((*E*)-prop-1-en-1-yl)cyclopropyl)pyridazin-3-yl)pyrimidine-2,4(*1H,3H*)-dione**

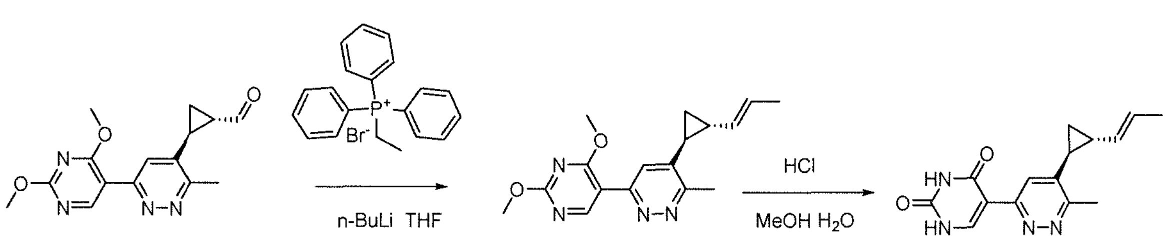


M6 8-1 8

Step 1: Synthesis of compound 8-1

The triphenyl ethyl phosphine bromide (185mg) was dissolved in THF (2mL) and cooled to 0 °C. N-butyl lithium (0.3mL, 1.6M in hexane) was slowly added. After stirring for 5min, compound M6(100mg) was added, and the reaction mixture was stirred at room temperature for 3h.After completion of the reaction, saturated ammonium chloride aqueous solution was added into the reaction solution for quenching reaction, extracted with ethyl acetate for three times,the organic phase was collected, which was washed with saturated aqueous NaCl, dried with anhydrous sodium sulfate, and separated by Pre-TLC (DCM:MeOH=25:1) to give 60mg of white solid product, which was compound 8-1.

Step 2: Synthesis of Compound 8

Compound 8-1 (60mg) was dissolved in 1M HCl (1mL) and methanol (1mL), the reaction mixture was stirred at 30°C for 3d. After completion of the reaction, the pH of the reaction solution was adjusted to neutral by sodium bicarbonate, extracted with EA for three times, the organic phase was collected, and concentrated to obtain the crude productthen separated by preparative liquid chromatography to obtain 6.5 mg white solid product, which was compound 8.

LCMS: $[M+H]^+$= 285.3

$^1H$ NMR (500 MHz, DMSO-$d_6$) $\delta$ 11.41 (s, 2H), 8.24 (s, 1H), 7.81 (s, 1H), 5.51-5.42 (m, 7.0 Hz, 1H), 5.12 - 5.04 (m, 1H), 2.65 (s, 3H), 2.06 - 1.96 (m, 1H), 1.90 - 1.80 (m, 1H), 1.68 (dd, *J* = 6.9, 1.6 Hz, 3H), 1.30 (dt, *J*= 8.6, 5.3 Hz, 1H), 1.15 (dt, *J*= 8.7, 5.1 Hz, 1H).

**Example 9: Synthesis of compound 9 5-(5-((*1S,2R*)-2-((*E*)-2-cyclopropylvinyl)cyclopropyl)-6-methylpyridazin-3-yl)pyrimidine-2,4(*1H,3H*)-dione**

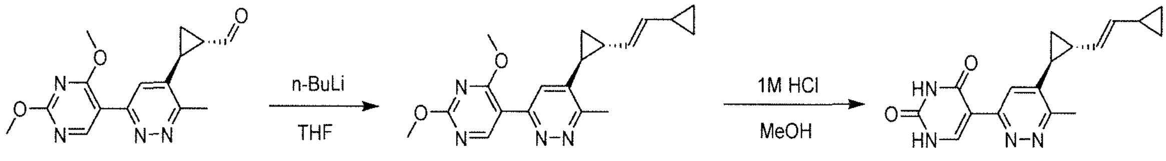


M6 9-1 9

Step 1: Synthesis of compound 9-1

Cyclopropyl methyl triphenyl phosphorus bromide (100mg) was added to a 25ml three-necked flask, dissolved in THF(5ml) under ice bath and $N_2$ protection, n-BuLi(0.15ml) was added to the reaction solution, the reaction mixture was stirred for 15 minutes, then compound M6(50mg) was added, the reaction mixture was stirred under ice bath for 1 hour. A small amount of water was added to the reaction solution to quench the reaction, and the reaction solution was concentrated and separated by column chromatography (DCM:MeOH=15:1)to obtain 20 mg white solid was, which was compound 9-1.

Step 2: Synthesis of compound 9

Compound 1-1 (20 mg) was added to 25 mL single-mouth falsk, dissolved in methanol (2 ml) and 1M hydrochloric acid (2 ml), and the reaction mixture was stirred at 40°C for 12 h. The reaction solution was adjusted to a neutral pH with saturated sodium bicarbonate solution,and concentrated to obtain the crude product, the crude product was separated and purified by column chromatography (DCM:MeOH=10:1) to obtain 8 mg white solid, which was compound 9 (purity 99.16%).

LCMS: $[M+H]^+$= 311.2

**Example 10: Synthesis of compound 10 (*E*)-3-((*1R*,*2S*)-2-(6-(2,4-dioxo-1,2,3,4-tetrahydropyrimidin-5-yl)-3-methylpyridazin-4-yl)cyclopropyl)acrylonitrile**

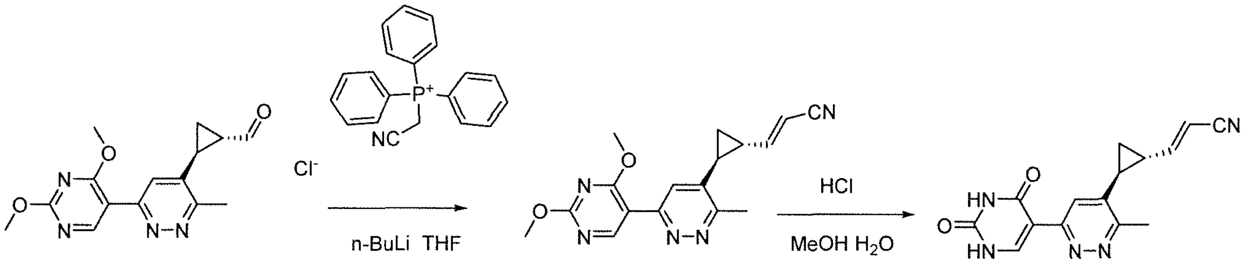


M6 10-1 10

Step 1: Synthesis of compound 10-1

Chlorinated (cyanomethyl) triphenylphosphorus (225 mg), anhydrous THF (5 mL) were added into a 25mL three-necked flask, 2.5M n-butyl lithium solution (0.3 mL) was slowly added under the protection of $N_2$ in an ice bath, the reaction mixture was stirred for 10 min under the ice bath, then compound M6 (100 mg) was added, and the reaction mixture was stirred for 1 h at room temperature. After completion of the reaction, saturated ammonium chloride solution was added to the reaction solution, extracted by ethyl acetate, the organic layer was washed with saturated aqueous NaCl, dried and concentrated to obtain the crude product, and the crude product was purified by column chromatography to a white solid (61 mg, 56.65% yield), which was compound 10-1.

Step 2: Synthesis of compound 10

Compound 10-1 (61 mg), methanol (1 mL), water (4 mL) and concentrated hydrochloric acid (1 mL) were added into a 25ml three-necked flask, the reaction mixture was stirred at room temperature for 72h. After completion of the reaction, saturated sodium bicarbonate aqueous solution was added to adjust pH to 7. the reaction solution was extracted with ethyl acetate . and washed with saturated aqueous NaCl, dried and concentrated to a white solid (13 mg, 23.65%

yield) which was compound 10 (99.23% purity).

LCMS: $[M+H]^+$= 296.2

$^1H$ NMR (500 MHz, DMSO-$d_6$) δ 11.38 (s, 2H), 8.25 (s, 1H), 7.80 (s, 1H), 6.65 (dd, J = 16.1, 9.6 Hz, 1H), 5.88 (d, J = 16.0 Hz, 1H), 2.65 (s, 3H), 2.04 - 1.95 (m, 1H), 1.54 - 1.44 (m, 2H), 1.24 (s, 1H).

**Example 11: Synthesis of compound 11 5-(5-((*1S,2S*)-2-(2,2-difluorovinyl)cyclopropyl)-6-methylpyridazin-3-yl)pyrimidine-2,4(*1H,3H*)-dione**

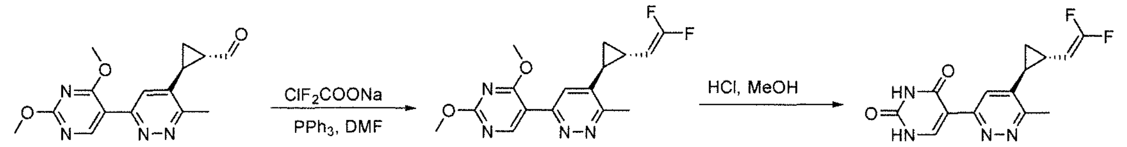


M6 11-1 11

Step 1: Synthesis of compound 11-1

Compound of M6 (100mg) and sodium difluorochloroacetic acid (102mg) were dissolved in DMF (3mL), followed by the addition of triphenylphosphine (175mg) under nitrogen protection, and the reaction mixture was stirred at 100°C for 2h. After completion of the reaction, water (10mL) was added to the reaction solution, the mixture was extracted with ethyl acetate for three times, the organic phase was washed with saturated aqueous NaCl, and dried with anhydrous sodium sulfate, separated by Pre-TLC (DCM:MeOH=25:1) to obtain 60mg of white solid product, which was compound 11-1.

Step 2: Synthesis of compound 11

Compound 11-1 (60mg) was dissolved in 1M HCl (1mL) and methanol (1mL), the reaction mixture was stirred at 30 °C for 3d. After completion of the reaction, the pH of the reaction solution was adjusted to neutral with sodium bicarbonate, the reaction solution was extracted for three times by EA, and the organic phase was collected. The organic phase was dried with anhydrous sodium sulfate and separated by Pre-TLC (DCM:MeOH=10:1) to obtain 15.5mg of white solid product, which was compound 11.

LCMS: $[M+H]^+$= 307.1

$^1H$ NMR (500 MHz, $CDCl_3$) δ 11.50 (s, 1H), 10.01 (s, 1H), 8.74 (s, 1H), 7.99 (s, 1H), 4.11 (d, J = 19.2 Hz, 1H), 2.78 (s, 3H), 1.96-1.84 (m, 1H), 1.72-1.61 (m, 1H), 1.48-1.38 (m, 1H), 1.25-1.15 (m, 1H).

**Embodiment 12: Synthesis of compound 12 5-(5-((*1S,2S*)-2-ethynylcyclopropyl)-6-methylpyridazin-3-yl)pyrimidine-2,4(*1H,3H*)-dione**

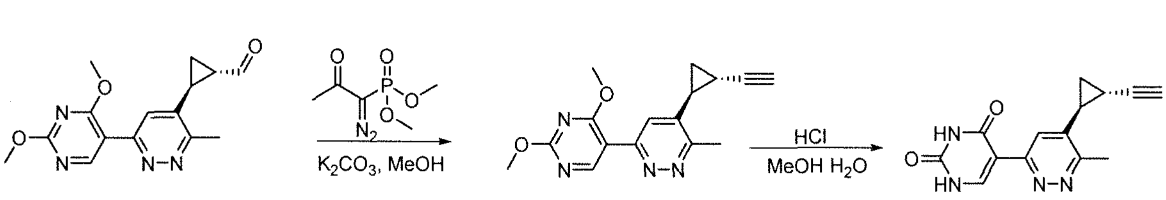


M6 12-1 12

Step 1: Synthesis of compound 12-1

Compound M6 (100mg) was dissolved in methanol (4mL), followed by the addition of potassium carbonate (87mg) to cool down to 0°C, followed by the addition of (1-diazo-2-oxo-propanol) -dimethyl phosphonate (185mg), the reaction mixture was then raised to room temperature for 1h. After completion of the reaction, saturated ammonium chloride aqueous solution was added to quench the reaction. The reaction solution was extracted with ethyl acetate for three times, the organic phase was washed with saturated aqueous NaCl, dried with anhydrous sodium sulfate, and separated by Pre-TLC (DCM:MeOH=25:1) to give a white solid product of 76mg, which was compound 12-1.

Step 2: Synthesis of compound 12

The compound 12-1 (76mg) was dissolved in 1M HCl (1mL) and methanol (1mL), and the reaction mixture was stirred at 30°C for 3d. After completion of the reaction, the solid was precipitated, filtered, and the filter cake was washed with water and dried to obtain 15 mg of white-like solid product, which was compound 12.

LCMS: $[M+H]^+$= 269.3

$^1H$ NMR (500 MHz, DMSO-$d_6$) δ 11.81 (s, 1H), 11.66 (s, 1H), 8.35 (d, *J* = 5.8 Hz, 1H), 8.01 (s, 1H), 2.97 (d, *J* = 2.0 Hz, 1H), 2.78 (s, 3H), 2.44 (dd, *J* = 12.3, 7.4 Hz, 1H), 1.81 (dd, *J* = 11.8, 5.5 Hz, 1H), 1.45 (t, *J* = 7.1 Hz, 2H).

**Example 13: Synthesis of compound 13 5-(6-chloro-5-((*1S,2R*)-2-vinylcyclopropyl)pyridazin-3-yl)pyrimidine-2,4(*1H,3H*)-dione**

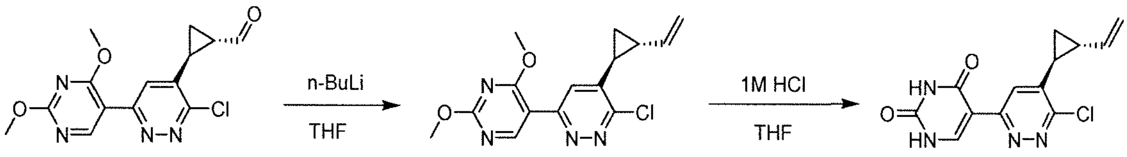


M8 13-1 13

Step 1: Synthesis of compound 13-1

Methyl triphenyl phosphorous bromide (580mg) was added to a 50ml three-necked flask, dissolved in THF(10ml)under $N_2$ protection and ice bath, n-BuLi(1.0ml) was added to the reaction solution, the reaction mixture was stirred for 15 minutes, and then compound M3(200mg) of THF solution was added to the reaction solution, the reaction mixture was stirred under ice bath for 1 hour. A small amount of water was added to the reaction solution to quench the reaction, the reaction solution was concentrated and separated by column chromatography (DCM:MeOH=15:1)to obtain 200mg of milky oil, which was compound 13-1.

Step 2: Synthesis of compound 13

Compound 13-1 (150 mg) was added to 25 mL single-mouth bottle, dissolved in THF (4 ml) and 1M hydrochloric acid (4 ml), and the reaction mixture was stirred at 50°C for 12 h. The reaction solution was adjusted to a neutral pH with a saturated sodium bicarbonate solution. The reaction solution was concentrated and separated by column chromatography (DCM: MeOH = 10:1) to obtain 30 mg of white solid, which was compound 13 (purity 99.16%).

LCMS: $[M+H]^+$= 291.1

$^1H$ NMR (500 MHz, DMSO-$d_6$) δ 11.57 (d, J = 6.2 Hz, 1H), 11.55 (s, 1H)., 8.31 (d, J = 6.2 Hz, 1H), 7.94 (s, 1H), 5.65 (ddd, J = 17.2, 10.3, 8.4 Hz, 1H), 5.22 (dd, J = 17.0, 1.6 Hz, 1H), 5.01 (dd, J = 10.2, 1.6 Hz, 1H), 2.26 -2.19 (m, 1H), 1.89 -1.80 (m, 1H), 1.42 -1.32 (m, 2H).

**Embodiment 14: Synthesis of compound 14 5-(6-chloro-5-((*1S,2S*)-2ethynylcyclopropyl)pyridazin-3-yl)pyrimidine-2,4(*1H,3H*)-dione**

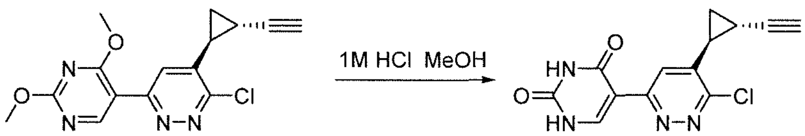


M9 14

Step 1: Synthesis of compound 14

Compound M9 (30 mg), methanol (4 mL), water (4 mL) and concentrated hydrochloric acid (0.5 mL) were added into a 25-ml single-mouth bottle and the reaction mixture was stirred at 40°C for 48h. After completion of the

reaction, the reaction solution was filtered to obtain a solid(crude product), the crude product was purified by the preparation liquid chromatography to obtain a white solid (8 mg, 29.30% yield), which was compound 14 (99.43% purity).

LCMS: $[M+H]^+$=289.2

$^1H$ NMR (500 MHz, DMSO-$d_6$) δ 11.55 (s, 2H), 8.31 (s, 1H), 7.97 (s, 1H), 2.95 (d, J = 2.1 Hz, 1H), 2.42 - 2.39 (m, 1H), 1.79 (ddq, J = 8.4, 4.6, 2.1 Hz, 1H), 1.49 - 1.39 (m, 2H).

**Example 15: Synthesis of compound 15 6-(2,4-dioxo-1,2,3,4-tetrahydropyrimidin-5-yl)-4-((*1S,2S*)-2-ethynylcyclopropyl)pyridazine-3-carbonitrile**

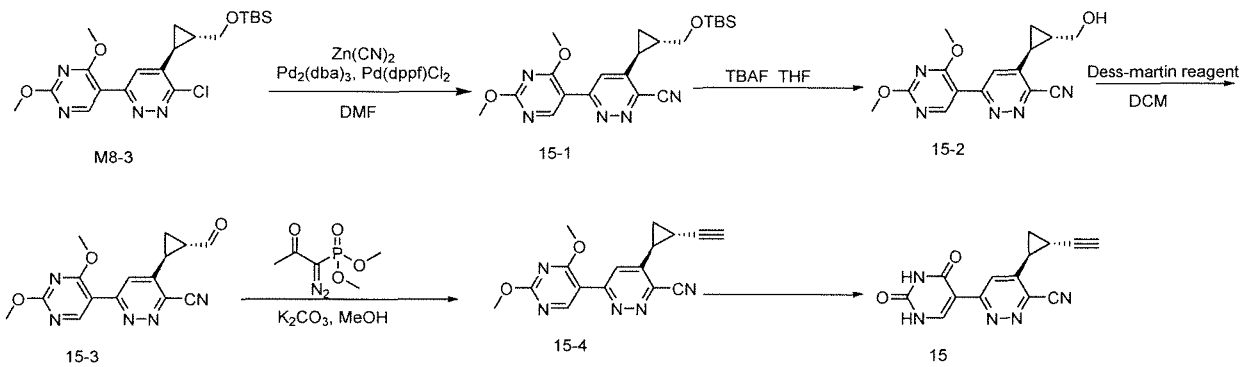


M8-3, 15-1, 15-2, 15-3, 15-4, 15

Step 1: Synthesis of compound 15-1

Compound M8-3 (400 mg), $Zn(CN)_2$ (110 mg), $Pd_2(dba)_3$ (40 mg), $Pd(dppf)Cl_2$ 41 (70 mg) were added to 25 ml three-necked flask, dissolved in DMF (10 mL), the reaction mixture was stirred at 120 °C overnight under the protection of $N_2$.The reaction solution was quenched with saturated sodium carbonate solution, extracted with EA for three times, the organic phase was washed twice with saturated sodium carbonate, concentrated to obtain the crude product. The crude product was separated by column chromatography (PE: EA = 5:1) to give 340 mg of a whitish solid, which was compound 15-1.

Step 2: Synthesis of Compound 15-2

Compound 15-1 (340 mg), TBAF(2 mL, 1 M solution in THF), THF(8 mL) were added into a 100 mL three-necked flask, the reaction mixture was stirred at room temperature for 2 h. After completion of the reaction, the saturated ammonium chloride solution was added to the reaction solution, extracted with ethyl acetate and the organic phase was concentrated to obtain the crude product, the crude product was purified by column chromatography to give a white solid (200 mg),which was compound 15-2..

Step 3: Synthesis of Compound 15-3

Compound 15-2 (200mg) and anhydrous dichloromethane (6 mL) were added to a 50-ml three-mouth bottle, the reaction mixture was stirred under $N_2$ protection and ice bath. Dess-Martin reagent(520mg) was added into the reaction solution under ice bath, then the reaction was carried out for 2 hours at room temperature. After completion of the reaction, sodium bicarbonate solid was added to the reaction solution, and the reaction solution was concentrated and purified by column chromatography (PE:EA=3:7) to obtain a white solid product (200mg), which was compound 15-3.

Step 4: Synthesis of Compound 15-4

Compound 15-3(100mg), potassium carbonate (87mg) and methanol (4mL) were added into a 100mL single-mouth flask, and (1-diazo-2-oxpropyl) dimethyl phosphonate (95mg) was added under an ice bath, then the reaction mixture was stirred at room temperature for 2h. After completion of the reaction, the reaction solution was quenched with saturated ammonium chloride solution. Then extracted with ethyl acetate, the organic phase was collected, and the organic phase was washed with saturated aqueous NaCl, dried, and concentrated to obtain a crude product, the crude product was separated by column chromatography (PE: EA = 1:1) to give a white solid (18mg), which was compound 15-4.

Step 5: Synthesis of compound 15

The compound 15-4 (18mg) was dissolved in 1M HCl (1mL) and THF (1mL), and the reaction mixture was stirred at 40°Covernight. After completion of the reaction, the reaction solution was neutralized with saturated sodium bicarbonate, extracted with ethyl acetate, and the organic phase was collectedand washed with saturated aqueous NaCl, dried with anhydrous sodium sulfate, and concentrated to obtain the crude product. The crude product was separated by Pre-TLC (DCM:MeOH=15:1) to obtain 8.7 mg of light yellow solid product, which was compound 15.
LCMS: $[M+H]^+$= 280.1

**Example 16: Synthesis of compound 16 5-(6-methyl-5-((*1S,2R*)-2-((*E*)-styryl)cyclopropyl)pyridazin-3-yl)pyrimidine-2,4(*1H,3H*)-dione**

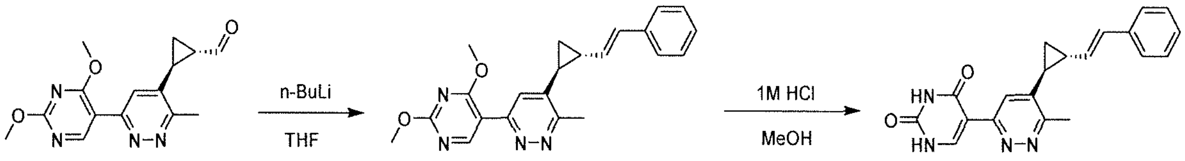

M6 16-1 16

Step 1: Synthesis of compound 16-1

Benzyl triphenyl phosphorus chloride (88mg) was added to a 25ml three-necked flask, dissolved in THF(5ml), cooled in an ice bath and under $N_2$ protection, and then n-BuLi(0.16ml) was added to the reaction solution , the reaction mixture was stirred for 15 minutes, then compound M6(50mg) was added to the reaction solution, the reaction mixture was stirred for 1 hour under an ice bath. A small amount of water was added to the reaction liquid to quench the reaction, and the reaction solution was concentrated and separated by column chromatography (DCM:MeOH=15:1) to give 30 mg of white solid, which was compound 16-1.

Step 2: Synthesis of compound 16

Compound 16-1 (30 mg) was added to 25 mL single-mouth flask and dissolved in methanol (2 ml) and 1M hydrochloric acid (2 ml). The reaction mixture was stirred at 40°C for 12 h. The reaction solution was adjusted to a neutral pH with saturated sodium bicarbonate solution.The reaction solution wasconcentrated and purified by Column chromatography (DCM:MeOH=10:1) to obtain
18 mg of white solid, which was compound 16 (purity 99.16%).
LCMS: $[M+H]^+$= 347.2
$^1H$ NMR (500 MHz, DMSO-$d_6$) δ 11.48-11.44 (m, 3H), 8.25-8.23 (m, 1H), 7.80 (s, 1H), 7.43 - 7.27 (m, 4H), 7.24 - 7.17 (m, 1H), 6.60 (d, *J* = 15.8 Hz, 1H), 6.15 (dd, *J* = 15.9, 8.8 Hz, 1H), 2.68 (s, 3H), 2.25-2.21 (m, 1H), 1.89-1.84 (m, 1H), 1.45 - 1.34 (m, 2H).

**Example17: Synthesis of compound 17 4-((*E*)-2-((*1R,2S*)-2-(3-chloro-6-(2,4-dioxo-1,2,3,4-**tetrahydropyrimidin-5-yl)pyridazin-4-yl)cyclopropyl)vinyl)benzonitrile

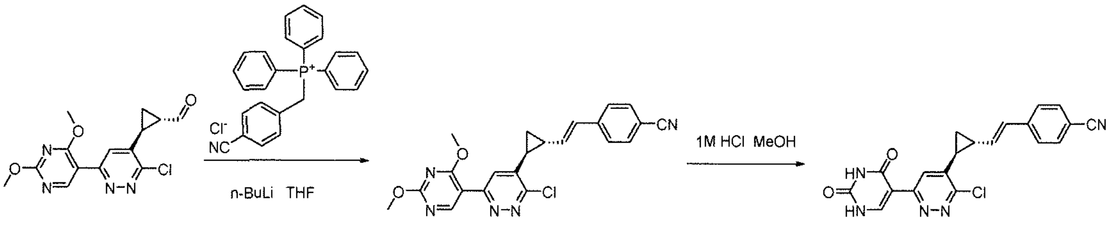

M8 17-1 17

Step 1: Synthesis of compound 17-1

(4-Cyanobenzyl)triphenylphosphonium chloride (180mg) and anhydrous THF(5mL) were added into a 25mL three-necked flask. 2.5M n-butyl lithium solution (0.3mL) was slowly dropped under the ice bath and the protection of $N_2$, the reaction mixture was stirred at 0°Cfor 10min. Then compound M8(100mg) was added and the reaction was maintained for 1h at room temperature. After completion of the reaction, saturated ammonium chloride solution was added to the reaction solution, the reaction solution was extracted with ethyl acetate, the organic phase was washed with saturated aqueous NaCl, dried and concentrated to obtain the crude product. The crude product was purified by column chromatography to obtain a white solid (40 mg, 30.55% yield), which was compound 17-1.

Step 2: Synthesis of compound 17

Compound 17-1 (40 mg), methanol (4 mL), water (4 mL), and concentrated hydrochloric acid (0.5 mL) were added into a 25 mL three-necked flask and the reaction mixture was stirred at room temperature for 72h. After completion of the reaction, the reaction solution was lyophilized to obtain the crude product, the crude product was purified by preparative liquid chromatography to obtain white solid (2 mg, 5.36% yield), which was compound 17 (purity 99.23%).
LCMS: $[M+H]^+$=392.2

**Example 18: Synthesis of compound 18 5-(6-chloro-5-((*1S*,*2S*)-2-(phenylethynyl)cyclopropyl)pyridazin-3-yl)pyrimidine-2,4(*1H*,*3H*)-dione**

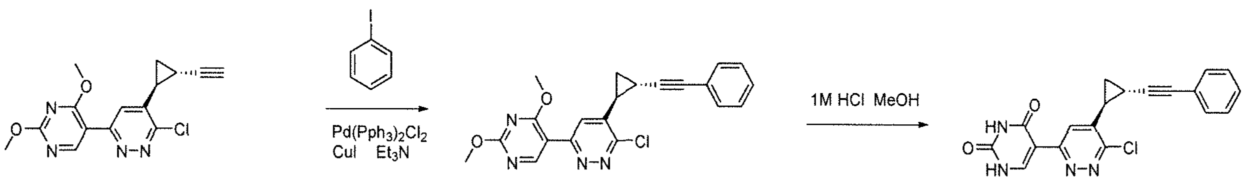


M9 18-1 18

Step 1: Synthesis of compound 18-1

Compound M9 (180 mg), iodobenzene (140 mg), bistriphenylphosphine dichloride (20 mg), cuprous iodide (10mg), triethylamine (5 mL) and THF(2 mL) were added into a 25ml three-necked flask, and the reaction mixture was stirred at room temperature for 12h under nitrogen protection. After completion of the reaction, the reaction solution was quenched with saturated ammonium chloride, extracted by EA and purified by Pre-TLC to obtain a yellow solid (50 mg, 22.40% 5 yield), which was compound 18-1.

Step 2: Synthesis of compound 18

Compound 18-1 (50 mg), methanol (4 mL), water (4 mL) and concentrated hydrochloric acid (0.5 mL) were added into a 25 mL single-mouth flask and the reaction mixture was stirred at 40°C for 24h. After completion of the reaction, the reaction solution was filtered to obtain the crude product. The crude product was purified by pre-HPLC to obtain a white solid (6.6 mg, 12.93% yield), which was compound 18 (99.62% purity).
LCMS: $[M+H]^+$=365.2
$^1$H NMR (500 MHz, DMSO-$d_6$) δ 8.36 (s, 1H), 8.04 (s, 1H), 7.39 (d, J = 32.8 Hz, 5H), 2.01 (d, J = 30.8 Hz, 1H), 1.56 (d, J = 33.6 Hz, 2H).

**Example 19: Synthesis of compound 19 3-(((*1S,2S*)-2-(3-chloro-6-(2,4-dioxo-1,2,3,4-tetrahydropyrimidin-5-yl)pyridazin-4-yl)cyclopropyl)ethynyl)benzonitrile**

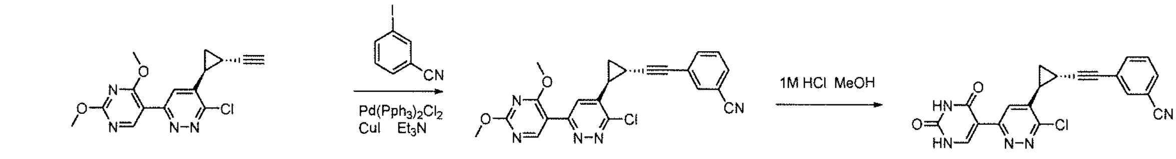


M9 19-1 19

Step 1: Synthesis of compound 19-1

Compound M9 (100 mg), 3-cyanoiodobenzene (90 mg), biphenylphosphine palladium dichloride (20 mg), cuprous iodide (10 mg), triethylamine (5 mL)and DMF(2 mL) were added into a 25ml three-necked flask, and the reaction mixture was stirred at room temperature for 12h under nitrogen protection. The reaction solution was quenched with saturated ammonium chloride, extracted by EA, and purified by Pre-TLC to obtain a light yellow solid (100 mg, 75.81%, 20 yield), which was compound 19-1.

Step 2: Synthesis of compound 19

Compound 19-1 (100 mg), THF(5 mL) and 1M hydrochloric acid (5 mL) were added into a 25 mL single-mouth flask and the reaction mixture was stirred at 40°C for 24h. The reaction solution was filtered, and the solid was slurried with a mixture of acetonitrile and methylene chloride to obtain a white solid (56.6 mg, 60.20% yield), which was compound 19 (99.59% purity).

LCMS: $[M+H]^+$=390.2

$^1$H NMR (500 MHz, DMSO-$d_6$) δ 11.64 - 11.56 (m, 2H), 8.32 (d, J = 6.2 Hz, 1H), 8.04 (s, 1H), 7.93 (t, J = 1.8 Hz, 1H), 7.83 (dt, J = 7.8, 1.5 Hz, 1H), 7.76 (dt, J = 7.9, 1.4 Hz, 1H), 7.58 (t, J = 7.8 Hz, 1H), 2.60 (ddd, J = 8.8, 6.4, 4.7 Hz, 1H), 2.14 - 2.07 (m, 1H), 1.64 (dt, J = 8.6, 5.3 Hz, 1H), 1.57 (ddd, J = 8.8, 6.5, 4.9 Hz, 1H).

**Example 20: Synthesis of compound 20 5-(6-methoxy-5-((*1S,2R*)-2-((*E*)-styryl)cyclopropyl)pyridazin-3-yl)pyrimidine-2,4(*1H,3H*)-dione**

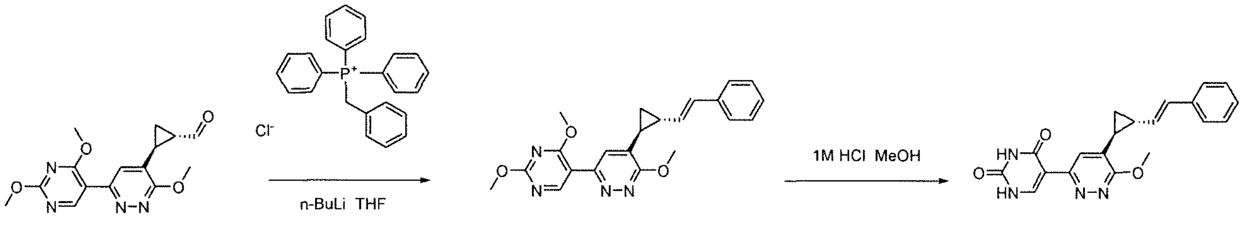


M10 20-1 20

Step 1: Synthesis of compound 20-1

Benzyl triphenyl phosphorus chloride (250mg) and anhydrous THF(5mL) were added into a 25mL three-necked flask, n-butyl lithium solution (2.5M , 0.25mL) was slowly dropped under ice bath and the protection of $N_2$, and the reaction mixture was stirred under the ice bath for 10min. Then compound M10(100mg) was added and the reaction solution was maintained for 1h at room temperature. Saturated ammonium chloride solution was added to the reaction solution, the reaction solution was extracted by ethyl acetate, the organic phase was washed with saturated aqueous NaCl, dried and concentrated to obtain the crude product, and the crude product was purified by column chromatography to obtain a white solid (95 mg, 76.99% yield), which was compound 20-1.

Step 2: Synthesis of compound 20

Compound 20-1 (40 mg), methanol (4 mL), water (4 mL) and concentrated hydrochloric acid (0.5 mL) were added to a 25ml three-necked flask and the reaction mixture was stirred at room temperature for 72h. The reaction solution was filtered to obtain a pale yellow solid (20 mg, 21.55% yield), which was compound 20 (purity 97.39%).

LCMS: $[M+H]^+$=363.2

$^1H$ NMR (500 MHz, DMSO-$d_6$) δ 11.71 (s, 1H), 11.64 (s, 1H), 8.27 (d, J = 6.1 Hz, 1H), 7.81 (s, 1H), 7.38 (d, J = 7.5 Hz, 2H), 7.31 (t, J = 7.7 Hz, 2H), 7.20 (td, J = 7.1, 1.5 Hz, 1H), 6.57 (d, J = 15.9 Hz, 1H), 6.12 (dd, J = 15.9, 8.9 Hz, 1H), 4.08 (s, 4H), 2.33 (ddd, J = 9.1, 5.7, 4.0 Hz, 1H), 2.16 - 2.06 (m, 1H), 1.58 (dt, J = 9.2, 5.3 Hz, 1H), 1.49 (dt, J = 8.6, 5.2 Hz, 1H).

**Example 21: Synthesis of compound 21 5-(6-methoxy-5-((*1S,2S*)-2-(phenylethynyl)cyclopropyl)pyridazin-3-yl)pyrimidine-2,4(*1H,3H*)-dione**

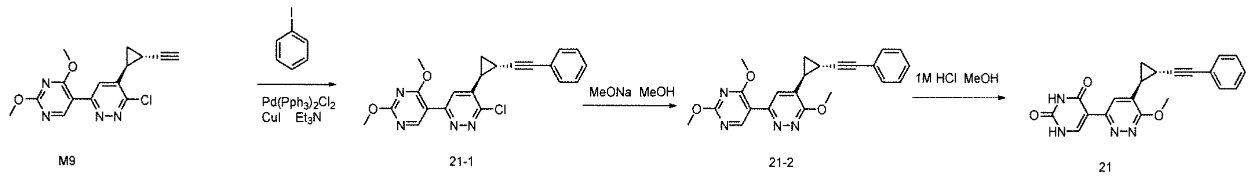


M9 21-1 21-2 21

Step 1: Synthesis of Compound 21-1

Compound M9 (180 mg), iodobenzene (140 mg), biphenylphosphine palladium dichloride (20 mg), cuprous iodide (10mg), triethylamine (5 mL) and THF(2 mL) were added to a 25ml three-necked flask, and the reaction mixture was stirred at room temperature for 12h under nitrogen protection.The reaction solution was quenched with saturated ammonium chloride, and extracted by EA to obtain the organic phase, then purified by Pre-TLC to give a yellow solid (50 mg, 22.40% yield), which was compound 21-1.

Step 2: Synthesis of Compound 21-2

Compound 21-1 (50 mg), sodium methanol (100 mg) and methanol (4 mL) were added into 25 mL sealed tube and the reaction mixture was stirred at 60°C for 12h. The reaction solution was concentrated and water was added. The mixture was extracted by EA, the organic phase was dried and concentrated to produce a white solid (45 mg, 90.94% yield), which was compound 21-2.

Step 3: Synthesis of compound 21

Compound 21-2 (45 mg), methanol (4 mL), water (4 mL) and concentrated hydrochloric acid (0.5 mL) were added into a 25 mL single-mouth flask and the reaction mixture was stirred at 40°C for 24h. the reaction mixture was filtered and slurried with methylene chloride to obtain a light yellow solid (9.9mg, 22.55% yield), which was compound 21 (97.34% purity).

LCMS: $[M+H]^+$=361.2

$^1H$ NMR (500 MHz, DMSO-$d_6$) δ 11.45 (s, 2H), 8.09 (s, 1H), 7.75 (s, 1H), 7.48 - 7.39 (m, 2H), 7.39 - 7.30 (m, 3H), 4.10 (s, 3H), 2.01 (dd, J = 12.5, 6.3 Hz, 1H), 1.50 (t, J = 7.4 Hz, 2H).

**Example 22: Synthesis of compound 22 5-(6-methyl-5-(2-phenylcyclopropyl)pyridazin-3-yl)pyrimidine-2,4(*1H,3H*)-dione**

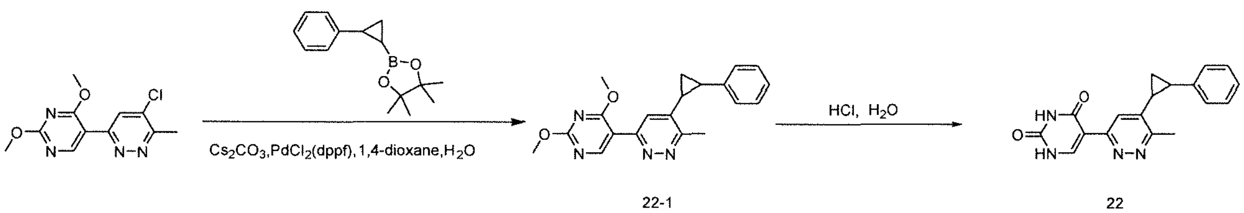


22-1 22

Step 1: Synthesis of compound 22-1

4-Chloro-6 - (2,4-dimethoxypyrimidin-5-yl) - 3-methylpyridazine (0.06 g) and compound M3 (0.06 g) were dissolved in the mixture of 1, 4-dioxane (10 mL) and water (2 mL) in a 50mL single-mouth flask, $Cs_2CO_3$ (0.15 g) (0.15g) and $PdCl_2$(dppf) (0.01g) were added, and the reaction mixture was stirred at 70°C for 2h under $N_2$ protection The reaction solution was cooled to room temperature, saturated aqueous NaCl (30 mL) was added and extracted by EA, the organic phase was dried with anhydrous sodium sulfate, and separated by pre-TLC to obtain white solid (15 mg, 19.13% yield), which was compound 22-1.

Step 2: Synthesis of compound 22

Compound 5-1 (15 mg) was added to a 25mL single-mouth bottle and dissolved in 1mol/L hydrochloric acid (3 mL), the reaction mixture was stirred at 70°C for 3h. The reaction solution was cooled to room temperature, filtered to obtain filter cake. The filter cake was slurried with acetonitrile and DCM to obtain a white solid (4.9mg, 35.52% yield), which was compound 22 (97.83% purity).

LCMS: $[M+H]^+$= 321.2

$^1$H NMR (500 MHz, DMSO-$d_6$) δ 12.04 - 11.93 (m, 1H), 11.73 (s, 1H), 8.43 (d, J = 5.2 Hz, 1H), 8.21 (s, 1H), 7.37 - 7.18 (m, 5H), 3.05 (qd, J = 7.3, 4.7 Hz, 1H), 2.72 (s, 3H), 2.43 (t, J = 7.4 Hz, 2H).

**Example 23: Synthesis of compound 23 5-(6-chloro-5-(2-phenylcyclopropyl)pyridazin-3-yl)pyrimidine-2,4(*1H,3H*)-dione**

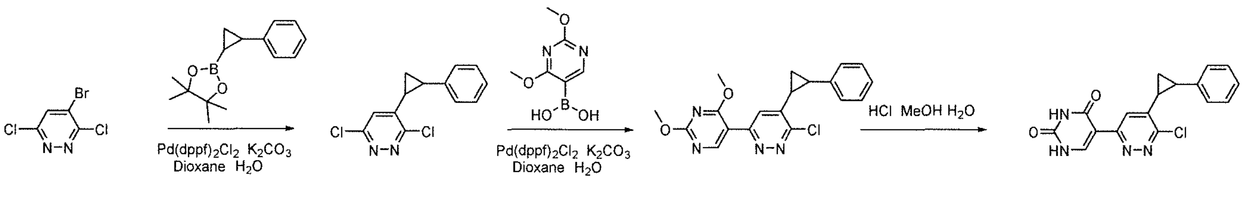


23-1 23-2 23

Step 1: Synthesis of compound 23-1

4-bromo-3, 6-dichloroprazine (0.54 g), compound M5 (0.86 g), [1,1 '- bis (diphenylphosphine) ferrocene] palladium dichloride dichloromethane complex (0.18 g), potassium carbonate (0.97 g), dioxane (5 mL) and water (1 mL) were added into 50ml three-necked flask. The reaction mixture was stirred at 60°C for 1h under $N_2$ protection. The reaction solution was poured into water, extracted by ethyl acetate, washed with saturated aqueous NaCl, dried with anhydrous $Na_2SO_4$, and concentrated to obtain the crude product. The crude product was purified by column chromatography to obtain a white solid (0.39 g, 60.16% yield), which was compound 23-1.

Step 2: Synthesis of compound 23-2

Compound 23-1(0.38g), (2,4-Dimethoxypyrimidin-5-yl) boric acid (0.31g), [1,1' -bis (diphenylphosphine) ferrocene] palladium dichloride dichloromethane complex (0.06g), potassium carbonate (0.59g), dioxane (520mL) and water (1mL) were added in a 50 mL three-necked flask, The reaction mixture was stirred at 60°C for 30min under $N_2$ protection. The reaction solution was poured into water, extracted with ethyl acetate, washed with saturated aqueous NaCl, dried with anhydrous $Na_2SO_4$ and concentrated to obtain the crude product, which was purified by column chromatography to obtain the white solid crude product, the white solid crude product was slurried with methanoland and filtered to obtain a white solid (0.2g,37.83%yield), which was compound 23-2.

Step 3: Synthesis of compound 23

Compound 23-2 (200 mg), 1M hydrochloric acid aqueous solution (5 mL), and methanol (5 mL) were added to a 25 mL single-mouth flask and the reaction mixture was stirred at 50°Cfor 24h. The reaction solution was filtered, and the filter cake was slurried with a small amount of methanol to obtain a white solid (72.9 mg, 39.13% yield), which was compound 23 (purity 95.73%).

LCMS: $[M+H]^+$= 341.2

$^1$H NMR (500 MHz, DMSO-$d_6$) δ 11.59 (dd, J = 9.7, 4.2 Hz, 2H), 8.33 (d, J = 6.2 Hz, 1H), 8.07 (s, 1H), 7.32 (t, J = 7.6 Hz, 2H), 7.28 - 7.25 (m, 2H), 7.24 - 7.20 (m, 1H), 2.37 - 2.31 (m, 2H), 1.71 (dt, J = 8.4, 5.7 Hz, 1H), 1.64 (dt, J

= 9.1, 5.6 Hz, 1H).

**Example 24: Synthesis of compound 24 5-(6-chloro-5-(2-(p-tolyl)cyclopropyl)pyridazin-3-yl)pyrimidine-2,4(*1H,3H*)-dione**

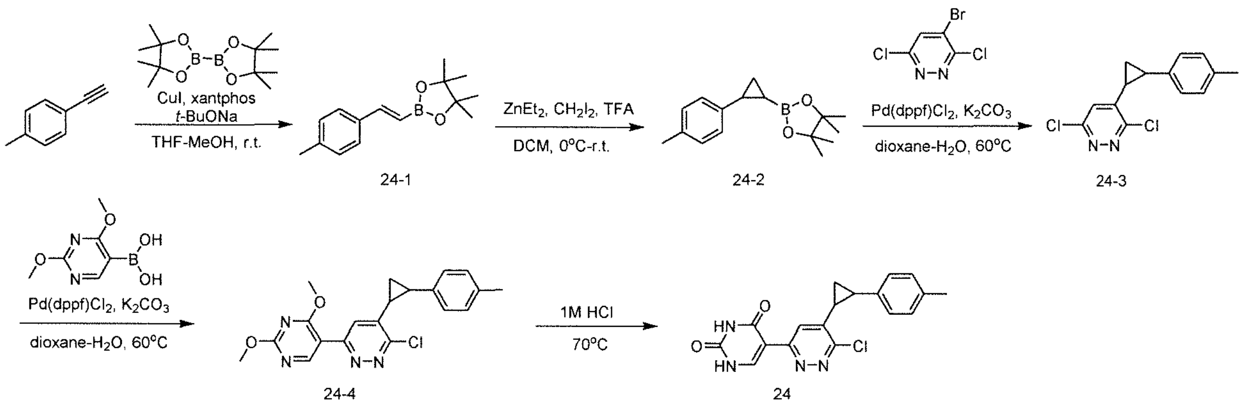

24-1 24-2 24-3 24-4 24

Step 1: Synthesis of compound 24-1

Bis(pinacolato)diboron (2.84 g), CuI (164 mg), xantphos (0.5 g) were added into 100 ml three-necked flask, dissolved in THF (20mL), The reaction mixture was stirred at room temperature for 10 min under $N_2$ protection, and p-methylphenylacetylene (1.0 g) was then added into the reaction solution. The reaction mixture was stirred for 5 minutes, sodium tert-butanol (1.65g) was added , and finally methanol (4 mL) was added to the reaction solution. The reaction mixture was stirred at room temperature for 2 hours. The reaction solution was concentrated to obtain the crude product, the crude productwas separated by column chromatography (PE:EA=10:1) to obtain 2.0 g light yellow liquid, which was compound 24-1.

Step 2: Synthesis of compound 24-2

Dry DCM(10mL) and diethyl zinc (35mL) were added in 250 mL three-necked flask under nitrogen protection and ice bath, then slowly dropped TFA(2.7mL) to the reaction solution. The reaction mixture was stirred under ice bath for 30 minutes, diiodomethane (9.9 g) was added, the reaction mixture was stirred under the ice bath for 30 minutes, and then compound 24-1(1.5g) was added to the reaction solution. The reaction solution was slowly raised to room temperature and stirred for 24h.The reaction solution was quenched by adding saturated ammonium chloride solution, extracted by EA for three times.The organic phase was washed with water and saturated aqueous NaCl successively, and then concentrated to obtain crude product. The crude product was separated by column chromatography (PE:EA=10:1)to obtain 1.0 g light yellow oil, which was compound 24-2.

Step 3: Synthesis of compound 24-3

Compounds 24-2 (0.5 g), 4-bromo-3, 6-dichloroprazine (0.53 g), $K_2CO_3$ (0.53g), Pd(dppf)Ch-$CH_2Cl_2$ (158 mg) were mixed in dioxane (20 mL) and water (4 mL). Under nitrogen protection, the reaction mixture was stirred at 60°C for 4 h. The reaction solution was dried by rotary evaporation to obtain the crude product. The crude product was separated and purified by column chromatography (PE: EA = 1:1) to obtain150 mg light yellow oil, which was compound 24-3.

Step 4: Synthesis of compound 24-4

Compounds 24-3 (150 mg), 2, 4-dimethoxypyrimidine-5-boric acid (120 mg), $K_2CO_3$ (150 mg), Pd(dppf)$Cl_2$-$CH_2Cl_2$ (44 mg) were mixed in dioxane (10 mL) and water (2 mL) Under nitrogen protection, the reaction mixture was stirred at 60°C for 4 h. The reaction solution was concentrated. The crude product was separated and purified by column chromatography (PE: EA = 1:1) to obtain 50 mg white solid, which was compound 24-4.

Step 5: Synthesis of compound 24

Compound 24-4 (50 mg) was added to 25 mL single-mouth flask, dissolved in 1M hydrochloric acid (4 mL), the reaction mixture was stirred at 70°C for 12 h.The pH of the reaction solution was adjusted to neutral by saturated sodium bicarbonate solution. The reaction solution was concentrated. The crude product was separated and purified by column chromatography (DCM:MeOH = 10:1) to obtain 10 mg white solid , which was compound 24 (purity 99.16%).

LCMS: $[M+H]^+$= 355.2

**Example 25: Synthesis of compound 25**

**4-(2-(3-chloro-6-(2,4-dioxo-1,2,3,4-tetrahydropyrimidin-5-yl)pyridazin-4-yl)cyclopropyl)benzonitrile**

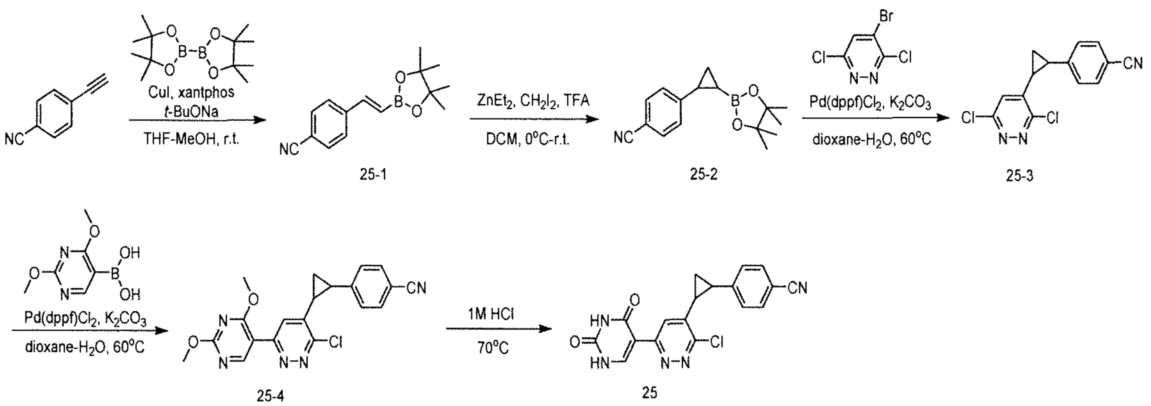


Step 1: Synthesis of compound 25-1

Bis(pinacolato)diboron (2.84 g), CuI (164 mg), xantphos (0.5 g) were added into 100 ml three-necked flask, dissolved in THF (20mL), the reaction mixture was stirred at room temperature for 10 min under $N_2$ protection, then p-cyanophenyl acetylene (1.0 g) was added into the reaction solution. After stirring for 5 minutes, sodium tert-butanol (1.65g) was added to the reaction solution, and finally methanol (4 mL) was added to the reaction solution. The reaction mixture was stirred for 2 hours at room temperature. The reaction solution was concentrated, The crude product was separated by column chromatography (PE:EA=10:1) to obtain 1.0g yellow solid, which was compound 25-1.

Step 2: Synthesis of compound 25-2

Dry DCM(10mL) and diethyl zinc (30mL)were added in a 250ml three-neck flask under nitrogen protection and ice bath, then TFA(2.0mL) was slowly dropped to the reaction solution, the reaction mixture was stirred for 30 minutes under ice bath, then diiodomethane (9.0g) was added to the reaction solution,the reaction was continued under the ice bath for 30 minutes, and then compound 25-1(1.0g) was added to the reaction solution. The reaction mixture was slowly raised to room temperature and stirred for 24h.The reaction solution was quenched by adding saturated ammonium chloride solution, extracted by EA for three times. The organic phase was washed with water and saturated aqueous NaCl successively, and then concentrated to obtain crude product, the crude product was separated by column chromatography (PE:EA=10:1)to obtain 0.46g of light yellow oil, which was compound 25-2.

Step 3: Synthesis of compound 25-3

Compounds 25-2 (0.46 g), 4-bromo-3, 6-ichloropyridazine (0.53 g), $K_2CO_3$ (0.53 g), $Pd(dppf)Cl_2$-$CH_2Cl_2$ (158 mg) were mixed in dioxane (20 mL) and water (4 mL). Under nitrogen protection, the reaction mixture was stirred at 60°C for 4 h. The reaction solution was concentrated to obtain the crude product. The crude product was separated and purified by column chromatography (PE: EA = 1:1) to obtain 270 mg light yellow oil, which was compound 25-3.

Step 4: Synthesis of compound 25-4

Compounds 25-3 (270 mg), 2,4-dimethoxypyrimidin-5-boric acid (170 mg), $K_2CO_3$ (150 mg),

$Pd(dppf)Cl_2$-$CH_2Cl_2$ (76 mg) were mixed in dioxane (10 mL) and water (2 mL) under nitrogen protection. The reaction mixture was stirred at 60°C for 4 h. The reaction solution was concentrated to obtain crude product. The crude product was separated and purified by column chromatography (PE: EA = 1:1) to obtain 200 mg light yellow oil, which was compound 25-4.

Step 5: Synthesis of compound 25

Compound 25-4 (200 mg) was added to 25 mL single-mouth bottle, dissolved in 1M hydrochloric acid (4 mL), and the reaction mixture was stirred at 40°C for 12 h.The pH of the reaction solution was adjusted to neutral by saturated sodium bicarbonate solution. The reaction solution was concentrated to obtain the crude product. The crude product was separated and purified by column chromatography (DCM:MeOH = 10:1) to obtain 50 mg white solid, which was compound 25 (purity 99.16%).

LCMS: $[M+H]^+$= 366.2

$^1H$ NMR (500 MHz, DMSO-$d_6$) δ 11.44 (s, 2H), 8.37 (s, 1H), 8.12 (s, 1H), 7.78 (d, J= 8.1 Hz, 2H), 7.48 (d, *J*= 8.2 Hz, 2H), 2.47 - 2.41 (m, 2H), 1.81-1.72 (m, 2H).

**Example 26: Synthesis of compound 26**

**3-(2-(3-chloro-6-(2,4-dioxo-1,2,3,4-tetrahydropyrimidin-5-yl)pyridazin-4-yl)cyclopropyl)benzonitrile**

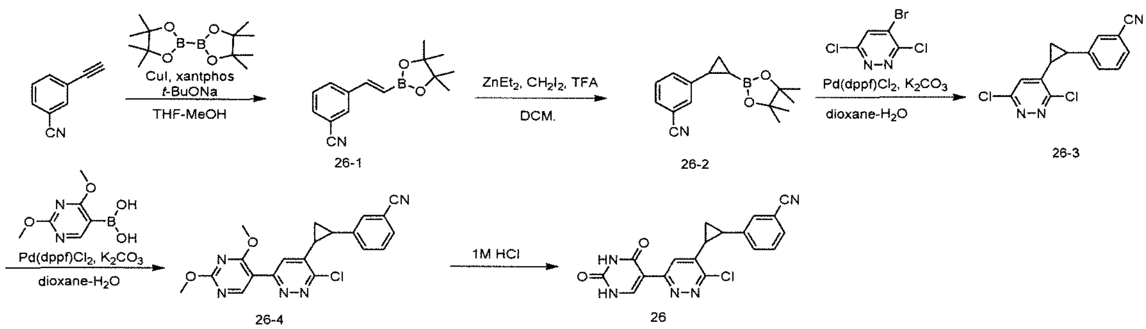


Step 1: Synthesis of compound 26-1

Bis(pinacolato)diboron (4.79g), CuI(300mg), xantphos(0.5g) were added into 100ml three-necked flask, dissolved in THF(40mL), the reaction mixture was stirred at room temperature for 10 min under $N_2$ protection, and m-cyanophenylacetylene (1.0g) was then added into the reaction solution, continue stirring for 5min.Then sodium tert-butanol (3.02g) was added, and finally methanol (10mL) was added to the reaction solution, and the reaction was carried out for 2 hours at room temperature.The reaction solution was diluted with ethyl acetate, and filtered with diatomaceous earth.The filtrate was concentrated to obtain the crude product. The crude product was separated by column chromatography (PE:EA=10:1) to obtain 2.6 g colorless transparent oil, which was compound 26-1.

Step 2: Synthesis of compound 26-2

Dry DCM(50mL) was added to a 250mL three-necked flask, protected by nitrogen, cooled under an ice bath, then diethylzinc (61mL, 1Minhexane) was added, and TFA(4.5mL) was slowly dropped to the reaction solution. The reaction mixture was stirred under the ice bath for 30 minutes, and then compound 26-1(2.6g) was added to the reaction solution. The reaction solution was slowly raised to room temperature and stirred for 24h.The reaction solution was quenched by adding saturated ammonium chloride solution, extracted by EA for three times.The organic phase was washed with water and saturated aqueous NaCl successively, and then concentrated to obtain crude product. The crude product was separated by column chromatography (PE:EA=10:1)to obtain 0.6 g light yellow oily substance,which was compound 26-2.

Step 3: Synthesis of compound 26-3

Compounds 26-2 (0.6 g), 4-bromo-3, 6-dichloroprazine (0.45 g), $K_2CO_3$ (0.41 g), $Pd(dppf)Cl_2$-$CH_2Cl_2$ (144.5mg)

were mixed in dioxane (5 mL) and water (1 mL).The reaction mixture was stirred at 90°C for 12 h under nitrogen protection. The organic phase was washed with saturated aqueous NaCl and dried with anhydrous sodium sulfate to obtain the crude product. The crude product was separated and purified by column chromatography (PE: EA = 3:1) to obtain 310 mg light yellow oil, which was compound 26-3.

Step 4: Synthesis of compound 26-4

Compound 26-3 (310 mg), 2, 4-dimethoxypyrimidin -5-boric acid (490 mg), $K_2CO_3$ (350 mg), and Pd(dppf)$Cl_2$-$CH_2Cl_2$ (76 mg) were mixed in dioxane (10 mL) and water (2 mL) , the reaction mixture was stirred at 60°Cfor 4 h under nitrogen protection.The reaction solution was diluted with water, extracted with ethyl acetate for three times. The organic phase was washed with saturated brine, and dried with anhydrous sodium sulfate to obtain the crude product. The crude product was separated and purified by column chromatography (PE: EA = 2:1) to obtain 40 mg light yellow oil, which was compound 26-4.

Step 5: Synthesis of compound 26

Compound 26-4 (40 mg) was added to 25 mL single-mouth flask, dissolved in 1M hydrochloric acid (8 mL), and the reaction mixture was stirred at 40°C for 12 h.The pH of the reaction solution was adjusted to neutral by saturated sodium bicarbonate solution. The reaction solution was concentrated to obtain the crude product. The crude product was separated and purified by column chromatography (DCM:MeOH = 10:1)to obtain 11 mg white solid, which was compound 26 (purity 94.97%).
LCMS: $[M+H]^+$= 366.2

**Example 27: Synthesis of compound 27 5-(6-chloro-5-(2-(4-fluorophenyl)cyclopropyl)pyridazin-3-yl)pyrimidine-2,4(*1H,3H*)-dione**

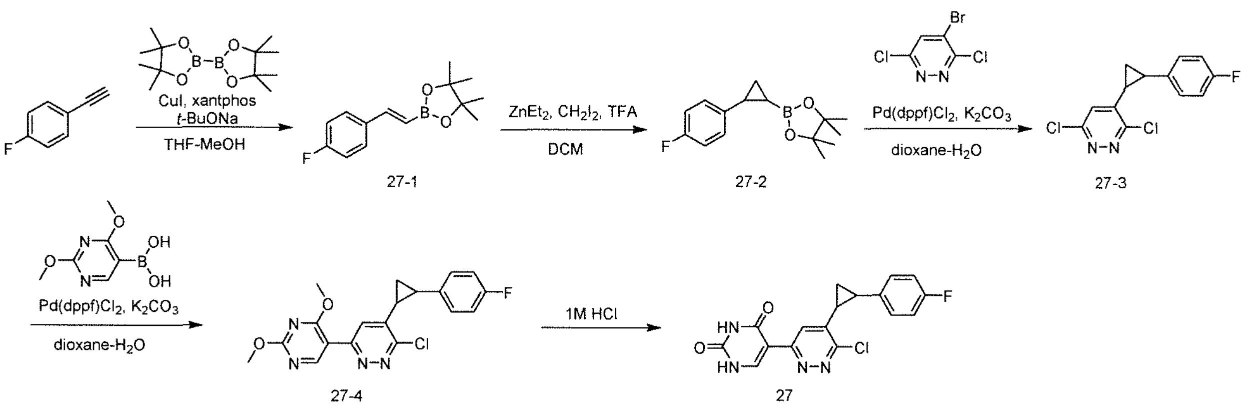


Step 1: Synthesis of compound 27-1

Bis(pinacolato)diboron (2.54 g), CuI (150 mg), xantphos (0.45 g) were added into 100 ml three-necked flask, dissolved in THF (40mL), the reaction mixture was stirred at room temperature for 10 min under $N_2$ protection, then p-fluoropheneacetylene (1.0 g) was added into the reaction solution and continue stirring for 5 minutes, then sodium tert-butanol (1.5 g) was added to the reaction solution, and finally methanol (8 mL) was added to the reaction solution, the reaction mixture was stirred for 2 hours at room temperature. The reaction solution was concentratedto obtain the crude product. The crude product was separated by column chromatography (PE:EA=10:1) to obtain 1.7 g light yellow oil, which was compound 27-1.

Step 2: Synthesis of compound 27-2

Dry DCM(30mL) and diethyl zinc (20mL) were added into 250mL three-necked flask under nitrogen protection and cooling under ice bath, then TFA(1.5mL)was slowly added to the reaction solution, the reaction mixture was stirred for 30min under ice bath, then diiodomethane (1.65mL) was dropped to the reaction solution.The reaction mixture was stirred under the ice bath for 30min, and then compound 27-1(1.7g) was added to the reaction solution. The reaction

solution was slowly raised to room temperature and stirred for 48h.The reaction solution was quenched by adding saturated ammonium chloride solution, extracted by EA for three times. The organic phase was washed with water and saturated aqueous NaCl successively, and then concentratedto obtain crude product. The crude product was separated by column chromatography (PE:EA=10:1)to obtain 0.9g opalescence oil, which was compound 27-2.

Step 3: Synthesis of compound 27-3

Compounds 27-2 (0.9 g), 4-bromo-3, 6-dichloroprazine (1.4 g), $K_2CO_3$ (0.64 g), Pd(dppf)$Cl_2$-$CH_2Cl_2$ (250 mg) were mixed in dioxane (20 mL) and water (4 mL). Under nitrogen protection, the reaction mixture was stirred at 90 °C for 12 h. The reaction solution was concentratedto obtain the crude product. The crude product was separated and purified by column chromatography (PE: EA = 5:1) to obtain 500 mg white solid, which was compound 27-3.

Step 4: Synthesis of compound 27-4

Compounds 27-3 (500 mg), 2, 4-dimethoxypyrimidin -5-boric acid (330 mg), $K_2CO_3$ (300 mg), Pd(dppf)$Cl_2$-$CH_2Cl_2$ (150 mg) were mixed in dioxane (20 mL) and water (1 mL) under nitrogen protection.The reaction mixture was stirred at 60°C for 12 h. The reaction solution wasconcentrated to obtain the crude product. The crude product was separated and purified by column chromatography (PE: EA = 1:1) to obtain 300 mg white solid, which was compound 27-4.

Step 5: Synthesis of compound 27

Compound 27-4 (300 mg) was added to 25 mL single-mouth flask, dissolved in 1M hydrochloric acid (10 mL) , the reaction mixture was stirred at 70°C for 12 h.The pH of the reaction solution was adjusted to neutral by saturated sodium bicarbonate solution. After filtration, the filter cake was dried and 200 mg of white solid was obtained, which was compound 27 (purity 99.16%).

LCMS: $[M+H]^+$= 359.2

**Example 28: Synthesis of compound 28 5-(6-chloro-5-(2-(4-chlorophenyl)cyclopropyl)pyridazin-3-yl)pyrimidine-2,4(*1H,3H*)-dione**

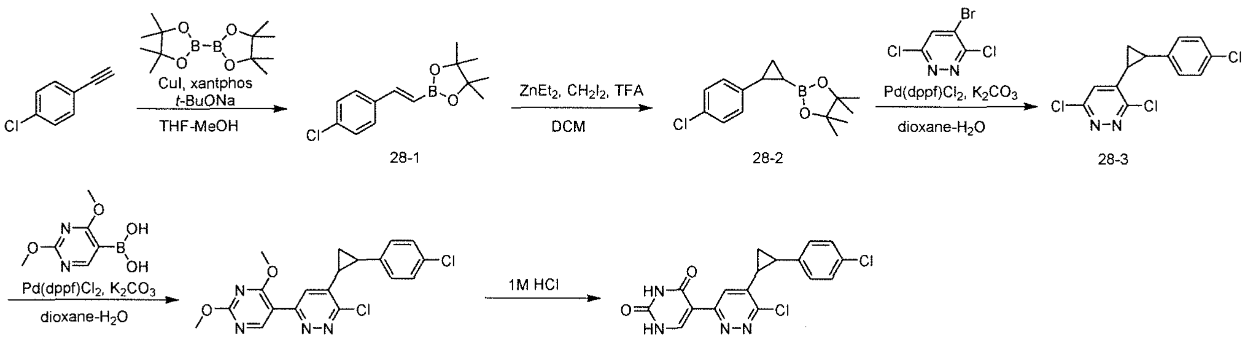


28-1 28-2 28-3 28-4 28

Step 1: Synthesis of compound 28-1

Bis(pinacolato)diboron (2.23g), CuI (150 mg), xantphos (0.45g) were added to 100 ml three-necked flask, dissolved in THF (40mL), the reaction mixture was stirred at room temperature for 10 min under $N_2$ protection,and then p-chloropheneacetylene (1.0g) was added to the reaction solution,continue stirring for 5 minutes, then sodium tert-butanol (1.5 g)was added to the reaction solution, and finally methanol (8 mL) was added to the reaction solution, the reaction mixture was stirred at room temperature for 12 hours. The reaction solution was concentrated to obtain the crude product. The crude product was separated by column chromatography (PE:EA=10:1) to obtain 1.6g light yellow oil, which was compound 28-1.

Step 2: Synthesis of compound 28-2

Dry DCM(30mL) and diethyl zinc (17mL) were added in 250mL three-necked flask under nitrogen protection and ice bath, then TFA(1.26mL) was slowly dropped to the reaction solution, the reaction mixture was stirred under ice bath for 30min, then diiodomethane (1.37mL) was added to the reaction solution, the reaction was continued under the ice bath for 30min, and then the compound 28-1(1.5g) was added to the reaction solution, the reaction mixture was slowly raised to room temperature and stirred for 24h.The reaction solution was quenched by adding saturated ammonium chloride solution, extracted by EA for three times.The organic phase was washed with water and saturated aqueous NaCl successively, and then concentrated to obtain crude product. The crude product was separated by column chromatography (PE:EA=10:1)to obtain 1.0 milky oil,which was compound 28-2.

Step 3: Synthesis of compound 28-3

Compounds 28-2 (1.0 g), 4-bromo-3, 6-dichloroprazine (1.53 g), $K_2CO_3$ (0.64 g), Pd(dppf)$Cl_2$-$CH_2Cl_2$ (250 mg) were mixed in dioxane (20 mL) and water (4 mL). The reaction mixture was stirred at 90 °C to react for 12h under nitrogen protection. The reaction solution was concentrated to obtain crude product. The crude product was isolated and purified by column chromatography (PE: EA = 3:1) to obtain 510 mg white solid, which was compound 28-3.

Step 4: Synthesis of compound 28-4

Compounds 28-3 (510 mg), 2, 4-dimethoxypyrimidin -5-boric acid (400 mg), $K_2CO_3$ (340 mg), Pd(dppf)$Cl_2$-$CH_2Cl_2$ (170 mg) were mixed in dioxane (20 mL) and water (1 mL) under nitrogen protection. The reaction mixture was stirred at 60°C for 12 h.The reaction solution was concentrated to obtain the crude product. The crude product was separated and purified by column chromatography (PE: EA = 1:1) to obtain 350 mg white solid, which was compound 28-4.

Step 5: Synthesis of compound 28

Compound 28-4 (350 mg) was added to 25 mL single-mouth flask, dissolved in 1M hydrochloric acid (10 mL) , the reaction mixture was stirred at 70°C for 12 h.The pH of the reaction solution was adjusted to neutral by saturated sodium bicarbonate solution, the reaction solution was filtered and the filter cake was dried to obtain150 mg white solid, which was compound 28 (purity 99.16%).

LCMS: $[M+H]^+$= 375.2

$^1$H-NMR (500 MHz, DMSO-$d_6$) δ 11.60 (d, *J* = 11.6 Hz, 2H), 8.20 (d, *J*= 130.8 Hz, 2H), 7.34 (dd, *J*= 33.1, 8.4 Hz, 4H), 2.42 - 2.31 (m, 2H), 1.72 - 1.63 (m, 2H).

**Example 29: Synthesis of compound 29 5-(6-chloro-5-(2-(3-methoxyphenyl)cyclopropyl)pyridazin-3-yl)pyrimidine-2,4(*1H,3H*)-dione**

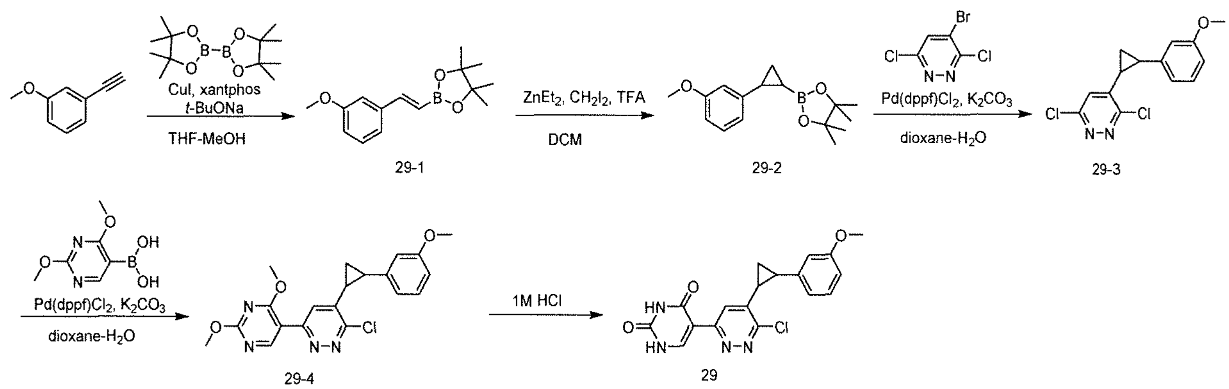


Step 1: Synthesis of compound 29-1

Bis(pinacolato)diboron (2.31 g), CuI (144 mg), xantphos (0.44 g) were added to 100 ml three-necked flask,

dissolved in THF (40mL), the reaction mixture was stirred at room temperature for 10 min under $N_2$ protection, and then m-methoxy-phenylacetylene (1.0 g) was added to the reaction solution ,continue stirring for 5 minutes, then sodium tert-butanol (1.45g) was added to the reaction solution, and finally methanol (8 mL) was added to the reaction solution, the reaction mixture was stirred for 2 hours at room temperature. The reaction solution was concentratedto obtain the crude product. The crude product was separated and purified by column chromatography (PE:EA=10:1) to obtain 1.68 g of light yellow oil, which was compound 29-1.

Step 2: Synthesis of compound 29-2

Dry DCM(30mL) and 1M diethyl zinc (5.8mL) were added in three-necked flask under nitrogen protection and ice bath, then TFA(0.959mL) was slowly dropped to the reaction solution, the reaction mixture was stirred under ice bath for 30 minutes, then diiodomethane (3.46g) was dropped to the reaction solution, the reaction was continued under the ice bath for 30 minutes, and then compound 29-1(1.68g) was added to the reaction solution. The reaction mixture was slowly raised to room temperature and stirred for 24h.The reaction solution was quenched by adding saturated ammonium chloride solution, extracted by EA for three times.The organic phase was washed with water and saturated aqueous NaCl successively, and then concentratedto obtain crude product. The crude product was separated by by column chromatography (PE:EA=10:1) to obtain 0.92 milky oily substance, which was compound 29-2.

Step 3: Synthesis of compound 29-3

Compounds 29-2 (0.92 g), 4-bromo-3, 6-dichloroprazine (306 mg), $K_2CO_3$ (371mg), Pd(dppf)$Cl_2$-$CH_2Cl_2$ (37.2mg) were mixed into dioxane (10 mL) and water (1 mL). The reaction mixture was stirred at 60°C for 4 h under nitrogen protection.The reaction solution was concentrated to obtain the crude product. The crude product was separated and purified by by column chromatography (PE: EA = 5:1) to obtain 240 mg white solid, which was compound 29-3.

Step 4: Synthesis of compound 29-4

Compounds 29-3 (240 mg), 2, 4-dimethoxy-pyrimidine-5-boric acid (649 mg), $K_2CO_3$ (123 mg), Pd(dppf)$Cl_2$-$CH_2Cl_2$ (13.5 mg) were mixed in dioxane (10 mL) and water (1 mL).The reaction mixture was stirred at 60 °C for 5 h under nitrogen protection.The reaction solution was concentrated to obtain the crude product. The crude product was separated and purified by column chromatography (PE: EA = 1:1) to obtain 16 mg yellow solid, which was compound 29-4.

Step 5: Synthesis of compound 29

Compound 29-4 (16 mg) was added to 25 mL single-mouth bottle, dissolved in 1M hydrochloric acid (3 mL), the reaction mixture was stirred at 40°C for 15 h. The reaction solution was purified by pre-HPLC and lyophilized to obtain 2 mg white solid, which was compound 29 (96.27% purity).

LCMS: $[M+H]^+$= 371.2

**Example 30: Synthesis of compound 30 5-(6-chloro-5-(2-(naphthalen-1yl)cyclopropyl)pyridazin-3-yl)pyrimidine-2,4(*1H*,*3H*)-dione**

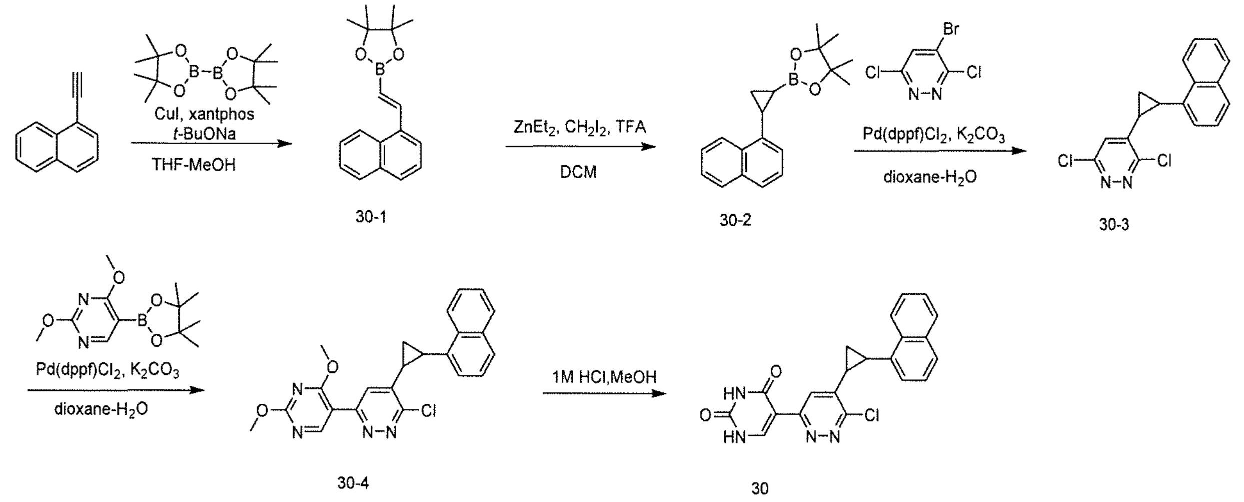


30-1 30-2 30-3 30-4 30

Step 1: Synthesis of compound 30-1

Bis(pinacolato)diboron (1.40g), CuI (90 mg), xantphos (0.27g) were added into 100 ml three-necked flask, dissolve in THF (20 mL), the reaction mixture was stirred at room temperature for 10 min under $N_2$ protection, and then 1-acetylene naphthalene (0.7 g)was added into the reaction solution,continue stirring for 5 minutes, then sodium tert-butanol (0.66 g) was added to the reaction solution, and finally methanol (4 mL) was added to the reaction solution, the reaction mixture was stirred for 2 hours at room temperature.The reaction solution was concentrated to obtain the crude product. The crude product was separated and purified by column chromatography (PE:EA=10:1) to obtain 1.1g light yellow oily substance, which was compound 30-1.

Step 2: Synthesis of compound 30-2

Dry DCM(20mL) and diethylzinc (10mL)were added in 100 mL three-necked flask under nitrogen protection and ice bath, then TFA(0.5mL) was slowly dropped to the reaction solution, the reaction mixture was stirred under ice bath for 30min, then diiodomethane (1.53g) was dropped to the reaction solution, the reaction was continued under the ice bath for 30min, and then compound 30-1(0.8g) was added to the reaction solution. The reaction mixture was slowly raised to room temperature and stirredfor 12h.The reaction solution was quenched by adding saturated ammonium chloride solution, extracted by EA for three times. The organic phase was washed with water and saturated aqueous NaCl successively, and then concentrated to obtain crude product. The crude product was separated by column chromatography (PE:EA=10:1) to obtain 0.6g milky oil, which was compound 30-2.

Step 3: Synthesis of compound 30-3

Compounds 30-2 (0.71 g), 4-bromo-3, 6-dichloroprazine (0.5 g), $K_2CO_3$ (0.61 g), $Pd(dppf)Cl_2$-$CH_2Cl_2$ (180 mg) were mixed in dioxane (20 mL) and water (4 mL).The reaction mixture was stirred at 90°C for 2 h under the protection of nitrogen. The reaction solution was concentrated to obtain the crude product. The crude product was separated and purified by column chromatography (PE: EA = 5:1) to obtained 200 mg white solid, which was compound 30-3.

Step 4: Synthesis of compound 30-4

Compounds 30-3 (200 mg), 2, 4-d imethoxypyrimidin -5-boric acid (168 mg), $K_2CO_3$ (175.4 mg), $Pd(dppf)Cl_2$-$CH_2Cl_2$ (51.9 mg) were mixed into dioxane (10 mL) and water (1 mL).The reaction mixture was stirred at 60°C for 2 h under the protection of nitrogen.The reaction solution was concentrated to obtain the crude product. The crude product was separated and purified by column chromatography (PE: EA = 1 :1)to obtain 81 mg white solid, which was compound 30-4.

Step 5: Synthesis of compound 30

Compound 30-4 (40 mg) was added to 25 mL single-mouth flask, dissolved in methanol (5 mL), and 1 M hydrochloric acid (5 mL) was added, the reaction mixture was stirred at 60 °C for 5 h. After completion of the reaction, the reaction solution was cooled to room temperature. The reaction solution was concentrated to obtain the crude product.

The crude product was separated by reverse phase chromatography ($CH_3CN/H_2O$ = 30%-55%) to obtain 5.2 mg white solid, which was compound 30(Purity 95.12%).

LCMS: $[M+H]^+$= 391.2

$^1H$ NMR (500 MHz, DMSO-$d_6$) δ 11.46 (s, 1H), 8.41 (s, 1H), 8.25 (s, 1H), 8.13 - 8.06 (m, 1H), 8.01 - 7.93 (m, 1H), 7.86 (d, *J* = 7.3 Hz, 1H), 7.55 (dd, *J* = 6.3, 3.2 Hz, 2H), 7.49 (t, *J* = 6.7 Hz, 2H), 2.91 (t, *J* = 10.2 Hz, 1H), 1.85 (dd, *J* = 13.9, 6.4 Hz, 1H), 1.74 (dd, *J* = 14.1, 5.3 Hz, 1H), 1.23 (s, 1H).

**Example 31: Synthesis of compound 31 5-(6-methoxy-5-(2-phenyleyclopropyl)pyridazin-3-yl)pyrimidine-2,4(*1H*,*3H*)-dione**

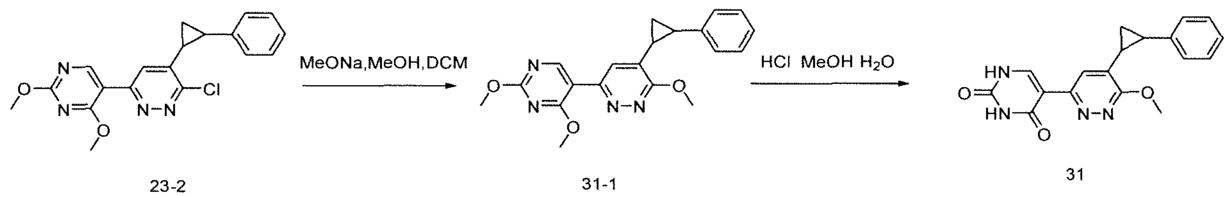


23-2 31-1 31

Step 1: Synthesis of compound 31-1

Compound 23-2 (0.40g) was added to a 45 mL threaded pressure tube, dissolved in methylene chloride (3 ml) and then methyl alcohol (15 ml) and sodium methanol (1.17 g) were added, the reaction mixture was stirred at 80°C for 5h under seal.After completion of the reaction, the reaction solution was mixed with silica gel and purified by column chromatography to obtain a white solid (0.38g, 71.41% yield), which was compound 31-1.

Step 2: Synthesis of compound 31

Compound 31-1(380mg), 1M hydrochloric acid aqueous solution (5mL) and methanol (5mL) were added to a 50mL single-mouth flask, and the reaction mixture was stirred at 30°C for 24h.After completion of the reaction, the reaction solution was filtered, the filter residue was slurried with a small amount of methanol, and the crude white solid was obtained by filter, and separated and purified by liquid chromatography column and freeze-dried to obtain white solid (22.1mg,8.4%yield), which was compound 31 (97.73% purity).

LCMS: $[M+H]^+$= 337.2

Example 32: **Synthesis of compound 32 5-(5-(2-(4-fluorophenyl)cyclopropyl)-6-methoxypyridazin-3-yl)pyrimidine-2,4(*1H*,*3H*)-dione**

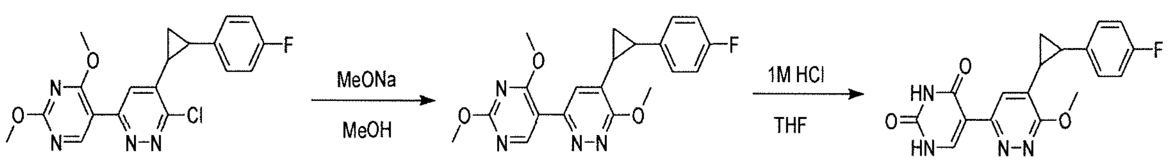


M1 32-1 32

Step 1: Synthesis of compound 32-1

The compounds M1 (30 mg) and sodium methanol (50 mg) were dissolved in methanol (2 mL) and the reaction mixture was stirred at 80°C for 3h.The reaction solution was concentrated to obtain the crude product. The crude product was separated and purified by by column chromatography (PE: EA = 3:1) to obtain 15 mg white solid, which was compound 32-1.

Step 2: Synthesis of compound 32

Compound 32-1(15mg) was dissolved in THF(1mL) and 1M hydrochloric acid (1mL) , the reaction mixture was stirred at 35°C for 12h.The reaction solution was adjusted to a neutral pH with a saturated sodium bicarbonate solution. The reaction solution was filtered and the filter cake was dried to obtain 5 mg of white solid,which was compound 32.

LCMS: $[M+H]^+$= 355.2

**Example 33: Synthesis of compound 33 5-(5-(2-(4-chlorophenyl)cyclopropyl)-6-methoxypyridazin-3-yl)pyrimidine-2,4(*1H*,*3H*)-dione**

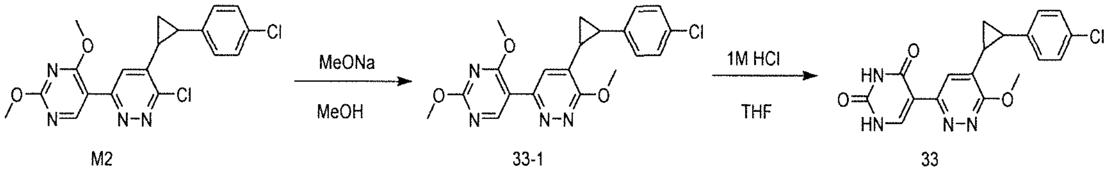


M2 33-1 33

Step 1: Synthesis of compound 33-1

Compound M2 (30 mg) and sodium methanol (50 mg) were dissolved in methanol (2 mL) and the reaction mixture was stirred at 80°C for 3h.The reaction solution was concentratedto obtain the crude product. The crude product was separated and purified by column chromatography (PE: EA = 1:1) to obtain 15 mg white solid, which was compound 33-1.

Step 2: Synthesis of compound 33

Compound 33-1(15mg) was dissolved in THF(1mL) and 1M hydrochloric acid (1mL) , the reaction mixture was stirred at 35°C for 12h.The reaction solution was adjusted to a neutral pH with a saturated sodium bicarbonate solution. The reaction solution was filtered and the filter cake was dried to obtain 3mg of white solid, which was compound 33.

LCMS: $[M+H]^+$= 371.2

**Example 34: Synthesis of compound 34 6-(2,4-dioxo-1,2,3,4-tetrahydropyrimidin-5-yl)-4-(2-phenylcyclopropyl)pyridazine-3-carbonitrile**

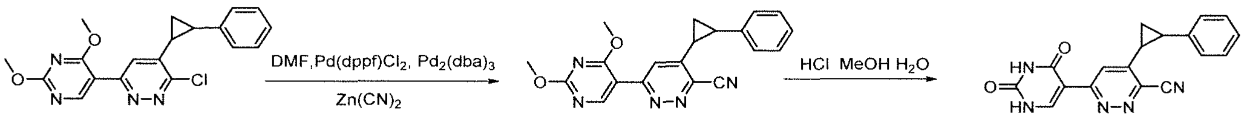


23-2 34-1 34

Step 1: Synthesis of compound 34-1

Compound 23-2 (0.41 g) was dissolved in 50 ml single-mouth flask with DMF(10 ml), $Pd_2(dba)_3$ (51 mg) and $Pd(dppf)Cl_2$-$CH_2Cl_2$ (90.8 mg) were added, the reaction mixture was stirred at 120°C under nitrogen protection to react for 3h.After completion of the reaction, 20ml water was added to the reaction solution, the reaction solution was extracted with EA (30ml each time) for three times, the organic phase was dried with anhydrous sodium sulfate to obtain the crude product, the crude product was purified by column chromatography to a white solid (0.31g, 62.07% yield), which was compound 34-1.

Step 2: Synthesis of compound 34

Compound 34-1(380mg), 1M hydrochloric acid aqueous solution (5mL), and methanol (5mL) were added to a 50mL single-mouth flask and the reaction mixture was stirred at 50°C for 15h. After completion of the reaction, the

reaction solution is filtered, the filter residue is slurried with a small amount of methanol, and the white solid (crude product) was obtained by filtered, then isolated and purified by pre-HPLC and lyophilized to obtain compound 34 (74.2 mg, 35.93% yield).

LCMS: $[M+H]^+$= 332.2

**Embodiment 35: Synthesis of compound 35 6-(2,4-dioxo-1,2,3,4-tetrahydropyrimidin-5-yl)-4-(2-(4-fluorophenyl)cyclopropyl)pyridazine-3-carbonitrile**

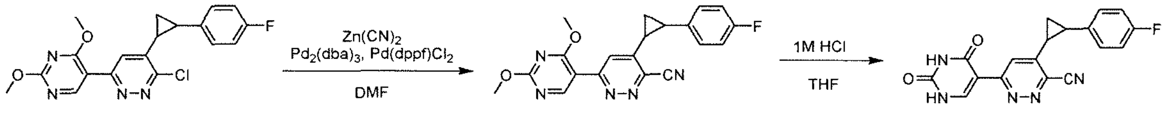


M1 35-1 35

Step 1: Synthesis of compound 35-1

Compound M1 (50 mg), $Zn(CN)_2$ (30 mg), $Pd_2(dba)_3$ (5 mg), $Pd(dppf)Cl_2$ (10 mg) were added to 25 ml three-necked flask, dissolved in DMF (4 mL), the reaction mixture was stirred for 2 h at 120°C under $N_2$ protection The reaction solution was quenched with saturated sodium carbonate solution, extracted with EA for three times, the organic phase was washed twice with saturated sodium carbonate, concentrated to obtain the crude product. The crude product was separated and purified by column chromatography(PE: EA = 3:1) to obtain 20 mg light yellow oily substance, which was compound 35-1.

Step 2: Synthesis of compound 35

Compound 35-1(20mg) was dissolved in THF(2mL) and 1M hydrochloric acid (2mL), the reaction mixture was stirred at 50°C for 12h.The reaction solution was adjusted to a neutral pH with a saturated sodium bicarbonate solution. The reaction solution was filtered and the filter cake was dried to obtain 6 mg of white solid, which was compound 35.

LCMS: $[M+H]^+$= 350.2

**Embodiment 36: Synthesis of compound 36 4-(2-(4-chlorophenyl)cyclopropyl)-6-(2,4-dioxo-1,2,3,4-tetrahydropyrimidin-5-yl)pyridazine-3-carbonitrile**

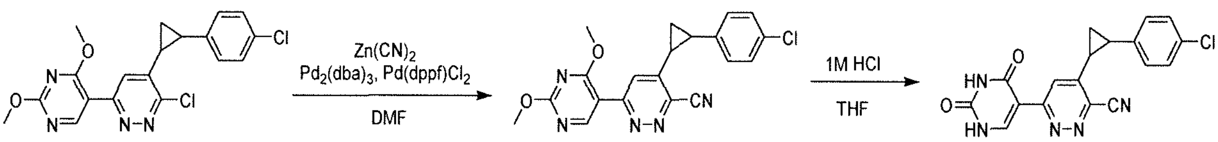


M2 36-1 36

Step 1: Synthesis of compound 36-1

Compound M2 (50 mg), $Zn(CN)_2$ (30 mg), $Pd_2(dba)_3$ (5 mg), $Pd(dppf)Cl_2$ (10 mg)were added into 25 ml three-necked flask, dissolve in DMF (4 mL), the reaction mixture was stirred for 2 h at 120°C under $N_2$ protection. The reaction solution was quenched with saturated sodium carbonate solution, extracted with EA for three times, the organic phase was washed twice with saturated sodium carbonate, and concentrated to obtain the crude product. The crude product was separated by column chromatography (PE: EA = 2:1) to obtain 15 mg light yellow oily, which was compound 36-1.

Step 2: Synthesis of compound 36

Compound 36-1(15mg) was dissolved in THF(2mL) and 1M hydrochloric acid (2mL), the reaction mixture was stirred at 50°C for 12h.The reaction solution was adjusted to a neutral pH with a saturated sodium bicarbonate solution. The reaction solution was filtered and the filter cake was dried to obtain 4mg of white solid,which was compound 36.

LCMS: $[M+H]^+$= 366.2

**Example 37: Synthesis of compound 37 5-(6-chloro-5-((*1S,2S*)-2-(pyridin-4-ylethynyl)cyclopropyl)pyridazin-3-yl)pyrimidine-2,4(*1H,3H*)-dione**

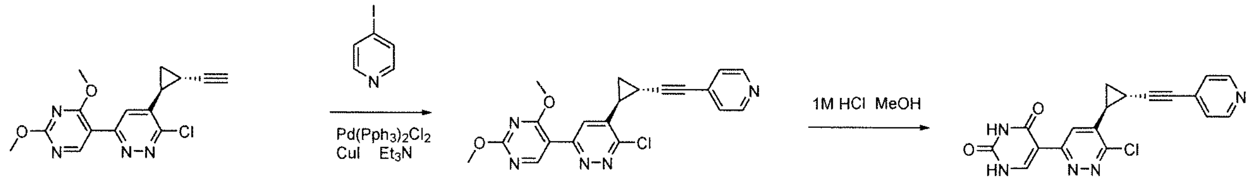


M9 37-1 37

**Step 1: Synthesis of compound 37-1**

Compound M9 (100 mg), 4-iodipyridine (90 mg), bistriphenylphosphine palladium dichloride (20 mg), cuprous iodide (10 mg), triethylamine (5 mL)and DMF(2 mL) were added into a 25-ml three-necked flask, and the reaction mixture was stirred at room temperature for 12h under nitrogen protection. After completion of the reaction, the reaction solution was quenched with saturated ammonium chloride, extracted by EA and purified by pre-TLC to obtain a light yellow solid, which was compound 37-1 (100 mg, 80.45% yield).

**Step 2: Synthesis of compound 37**

Compound 37-1 (100 mg), THF(5 mL) and 1M hydrochloric acid (5 mL) were added into a 25 mL single-mouth flask and the reaction mixture was stirred at 50°C for 12h. After completion of the reaction, the reaction solution was filtered and slurried with methanol to produce a white solid (35.0 mg, 37.67% yield), which was compound 37.LCMS: $[M+H]^+$=366.2

$^1$H NMR (500 MHz, DMSO-$d_6$) $\delta$ 11.64 (dd, J = 6.3, 2.0 Hz, 1H), 11.58 (d, J = 1.9 Hz, 1H), 8.75 (d, J = 5.7 Hz, 2H), 8.33 (d, J = 6.3 Hz, 1H), 8.07 (s, 1H), 7.79 - 7.74 (m, 2H), 2.70 (ddd, J = 8.8, 6.6, 4.8 Hz, 1H), 2.24 - 2.18 (m, 1H), 1.72 (dt, J = 8.7, 5.2 Hz, 1H), 1.66 (ddd, J = 8.8, 6.6, 4.9 Hz, 1H).

**Example 38: Synthesis of compound 38 6-(((*1S,2S*)-2-(3-chloro-6-(2,4-dioxo-1,2,3,4-tetrahydropyrimidin-5-yl)pyridazin-4-yl)cyclopropyl)ethynyl)nicotinonitrile**

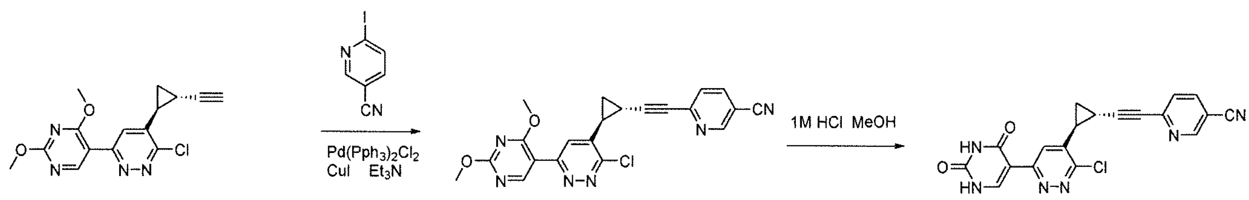


M9 38-1 38

**Step 1: Synthesis of compound 38-1**

Compound M9 (100 mg), 6-iodine-nicotinitrile (90 mg), ditriphenylphosphine dichloride (20 mg), cuprous iodide (10 mg), triethylamine (5 mL), DMF(2 mL) were added into a 25ml three-necked flask, and the reaction mixture was stirred at room temperature for 12h under $N_2$ protection. After completion of the reaction, the reaction solution was quenched with saturated ammonium chloride, extracted by EA and purified by TLC to obtain a light yellow oily, which was compound 38-1 (100 mg, 75.64% yield).

**Step 2: Synthesis of compound 38**

Compound 38-1 (100 mg), THF(5 mL) and 1M hydrochloric acid (5 mL) were added into a 25 mL single-mouth flask and the reaction mixture was stirred at 50°C for 12h. After completion of the reaction, the reaction solution was

concentrated to obtain the crude product. The crude product was separated and purified by preparative liquid chromatography to obtain a brown solid which was compound 38 (9.0 mg, 10.72% yield).

LCMS: $[M+H]^+$=391.2

$^1H$ NMR (500 MHz, DMSO-$d_6$) δ 11.39 (s, 2H), 9.00 (dd, J = 24.4, 2.1 Hz, 1H), 8.38 - 8.29 (m, 2H), 8.07 (s, 1H), 7.71 (d, J = 8.2 Hz, 1H), 2.67 (dt, J = 8.4, 5.3 Hz, 1H), 2.17 (dt, J = 9.7, 5.4 Hz, 1H), 1.67 (ddt, J = 29.7, 9.2, 5.5 Hz, 2H).

**Example 39: Synthesis of compound 39 5-(6-methoxy-5-((*1S,2R*)-2-vinylcyclopropyl)pyridazin-3-yl)pyrimidine-2,4(*1H,3H*)-dione**

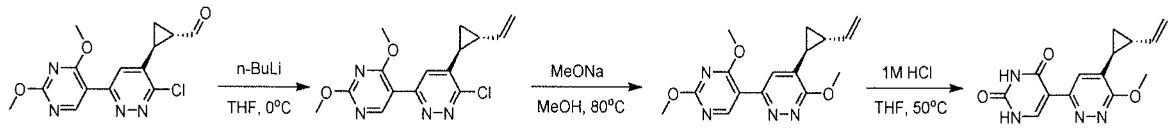


M8 39-1 39-2 39

**Step 1: Synthesis of compound 39-1**

Methyltriphenyl phosphorus bromide (580 mg) was added to a 50 ml three-necked flask, dissolved in THF (10 ml), cooling under an ice bath, n-BuLi (1.0 ml) was added to the reaction solution, the reaction mixture was stirred under $N_2$ protection for 15 minutes, and **compound** M3 (200 mg) of THF solution was dropped to the reaction solution. The reaction mixture was stirred under ice bath for 1h.A small amount of water is added to the reaction solution to quench the reaction, and the reaction solution was concentrated to obtain the crude product. The crude product was separated by column chromatography (DCM: MeOH = 15:1)to obtain 200 mg of milky oil, which was compound 39-1.

**Step 2: Synthesis of compound 39-2**

Compounds 39-2 (40 mg) and sodium methanol (70 mg) were dissolved in methanol (3 mL) and the reaction mixture was stirred at 80°C for 3h. The reaction solution was concentrated to obtain the crude product. The crude product was separated and purified by by column chromatography (PE: EA=1: 1) to obtain 20 mg of white oily, which was compound 39-2.

**Step 3: Synthesis of compound 39**

Compound 39-2(20mg) was added to a 25mL single-mouth bottle and dissolved in THF(2mL), then 1M hydrochloric acid (2mL) was added and the reaction mixture was stirred at 35°C for 12h.The pH of the reaction solution was adjusted to neutral by saturated sodium bicarbonate solution. Then the reaction solution was filtered and the filter cake was washed by water to obtain 10mg of white solid, which was compound 39.

LCMS: $[M+H]^+$= 287.2

**Example 40: Synthesis of compound 40 4-((E)-2-((*1R,2S*)-2-(3-chloro-6-(2,4-dioxo-1,2,3,4-tetrahydropyrimidin-5-yl)pyridazin-4-**yl)cyclopropyl)vinyl)benzonitrile

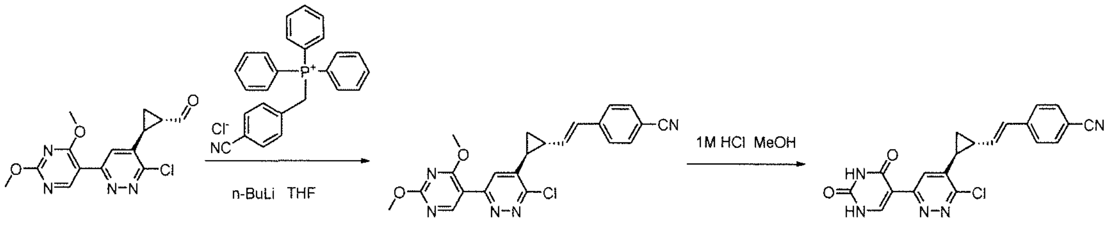


M8 40-1 40

**Step 1: Synthesis of compound 40-1**

Chlorinated (4-cyanobenzyl) triphenyl phosphorus (180mg) and anhydrous THF(5mL) were added into a 25mL three-necked flask, 2.5M n-butyl lithium solution (0.3mL)was slowly added into the reaction solution under the ice bath , the reaction mixture was stirred in the ice bath for 10min, and then compound M8(100mg) was added and the reaction mixture was stirred at room temperature for 1h under the protection of $N_2$. After completionof the reaction, saturated ammonium chloride solution was added to the reaction solution, the reaction solution was extracted with ethyl acetate, washed with saturated aqueous NaCl, dried and concentrated to obtain the crude product, the crude product was purified by column chromatography to obtain a white solid, which was compound 40-1 (40mg,30.55%yield).

**Step 2: Synthesis of compound 40**

Compound 40-1 (40 mg), methanol (4 mL), water (4 mL), and concentrated hydrochloric acid (0.5 mL) were added into a 25ml three-necked flask,and the reaction mixture was stirred at room temperature to react for 72h.After completion of the reaction, the reaction solution was lyophilized and purified by liquid chromatography to obtain a white solid, which was compound 40 (2 mg, 5.36% yield, 99.23% purity).
LCMS: $[M+H]^+$=392.2

**Example 41: Synthesis of compound 41 5-(6-methyl-5-((*1S*,*2S*)-2-(phenylethynyl)cyclopropyl)pyridazin-3-yl)pyrimidine-2,4(*1H*,*3H*)-dione**

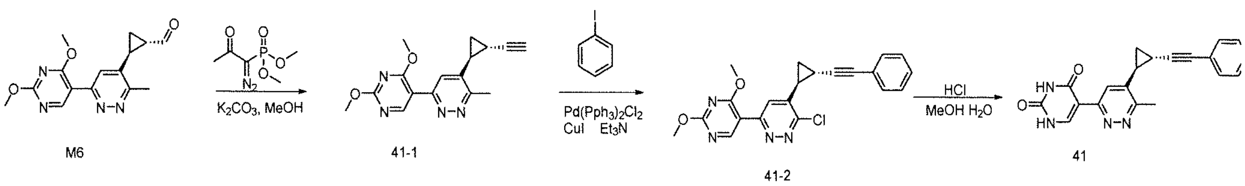

M6 41-1 41-2 41

**Step 1: Synthesis of Compound 41-1**

Compound M6 (220mg) was dissolved in methanol (6mL), potassium carbonate (190mg) was added , coolled it to 0 °C, (1-diazo-2-oxo-propanol) - dimethyl phosphonate (260mg) was added, and then the reaction mixture was stirred at room temperature for 1h.After completion of the reaction, saturated ammonium chloride aqueous solution was added to quench the reaction, The reaction solution was extracted with ethyl acetate for three times, the organic phase was washed with saturated brine, dried with anhydrous sodium sulfate, and separated by Pre-TLC (DCM:MeOH=25:1) to obtain 160mg colorless liquid, which was compound 41-1.

**Step 2: Synthesis of Compound 41-2**

Compound 50-1 (160 mg), iodobenzene (130 mg), ditriphenylphosphine dichloride (20 mg), cuprous iodide (10mg), triethylamine (5 mL)and THF(2 mL) were added to a 25ml three-necked flask, and the reaction mixture was stirred at room temperature to react for 12h under $N_2$ protection. After completion of the reaction, the reaction solution was quenched with saturated ammonium chloride, extracted with EA, and purified by pre-TLC to obtain a yellow solid, which was compound 41-2 (130 mg, 64.64% yield).

**Step 3: Synthesis of Compound 41**

Compound 41-2 (130 mg), methanol (5 mL), water (5 mL) and concentrated hydrochloric acid (0.5 mL) were added into a 25 mL single-mouth flask and the reaction mixture was stirred at 40°C for 24h.After completion of the reaction, the reaction solution was filtered to obtain the crude product. The crude product was purified by pre-HPLC to obtain a white solid, which was compound 41 (30 mg, 20.80% yield, 99.62% purity).
LCMS: $[M+H]^+$=345.2
The hydrogen spectrum data of compound 41 are as follows:
$^1H$ NMR (500 MHz, DMSO-$d_6$) δ 11.45 (s, 2H), 8.25 (s, 1H), 7.84 (s, 1H), 7.43 (d, *J* = 5.8 Hz, 2H), 7.39-7.30 (m, 3H), 2.75 (s, 3H), 1.98-1.88 (m, 1H), 1.56-1.48 (m, 1H), 1.46-1.39 (m, 1H), 1.28-1.20 (m, 1H).
Examples 42-48 were synthesized by referring to the preparation method of compound 18, or a similar method using the corresponding intermediate.

| | | | |
|---|---|---|---|
| 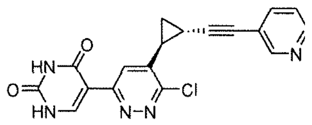 **42** [M+H]+: 366.2 | 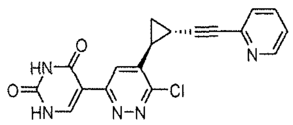 **43** [M+H]+: 366.2 | 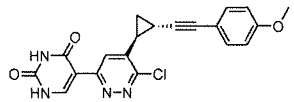 **44** [M+H]+: 395.3 | 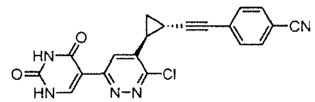 **45** [M+H]+: 390.2 |
| 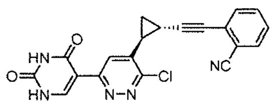 **46** [M+H]+: 390.2 | 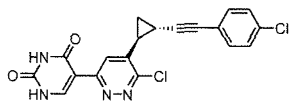 **47** [M+H]+: 399.2 | 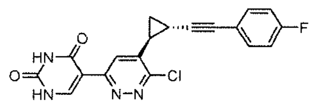 **48** [M+H]+: 383.2 | |

The hydrogen spectrum data of compound 44 are as follows:
$^1$H NMR (500 MHz, DMSO-$d_6$) δ 11.59 (s, 1H), 11.56 (s, 1H), 8.32 (s, 1H), 8.01 (s, 1H), 7.39-7.34 (m, 2H), 6.95-6.88 (m, 2H), 3.76 (s, 3H), 2.54 (s, 1H), 2.02 (ddd, *J* = 8.8, 5.9, 4.6 Hz, 1H), 1.58 (dt, *J* = 8.6, 5.2 Hz, 1H), 1.51 (ddd, *J* = 8.8, 6.3, 4.8 Hz, 1H).

The hydrogen spectrum data of compound 45 are as follows:
$^1$H NMR (500 MHz, DMSO-$d_6$) δ 11.61 (s, 1H), 11.52 (s, 1H), 8.33 (s, 1H), 8.05 (s, 1H), 7.86-7.80 (m, 2H), 7.64-7.59 (m, 2H), 2.12 (dt, *J* = 8.8, 5.4 Hz, 1H), 1.65 (dt, *J* = 8.7, 5.3 Hz, 1H), 1.58 (ddd, *J* = 8.9, 6.5, 4.8 Hz, 1H).

The hydrogen spectrum data of compound 46 are as follows:
$^1$H NMR (500 MHz, DMSO-d6) δ 11.67 - 11.54 (m, 2H), 8.33 (d, *J* = 6.5 Hz, 1H), 8.06 (s, 2H), 7.93-7.87 (m, 1H), 7.74-7.53 (m, 2H), 2.65 (s, 1H), 2.18 (td, *J* = 8.0, 5.9 Hz, 1H), 1.66 (t, *J* = 8.0 Hz, 2H).

The hydrogen spectrum data of compound 48 are as follows:
$^1$H NMR (500 MHz, DMSO-$d_6$) δ 11.61 (s, 1H), 11.58 (s, 1H), 8.32 (s, 1H), 8.02 (s, 1H), 7.49 (dd, *J*= 8.5, 5.4 Hz, 2H), 7.21 (t, *J* = 8.7 Hz, 2H), 2.59 - 2.53 (m, 1H), 2.07-2.03 (m, 1H), 1.64 - 1.49 (m, 2H).

Examples 49-53 were synthesized by referring to the preparation method of compound 21, or a similar method using the corresponding intermediates.

| | | | |
|---|---|---|---|
| 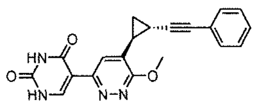 **49** [M+H]+: 361.2 | 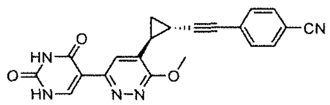 **50** [M+H]+: 386.3 | 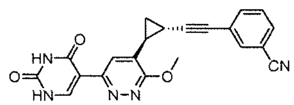 **51** [M+H]+: 386.3 | 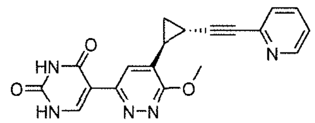 **52** [M+H]+: 362.2 |
| 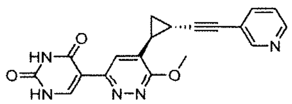 **53** [M+H]+: 362.3 | | | |

The hydrogen spectrum data of compound 49 are as follows:
$^1$H NMR (500 MHz, DMSO-$d_6$) δ 11.45 (s, 1H), 8.09 (s, 1H), 7.75 (s, 1H), 7.48-7.39 (m, 2H), 7.39-7.30 (m, 3H), 4.10 (s, 3H), 2.05-1.98 (m,1H), 1.53-1.49(m, 1H), 1.28-1.19 (m, 2H)

The hydrogen spectrum data of compound 50 are as follows:
$^1$H NMR (500 MHz, DMSO-$d_6$) δ 11.77 (d, *J* = 6.3 Hz, 1H), 11.63 (s, 1H), 8.29 (d, *J* = 5.9 Hz, 1H), 7.90 (s, 1H), 7.83 (d, *J* = 8.1 Hz, 2H), 7.61 (d, *J* = 8.1 Hz, 2H), 4.11 (s, 3H), 2.59-2.54(m, 1H), 2.25-2.18 (m, 1H), 1.71-1.60 (m, 2H).

The hydrogen spectrum data of compound 51 are as follows:
$^1$H NMR (500 MHz, DMSO-$d_6$) δ 12.14 (d, *J* = 6.2 Hz, 1H), 11.83 (s, 1H), 8.50 (d, *J* = 5.3 Hz, 1H), 8.04 (s, 1H), 7.92 (d, *J* = 1.7 Hz, 1H), 7.84 (dt, *J* = 7.8, 1.5 Hz, 1H), 7.77-7.75 (m, 1H), 7.58 (t, *J* = 7.8 Hz, 1H), 2.65-5.59(m, 1H), 2.39-2.31 (m, 1H), 1.82-1.75 (m, 1H), 1.73-1.65(m, 1H).

The hydrogen spectrum data of compound 53 are as follows:
$^1$H NMR (500 MHz, DMSO-$d_6$) δ 11.51-11.32 (m, 1H), 8.62 (s, 1H), 8.53 (d, *J* = 4.9 Hz, 1H), 8.10 (s, 1H), 7.85 (d, *J* = 7.8 Hz, 1H), 7.77 (s, 1H), 7.39 (dd, *J* = 7.8, 4.8 Hz, 1H), 4.10 (s, 3H), 2.06 (q, *J* = 5.4, 4.7 Hz, 1H), 1.54 (d, *J* = 8.3 Hz, 2H).

Examples 54-56 were synthesized by referring to the preparation method of above Example 34, or by a similar method using the corresponding intermediates.

| | | | |
|---|---|---|---|
| 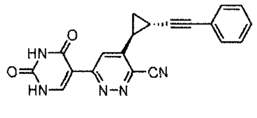 **54** [M+H]$^+$: 356 | 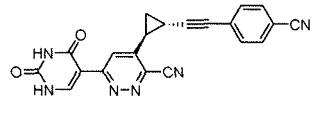 **55** [M+H] $^+$: 381 | 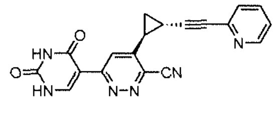 **56** [M+H]$^+$: 357 | |

The hydrogen spectrum data of compound 55 are as follows:
$^1$H NMR (500 MHz, DMSO-$d_6$) δ 11.80 (s, 1H), 11.66 (s, 1H), 8.56 (s, 1H), 8.19 (s, 1H), 7.87-7.82 (m, 2H), 7.64-7.60 (m, 2H), 2.67 (ddd, *J* = 8.8, 6.2, 4.5 Hz, 1H), 2.28 (ddd, *J* = 8.9, 6.1, 4.5 Hz, 1H), 1.76 (dt, *J* = 8.9, 5.4 Hz, 1H), 1.69-1.63 (m, 1H).

**Example 57: Synthesis of compound 57 5-(6-chloro-5-((*1S,2S*)-2-phenylcyclopropyl)pyridazin-3-yl)pyrimidine-2,4(*1H,3H*)-dione**

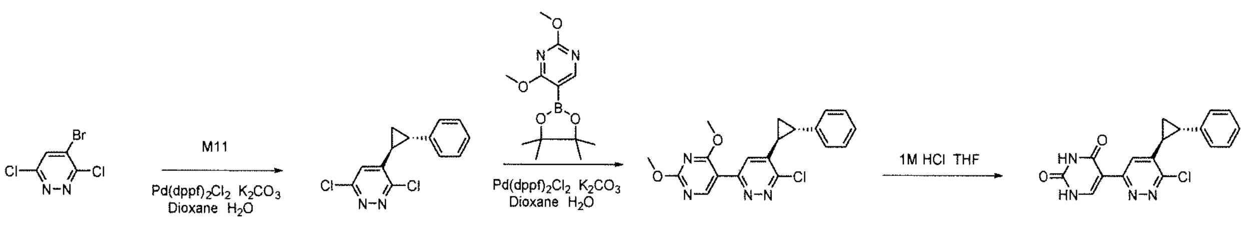


57-1 57-2 57

**Step 1: Synthesis of compound 57-1**

4-bromo-3, 6-dichloroprazine (1.8 g), compound 39-5 (2.3 g), [1,1 '- bis (diphenylphosphine) ferrocene] palladium dichloride dichloromethane complex (0.6 g), potassium carbonate (1.6 g), dioxane (20 mL) and water (4mL) were added to 50 mL three-necked flask, and the reaction mixture was stirred at 60 °C for 12h under $N_2$ protection.After completion of the reaction, the reaction solution was poured into water, extracted with ethyl acetate, washed with saturated aqueous NaCl, dried and concentrated to obtain the crude product, the crude product was purified by column chromatography to obtain a white solid, which was compound 57-1 (1.4g, 66.85% yield).

**Step2: Synthesis of compound 57-2**

Compound 57-1(1.3g), (2, 4-dimethoxypyrimidin-5-yl) boron ester (1.8g), [1,1 '- bis (diphenylphosphine) ferrocene] palladium dichloride dichloromethane complex (0.2g), potassium carbonate (1g), dioxane (15mL) and water

(3mL) were added into the 50mL three-necked flask and the reaction mixture was stirred at 60 °C for 2h under $N_2$ protection. After completion of the reaction, the reaction solution was poured into water, the reaction solution was extracted with ethyl acetate, washed with saturated aqueous NaCl, dried and concentrated to obtain the crude product. The crude product was purified by column chromatography to obtain white solid crude product, then slurried with methanol and filtered to obtain the white solid, which was compound 57-2.(0.6g,33.18%yield).

**Step 3: Synthesis of compound 57**

Compound 57-2 (0.6 g), 1M hydrochloric acid aqueous solution (5 mL) and THF(5 mL) were added to a 50 mL single-mouth bottle and the reaction mixture was stirred at 50°C for 8h. After completion of the reaction, the reaction solution was filtered, and the filter cake was slurried with a small amount of mixture of methylene chloride and methanol. White solid (0.4g, 66.67% yield) was obtained by filtration, which was compound 57(99.57% purity).

LCMS: $[M+H]^+$= 341.2

$^1H$ NMR (500 MHz, DMSO-$d_6$) δ 11.61 (s, 1H), 11.59 (s, 1H), 8.33 (s, 1H), 8.07 (s, 1H), 7.35-7.29 (m, 2H), 7.28-7.25 (m, 2H), 7.24-7.19 (m, 1H), 2.44-2.38 (m, 1H), 2.38-2.31 (m, 1H), 1.74-1.68 (m, 1H), 1.67-1.61 (m, 1H).

**Example 59: Synthesis of compound 59 5-(5-((*1S,2S*)-2-phenylcyclopropyl)pyridazin-3-yl)pyrimidine-2,4(*1H,3H*)-dione**

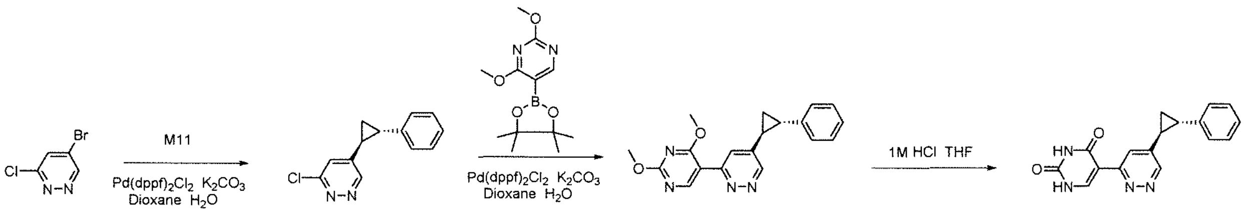


59-1 59-2 59

**Step 1: Synthesis of compound 59-1**

5-bromo-3-chlordadiazine (0.40 g), compound 53-5 (0.61 g), [1,1'-bis (diphenylphosphine) ferrocene] Palladium dichloromethane complex (0.17 g), potassium carbonate (0.86 g), dioxane (10 mL) and water (2 mL) were added to a 50 mL three-necked flask and the reaction mixture was stirred at 60 °C for 12h under $N_2$ protection. After completion of the reaction, the reaction solution was poured into water, the mixture was extracted with ethyl acetate, the organic phase was washed with saturated aqueous NaCl, dried and concentrated to obtain crude product, and the crude product was purified by column chromatography to obtain white solid, which was compound 59-1 (0.35g, 73.6 % yield).

**Step 2: Synthesis of compound 59-2**

Compound 59-1(0.3g), (2, 4-dimethoxy-pyrimidine-5-yl) boron ester (0.36g), [1,1-bis (diphenylphosphine) ferrocene] palladium dichloromethane complex (0.11g), potassium carbonate (0.54g), dioxyhexyclic (10mL) and water (2mL) were added to a 50 mL three-necked flask and the reaction mixture was stirred at 60°C for 2h under $N_2$ protection. After completion of the reaction, the reaction solution was poured into water, the mixture was extracted with ethyl acetate, the organic phase was washed with saturated aqueous NaCl, dried and concentrated to obtain the crude product, the crude product was purified by column chromatography to obtain the white solid crude product, then slurried with methanol and filtered to obtain the white solid, which was compound 59-2 (0.3g,69.00%yield).

**Step 7: Synthesis of Compound 59**

Compound 59-2 (0.3g), 1M hydrochloric acid aqueous solution (10 mL) and THF(10 mL) were added into a 25 mL single-mouth flask and the reaction mixture was stirred at 50°C for 8h. After completion of reaction, the reaction solution was filtered, and the filter cakewas slurried with a small amount of mixture of methylene chloride and methanol, and white solid was obtained by filtration, which was compound 59 (0.11 g, 40.03% yield, 99.57% purity).

LCMS: $[M+H]^+$= 307.2

**Example 60: Synthesis of compound 60 5-(5-((*1S*,*2S*)-2-(1H-benzo[d]imidazol-2-yl)cyclopropyl)-6-chloropyridazin-3-yl)pyrimidine-2,4(*1H*,*3H*)-dione**

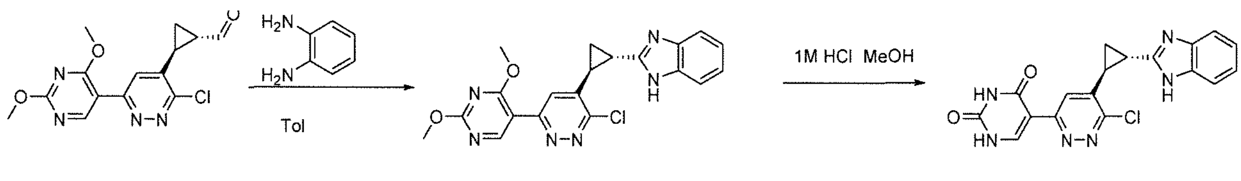


M8 60-1 60

**Step 1: Synthesis of Compound 60-1**

Compound M8 (80 mg), o-phenylenediamine (40 mg), and toluene (10 mL) were added to a 25 mL single-mouth flask and the reaction mixture was stirred at room temperature overnight. After completion of the reaction, the reaction solution was concentrated to obtain the crude product. The crude product was purified by column chromatography to obtain a white solid, which was compound 60-1 (60 mg, 58.82% yield).

**Step 2: Synthesis of Compound 60**

Compound 61-1 (60 mg), tetrahydrofuran (4 mL), water (4 mL), concentrated hydrochloric acid (0.5 mL) were added to a 25 mL single-mouth bottle and the reaction mixture was stirred at room temperature for 12h. After completion of the reaction, the reaction solution was lyophilized and purified by preparative liquid chromatography to obtain white solid,which was compound 60 (2.7mg, 5.37% yield,99.23% purity).

LCMS: $[M+H]^+$=381.2

Examples 61-77 were synthesized by the above example 23 method, or by a similar method using the corresponding intermediates.

| | | | |
|---|---|---|---|
| 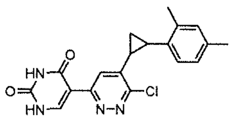 **61** [M+H]+: 369.2 | 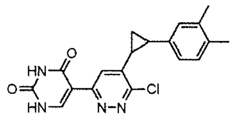 **62** [M+H]+: 369.2 | 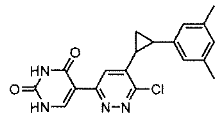 **63** [M+H]+: 369.4 | 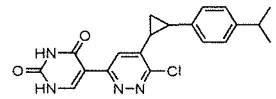 **64** [M+H]+: 383.2 |
| 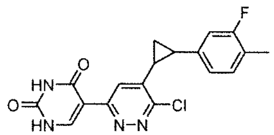 **65** [M+H]+: 373.1 | 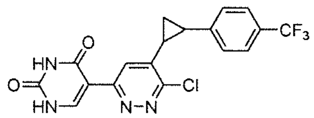 **66** [M+H]+: 409.2 | 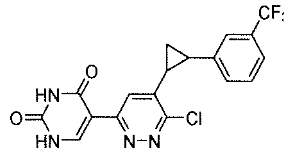 **67** [M+H]+: 409.2 | 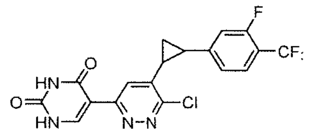 **68** [M+H]+: 427.4 |

| | | | |
|---|---|---|---|
| 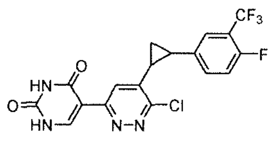 **69** [M+H]+: 427.4 | 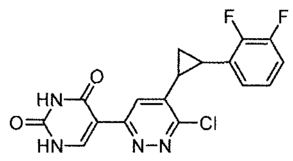 **70** [M+H]+: 377.3 | 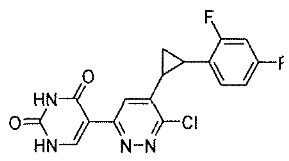 **71** [M+H]+: 377.1 | 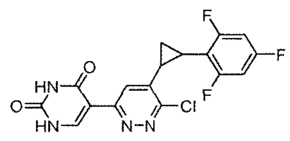 **72** [M+H]+: 395.7 |
| 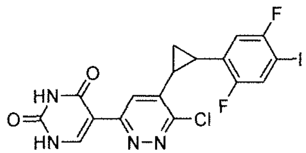 **73** [M+H]+: 395.6 | 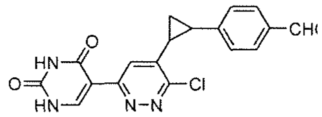 74 [M+H]+ : 369.1 | 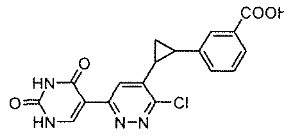 **75** [M+H]+: 385.2 | 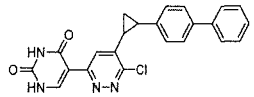 **76** [M+H]+: 417.3 |

The hydrogen spectrum data of compound 61 are as follows:
$^1$H NMR (500 MHz, DMSO-$d_6$) $\delta$ 11.60 (d, $J$ = 6.2 Hz, 1H), 11.58 (d, $J$ = 2.0 Hz, 1H), 8.34 (d, $J$ = 6.2 Hz, 1H), 8.09 (s, 1H), 7.06 (d, $J$ = 7.8 Hz, 1H), 7.00 (s, 1H), 6.98 (d, $J$ = 7.7 Hz, 1H), 2.33 (dt, $J$ = 8.3, 5.6 Hz, 1H), 2.25 (s, 3H), 2.24 (s, 3H), 2.20 (dt, $J$ = 8.8, 5.6 Hz, 1H), 1.71 (dt, $J$ = 8.2, 5.5 Hz, 1H), 1.55 (dt, $J$ = 9.0, 5.4 Hz, 1H).

The hydrogen spectrum data of compound 63 are as follows:
$^1$H NMR (500 MHz, DMSO) $\delta$ 11.56-11.41 (m, 2H), 8.37 (s, 1H), 8.07 (d, $J$ = 2.8 Hz, 1H), 6.85 (s, 3H), 2.24 (d, $J$ = 2.6 Hz, 6H), 1.68 (s, 1H), 1.59 (s, 1H), 1.23 (s, 2H).

The hydrogen spectrum data of compound 64 are as follows:
$^1$H NMR (500 MHz, DMSO) $\delta$ 11.60 (d, $J$ = 10.2 Hz, 2H), 8.33 (s, 1H), 8.05 (s, 1H), 7.18 (s, 4H), 2.86 (dt, $J$ = 13.8, 6.9 Hz, 1H), 2.37 (s, 1H), 2.31 (dt, $J$ = 10.9, 5.5 Hz, 1H), 1.68 (dd, $J$ = 14.1, 5.8 Hz, 1H), 1.60 (dd, $J$ = 10.0, 4.4 Hz, 1H), 1.19 (d, $J$ = 6.9 Hz, 6H).

The hydrogen spectrum data of compound 65 are as follows:
$^1$H NMR (500 MHz, DMSO-$d_6$) $\delta$ 11.62 (dd, $J$ = 6.3, 2.0 Hz, 1H), 11.58 (d, $J$ = 2.0 Hz, 1H), 8.33 (d, $J$ = 6.2 Hz, 1H), 8.06 (s, 1H), 7.21 (t, $J$ = 8.0 Hz, 1H), 7.06 (dd, $J$ = 11.2, 1.8 Hz, 1H), 7.02 (dd, $J$ = 7.8, 1.8 Hz, 1H), 2.43-2.37 (m, 1H), 2.35-2.28 (m, 1H), 2.20 (d, $J$ = 1.9 Hz, 3H), 1.70 (dt, $J$ = 8.5, 5.8 Hz, 1H), 1.64 (dt, $J$ = 9.1, 5.7 Hz, 1H).

The hydrogen spectrum data of compound 67 are as follows:
$^1$H NMR (500 MHz, DMSO) $\delta$ 11.60 (s, 1H), 11.58 (s, 1H), 8.34 (s, 1H), 8.10 (s, 1H), 7.66 (s, 1H), 7.58-7.54 (m, 3H), 2.46-2.42 (m, 2H), 1.84-1.77 (m, 1H), 1.71-1.64 (m, 1H).

The hydrogen spectrum data of compound 68 are as follows:
$^1$H NMR (500 MHz, DMSO) $\delta$ 11.72 - 11.49 (m, 2H), 8.34 (d, J = 6.2 Hz, 1H), 8.11 (s, 1H), 7.71 (t, $J$ = 8.1 Hz, 1H), 7.46 (d, $J$ = 12.4 Hz, 1H), 7.35 (d, $J$ = 7.8 Hz, 1H), 2.64 (s, 1H), 2.36 (s, 1H), 1.82 (d, $J$ = 8.8 Hz, 1H), 1.79-1.71 (m, 1H).

The hydrogen spectrum data of compound 70 are as follows:
1H NMR (500 MHz, DMSO) $\delta$ 11.58 (s, 2H), 8.34 (s, 1H), 8.13 (s, 1H), 7.31 (dd, $J$ = 17.8, 8.2 Hz, 1H), 7.22-7.16 (m, 1H), 7.11 (t, $J$ = 7.2 Hz, 1H), 2.64 (s, 1H), 2.36 (s, 1H), 1.78 (dd, $J$ = 14.4, 5.9 Hz, 1H), 1.71-1.63 (m, 1H).

The hydrogen spectrum data of compound 71 are as follows:
$^1$H NMR (500 MHz, DMSO-$d_6$) $\delta$ 11.61 (dd, $J$ = 6.2, 2.1 Hz, 1H), 11.58 (d, $J$ = 2.1 Hz, 1H), 8.33 (d, $J$ = 6.3 Hz, 1H), 8.10 (s, 1H), 7.35 (td, $J$ = 8.8, 6.4 Hz, 1H), 7.25 (ddd, $J$ = 10.7, 9.2, 2.6 Hz, 1H), 7.08 (td, $J$ = 8.4, 2.6 Hz, 1H), 2.43 (dt, $J$ = 8.7, 5.5 Hz, 1H), 2.41 - 2.35 (m, 1H), 1.75 (dt, $J$ = 8.5, 5.7 Hz, 1H), 1.64 (dt, $J$ = 9.1, 5.6 Hz, 1H).

The hydrogen spectrum data of compound 72 are as follows:
$^1$H NMR (500 MHz, DMSO-$d_6$) $\delta$ 11.61 - 11.58 (m, 1H), 11.56 (d, $J$ = 2.0 Hz, 1H), 8.33 (d, $J$ = 6.3 Hz, 1H), 8.07 (s, 1H), 7.20 (t, $J$ = 8.9 Hz, 2H), 2.60 (dt, $J$ = 8.9, 5.5 Hz, 1H), 2.26 (dt, $J$ = 9.1, 5.8 Hz, 1H), 1.78 (dt, $J$ = 8.3, 5.6 Hz, 1H), 1.62 (dt, $J$ = 9.2, 5.5 Hz, 1H).

The hydrogen spectrum data of compound 73 are as follows:
$^1$H NMR (500 MHz, DMSO-$d_6$) $\delta$ 11.59 (s, 1H), 11.57 (s, 1H), 8.33 (s, 1H), 8.11 (s, 1H), 7.55 (td, $J$ = 10.4, 6.9 Hz, 1H), 7.46 (ddd, $J$ = 11.5, 8.9, 7.1 Hz, 1H), 2.48 - 2.46 (m, 1H), 2.42 - 2.36 (m, 1H), 1.79 (dt, $J$ = 8.6, 5.8 Hz, 1H), 1.65 (dt, $J$ = 9.0, 5.7 Hz, 1H).

The hydrogen spectrum data of compound 74 are as follows:
$^1$H NMR (500 MHz, DMSO-$d_6$) $\delta$ 11.60 (s, 2H), 9.97 (s, 1H), 8.34 (s, 1H), 8.11 (s, 1H), 7.86 (d, J = 7.9 Hz, 2H), 7.50 (d, J = 7.9 Hz, 2H), 2.56-2.53 (m, 1H), 2.48-2.44 (m, 1H), 1.84-1.74 (m, 2H).

The hydrogen spectrum data of compound 76 are as follows:
$^1$H NMR (500 MHz, DMSO-$d_6$) $\delta$ 11.64 (dd, J = 6.2, 2.0 Hz, 1H), 11.59 (d, J = 2.0 Hz, 1H), 8.35 (d, J = 6.2 Hz, 1H), 8.09 (s, 1H), 7.68 - 7.64 (m, 2H), 7.62 (d, J = 8.3 Hz, 2H), 7.46 (t, J = 7.7 Hz, 2H), 7.36 (d, J = 7.8 Hz, 3H),2.46 (m, 1H), 2.39 (m, 1H), 1.76 (m, 1H), 1.69 (m, 1H).

**Examples 77: Synthesis of compound 77 4-(2-(6-(2,4-dioxo-1,2,3,4-tetrahydropyrimidin-5-yl)-3-methoxypyridazin-4-**yl)cyclopropyl)benzonitrile

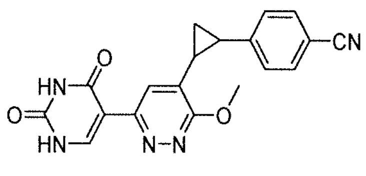

77

The preparation method of compound 77 is obtained by referring to the preparation method of compound 31.

$[M+H]^+$=362

**Examples 78: Synthesis of compound 78 4-(2-(4-cyanophenyl)cyclopropyl)-6-(2,4-dioxo-1,2,3,4-tetrahydropyrimidin-5-yl)pyridazine-3-carbonitrile**

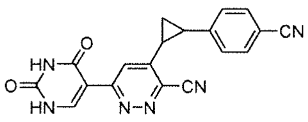

78

The preparation method of compound 78 refers to the preparation method of compound 34. $[M+H]^+$=357。

**Comparative examples 1**

The compound D1 was synthesized according to the method in Example 2 of WO2019168744A1.

LCMS: $[M+H]^+$=287。

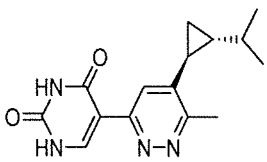

D1

**Comparative examples 2**

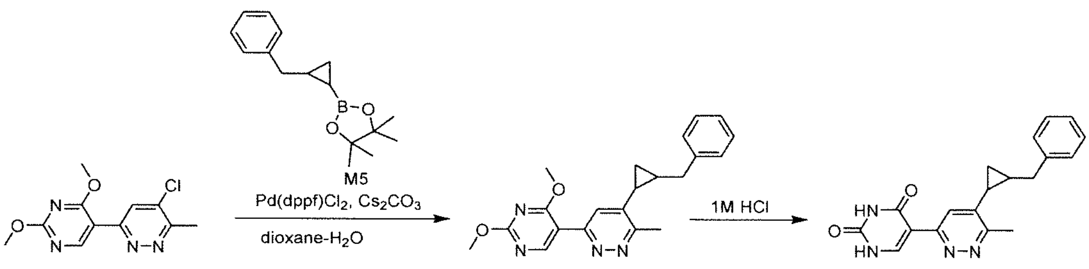


**Step 1: Synthesis of compound D2-1**

Compound M4(50 mg), compound M5 (131 mg), cesium carbonate (122 mg), Pd(dppf)$Cl_2$ (6 mg), dioxane (4 mL)and water (0.5 mL) were added to a 50 mL three-necked flask, and the reaction mixture was stirred at 90°C for 2h under $N_2$ protection. The reaction solution was concentrated to obtain the crude product, the crude product was separated by column chromatography (PE: EA = 5:1) to obtain 11 mg of yellow solid, which was compound D2-1.

**Step 2: Synthesis of compound D2**

Compound D2-1 (11 mg) was added to 25 mL single-mouth bottle and dissolved in 1M hydrochloric acid (2 mL), the reaction mixture was stirred at 70°C for 3 h. The reaction solution was cooled to room temperature, 2.84 mg white solid was prepared by pre-HPLC, which was compound D2 (purity 98.36%).
LCMS: $[M+H]^+$= 335.3

**Comparative examples 3**

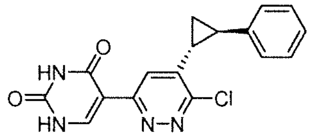

**D3**

The preparation method of compound D3 is obtained by referring to the synthesis method of compound 57.
$[M+H]^+$=341.2
$^1H$ NMR (500 MHz, DMSO-$d_6$) δ 11.59 (s, 1H), 11.57 (s, 1H), 8.33 (s, 1H), 8.07 (s, 1H), 7.35-7.29(m, 2H), 7.28-7.25 (m, 2H), 7.24-7.19 (m, 1H), 2.44-2.38 (m, 1H), 2.37 - 2.31 (m, 1H), 1.74-1.68 (m, 1H), 1.67-1.61(m, 1H).

**Pharmacological experiments**

Example 1: Enzymatic activity detection at the cellular level

The Calu-6 cells were digested and re-suspended using TM buffer (25mM Tris, 5mM $MgCl_2$, pH 7.5) . Calu-6 cell suspension was seeded into 96 well cell culture plate, and the number of cells per well was $2.5 \times 10^4$, adding volume of 100 μL/well.Compound solution with gradient concentration was prepared by TM buffer, and 50 μL DMSO solution with different concentration was added into each pore cell.The final concentration of the compound was 20000, 66666.7, 2222.2, 740.7, 246.9, 82.3, 27.4, 9.1, 3.0, 1.0, 0.3, 0 nM (DMSO final concentration was 0.625%).Preincubated in a shaker at 37°C for 30 min. Each well was added with 50 μ L 800 μ M AMP solution. The shaker at 37°C for 120 min was continued to incubate, then the 96-well plates were centrifuged and 50 μ L supernatant was transferred to the new 96-well plates.50μL 130μM ATP solution and 100μL Cell-titer Glo working solution were added to each well and incubated at room temperature for 10min after being shaken and mixed. The Luminescence luminescence value was read by the multi-function enzymatic reader and converted into the inhibition percentage:

$$\text{Inhibition percentage} = (1\text{- reading/Max}) * 100.$$

The "maximum value" was DMSO control.
$IC_{50}$ values were obtained by curve fitting with GraphPad Prism software.
The enzymatic $IC_{50}$ data of embodiments at the Calu-6 cell level are shown in Table 1.The compound has good activity.

Table 1

| Compound | $IC_{50}$ (nM) | Compound | $IC_{50}$ (nM) |
|---|---|---|---|
| 1 | 21.6 | 42 | 4.4 |
| 2 | 329 | 43 | 3.7 |
| 3 | 721 | 44 | 3.1 |
| 4 | 22.6 | 45 | 1.5 |
| 6 | 50.7 | 46 | 4.4 |
| 7 | 21 | 47 | 7.8 |
| 8 | 92.7 | 48 | 3.8 |
| 9 | 200 | 49 | 5.5 |
| 10 | 31 | 50 | 12.6 |
| 11 | 46 | 51 | 4.3 |

(continued)

| Compound | $IC_{50}$ (nM) | Compound | $IC_{50}$ (nM) |
|---|---|---|---|
| 12 | 19.8 | 52 | 5.8 |
| 13 | 8.5 | 53 | 2.4 |
| 14 | 28 | 54 | 8.2 |
| 15 | 8.8 | 55 | 5.6 |
| 16 | 2.3 | 56 | 19.4 |
| 17 | 2.3 | 57 | 1.3 |
| 18 | 5.6 | 59 | 9.9 |
| 19 | 2.99 | 60 | 23.3 |
| 20 | 1.2 | 61 | 19 |
| 21 | 5.5 | 62 | 5.6 |
| 22 | 22.7 | 63 | 19 |
| 23 | 1.4 | 64 | 22.3 |
| 24 | 5.2 | 65 | 4.7 |
| 25 | 1.8 | 66 | 3.9 |
| 26 | 8.7 | 67 | 7.3 |
| 27 | 1.8 | 68 | 15 |
| 28 | 2.7 | 69 | 13.4 |
| 29 | 4.1 | 70 | 7 |
| 30 | 60 | 71 | 4.6 |
| 31 | 4.2 | 72 | 4.9 |
| 32 | 5.4 | 73 | 5.9 |
| 33 | 3.9 | 74 | 2.7 |
| 34 | 5.9 | 75 | 32 |
| 35 | 1.1 | 76 | 51.9 |
| 36 | 1.9 | 77 | 7.2 |
| 37 | 1.2 | 78 | 8.5 |
| 38 | 3.7 | D1 | 49.1 |
| 39 | 2.5 | D2 | 114 |
| 40 | 2.3 | D3 | 1431 |
| 41 | 7.5 | | |

Example 2: Pharmacokinetic Evaluation

LC/MS/MS method was used to determine the plasma concentration of the embodiment compound at different time after intragastric administration in mice. Study the pharmacokinetic behavior of the compound in mice and evaluate its pharmacokinetic characteristics.

Methods: A single PK study (PO,10mg/kg) was performed on healthy Balb/C female mice (about 20g) purchased from Beijing Vital River Laboratory Animal Technology Co., Ltd.About 1 hour before administration, a certain amount of drug was weighed and added with 5% DMSO and 95% hydroxypropyl betascyclodextrin (HP-β-CD) solution to form a colorless and clear solution.HP-β-CD solution is a 20%HP-β-CD solution prepared by adding HP-β-CD powder into 0.9% normal saline.Intragastric administration was performed at the dosage volume of 10mL/kg, and blood was collected

through the orbital venous plexus of mice. The blood collection time points were as follows:At 0.25, 0.5, 1.0, 2.0, 4.0, 7.0 and 24.0 hours before and after drug administration, 100uL whole blood to $K_2$EDTA anticoagulant tubes were taken at each time point, and the collected whole blood samples were centrifuged at 4000rpm for 10 min. The plasma was separated and stored in the refrigerator at -80°C.

The above plasma samples were added to the pure acetonitrile solution containing the inner target for protein precipitation pre-treatment, centrifugation was performed at 10000rpm for 10 min, and the supernatant was taken, mixed with water 1:1, and 10μL to LC-MS/MS was used for detection. The linear range of compounds detected in plasma samples was 1-1000ng/mL, and the data results were as follows:

Experimental results: The pharmacokinetic parameters of the compounds were shown in Table 2.The compound of the invention has good pharmacokinetic absorption and obvious pharmacokinetic advantages.

Table 2.

| Compound | **Cmax (ng/mL)** | **Tmax (h)** | **AUC (ng/mL*h)** |
|---|---|---|---|
| 13 | 10400 | 0.5 | 16940 |
| 57 | 5407 | 0.5 | 10658 |
| D1 | 547 | 0.5 | 677 |

Although the invention has been fully described by means of its embodiments, it is worth noting that various variations and modifications are readily apparent to a person skilled in the field. Such changes and modifications are intended to be included within the scope of the appended claims of the present invention.

## Claims

**1.** A compound having formula (I), a tautomer, a deuterated compound, or a pharmaceutically acceptable salt thereof, wherein;

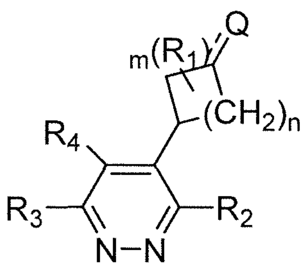

(I)

Q is a linear or cyclic unsaturated group, Q is $=CH_2$, $C_{2-6}$ alkenyl, $C_{2-6}$ alkynyl, or ring A; the $=CH_2$ is optionally substituted by one or more $R_5$; the $C_{2-6}$ alkenyl or $C_{2-6}$ alkynyl is optionally substituted by 1 or more $R_5$ or ring A; ring A is $C_{6-14}$ aryl or 5-14 heterary; the $C_{6-14}$ aryl or the 5-14 heterary is optionally substituted by one or more $R_6$;
$R_1$ is independently H, halogen, cyano, $C_{1-3}$ alkyl, $C_{1-3}$ alkoxy or $C_{1-3}$ haloalkyl, preferably H;
$R_2$ is H, halogen, cyano, substituted or unsubstituted $C_{1-3}$ alkyl, substituted or unsubstituted $C_{1-3}$ alkoxy, substituted or unsubstituted $C_{2-4}$ alkenyl, or substituted or unsubstituted $C_{2-4}$ alkynyl;
$R_3$ is

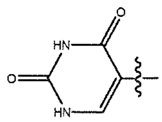

;

$R_4$ is H, halogen, or methyl;
$R_5$ is independently H, cyano, halogen, $C_{1-6}$ alkyl, $C_{1-6}$ haloalkyl, $-C_{0-6}$ alkylene $-OR_a$, $-C_{0-6}$ alkylene -OC(O)N

$(R_a)_2$, $-C_{0-6}$ alkylene -N $(R_a)_2$, $-C_{0-6}$ alkylene $-NR_aC(O)R_a$, $-C_{0-6}$ alkylene $-NR_aC$ (O) $R_a$, $-C_{0-6}$ alkylene $-NR_aC(O)N$ $(R_a)_2$, $-C_{0-6}$ alkylene $-NR_aS(O)R_a$, $-C_{0-6}$ alkylene $-NR_aS(O)_2R_a$, - $C_{0-6}$ alkylene -S $(=O)R_a$,- $C_{0-6}$ alkylene -S $(= O)_2R_a$, - $C_{0-6}$ alkylene $-SR_a$, - $C_{0-6}$ alkylene $-S(R_a)_5$, - $C_{0-6}$ alkylene $-C(=O)R_a$, - $C_{0-6}$ alkylene $-C(=O)OR_a$, - $C_{0-6}$ alkylene $-C(=O)N(R_a)_2$, $-C_{0-6}$ alkylene - $C_{3-14}$ cycloalkyl or $-C_{0-6}$ alkylene -(3-14) heterocyclic, the $C_{1-6}$ alkyl, $-C_{0-6}$ alkylene $-C_{3-14}$ cycloalkyl or $-C_{0-6}$ alkylene -(3-14 heterocyclic) is optionally substituted by one or more $R_a$;

$R_6$ is H, cyano, halogen, $C_{1-6}$ alkyl, $C_{1-6}$ halogen alkyl, $-C_{0-6}$ alkylene-$OR_a$, $-C_{0-6}$ alkylene-$OC(O)N(R_a)_2$, $-C_{0-6}$ alkylene-$N(R_a)_2$, $-C_{0-6}$ alkylene-$NR_aC(O)R_a$, $-C_{0-6}$ alkylene-$NR_aC(O)N(R_a)_2$, - $C_{0-6}$ alkylene-$NR_aS(O)R_a$, $-C_{0-6}$ alkylene-$NR_aS(O)_2R_a$, $-C_{0-6}$ alkylene-$S(=O)R_a$, $-C_{0-6}$ alkylene$S(=O)_2R_a$, $-C_{0-6}$ alkylene-$SR_a$, $-C_{0-6}$ alkylene-$S(R_a)_5$, $-C_{0-6}$ alkylene-$C(=O)R_a$, $-C_{0-6}$ alkylene-$C(=O)OR_a$, $-C_{0-6}$ alkylene-$C(=O)N(R_a)_2$, $C_{2-6}$ alkenyl, $C_{2-6}$ alkynyl, $-C_{0-6}$ alkylene-$C_{3-14}$ cycloalkyl, - $C_{0-6}$ alkylene(3-14 heterocyclic), $-C_{0-6}$ alkylene-$C_{6-14}$ aryl or $-C_{0-6}$ alkylene(5-14 heteroaryl), the $C_{1-6}$ alkyl, $C_{2-6}$ alkenyl, $C_{2-6}$ alkynyl, $-C_{0-6}$ alkylene-$C_{3-14}$ cycloalkyl, $-C_{0-6}$ alkylene(3-14 heterocyclic), $-C_{0-6}$ alkylene-$C_{6-14}$ aryl or $-C_{0-6}$ alkylene(5-14 heteroaryl) is optionally substituted by one or more $R_a$;

each $R_a$ is independently H, halogen, hydroxyl, amino, oxo, nitro, cyano, carboxyl, $C_{1-6}$ alkyl, $C_{1-6}$hydroxyalkyl, $C_{1-6}$aminoalkyl, $C_{1-6}$ haloalkyl, $C_{1-6}$ alkoxy, $C_{1-6}$ haloalkoxy, $C_{1-6}$ heteroalkyl, $C_{3-8}$ cycloalkyl, 3-8 heterocyclic, $C_{6-14}$ aryl, or 5-14 heterаryl;

m is 0, 1, 2, 3, or 4;

n is 0 or 1;

------ is single bond or double bond.

**2.** The compound, the tautomeric, deuterated compound or pharmaceutically acceptable salt thereof of claim 1, having structure:

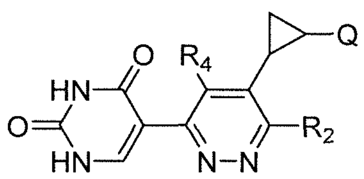

(IA)

or

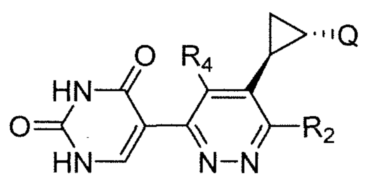

(IA-1)

wherein;

Q is

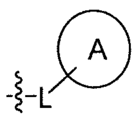

;

L is bond, $C_{2-6}$ alkenyl or $C_{2-6}$ alkynyl; the $C_{2-6}$ alkenyl or $C_{2-6}$ alkynyl is optionally substituted by one or more $R_5$;

$R_5$ is independently H, cyano, halogen, $C_{1-6}$ alkyl, $C_{1-6}$ haloalkyl, $-C_{0-6}$ alkylene $-OR_a$, $-C_{0-6}$ alkylene $-OC(O)N(R_a)_2$, $-C_{0-6}$ alkylene -N $(R_a)_2$, $-C_{0-6}$ alkylene $-NR_aC(O)R_a$, $-C_{0-6}$ alkylene - $NR_aC(O)N$ $(R_a)_2$, $-C_{0-6}$ alkylene $-NR_aS(O)R_a$, $-C_{0-6}$ alkylene $-NR_aS(O)_2R_a$, $-C_{0-6}$ alkylene -S $(=O)R_a$,$-C_{0-6}$ alkylene -S $(= O)_2R_a$, - $C_{0-6}$ alkylene $-SR_a$, - $C_{0-6}$ alkylene $-S(R_a)_5$, - $C_{0-6}$ alkylene $-C(=O)R_a$, - $C_{0-6}$ alkylene $-C(=O)OR_a$, - $C_{0-6}$ alkylene $-C(=O)N(R_a)_2$, $-C_{0-6}$ alkylene $-C_{3-14}$ cycloalkyl or $-C_{0-6}$ alkylene -(3-14 heterocyclic), the $C_{1-6}$ alkylene, $-C_{0-6}$ alkylene $-C_{3-14}$ cycloalkyl or $-C_{0-6}$ alkylene -(3-14 heterocyclic) is optionally substituted by one or more $R_a$;

ring A is $C_{6-14}$ aryl or 5-14 heteroaryl, and the $C_{6-14}$ aryl or 5-14 heteroaryl is optionally substituted by one or more $R_6$;

$R_6$ is H, cyano, halogen, $C_{1-6}$ alkyl, $C_{1-6}$ halogen alkyl, $-C_{0-6}$ alkylene-$OR_a$,$-C_{0-6}$ alkylene-$OC(O)N(R_a)_2$, $-C_{0-6}$ alkylene-$N(R_a)_2$, $-C_{0-6}$ alkylene-$NR_aC(O)R_a$,$-C_{0-6}$ alkylene-$NR_aC(O)N(R_a)_2$, - $C_{0-6}$ alkylene-$NR_aS(O)R_a$, $-C_{0-6}$ alkylene-$NR_aS(O)_2R_a$,$-C_{0-6}$ alkylene-$S(=O)R_a$, $-C_{0-6}$ alkylene$S(=O)_2R_a$, $-C_{0-6}$ alkylene-$SR_a$, $-C_{0-6}$ alkylene-$S(R_a)_5$, $-C_{0-6}$ alkylene-$C(=O)R_a$, $-C_{0-6}$ alkylene-$C(=O)OR_a$, $-C_{0-6}$ alkylene-$C(=O)N(R_a)_2$, $C_{2-6}$ alkenyl, $C_{2-6}$ alkynyl, $-C_{0-6}$ alkylene-$C_{3-14}$ cycloalkyl, - $C_{0-6}$ alkylene(3-14 heterocyclic), $-C_{0-6}$ alkylene-$C_{6-14}$ aryl or $-C_{0-6}$ alkylene(5-14 heteroaryl), the $C_{1-6}$ alkyl, $C_{2-6}$ alkenyl, $C_{2-6}$ alkynyl, $-C_{0-6}$ alkylene-$C_{3-14}$ cycloalkyl, $-C_{0-6}$

alkylene(3-14 heterocyclic), -$C_{0-6}$ alkylene-$C_{6-14}$ aryl or -$C_{0-6}$ alkylene(5-14 heteroaryl) is optionally substituted by one or more $R_a$;

each $R_a$ is independently H, halogen, hydroxyl, amino, oxo, nitro, cyano, carboxyl, $C_{1-6}$ alkyl, $C_{1-6}$ hydroxyalkyl, $C_{1-6}$ aminoalkyl, $C_{1-6}$ haloalkyl, $C_{1-6}$ alkoxy, $C_{1-6}$ haloalkoxy, $C_{1-6}$ heteroalkyl, $C_{3-8}$ cycloalkyl, 3-8 heterocycle, $C_{6-14}$ aryl, or 5-14 heteroaryl;

$R_2$ is H, halogen, cyano, $C_{1-3}$ alkyl, $C_{1-3}$ alkoxy, $C_{1-3}$ haloalkoxy, $C_{2-4}$ alkenyl or $C_{2-4}$ alkynyl;

$R_4$ is H, halogen, or methyl.

**3.** The compound, the tautomeric, deuterated compound or pharmaceutically acceptable salt thereof of claim 2, wherein; L is bond, ethylene or acetylene, the ethylene or acetylene is optionally substituted by one or more $R_5$; preferably L is bond, ethylene or acetylene, and preferably L is a bond.

**4.** The compound, the tautomeric, deuterated compound or pharmaceutically acceptable salt thereof of any one of claims 1-3, wherein;

$R_5$ is independently H, cyano, halogen, $C_{1-6}$ alkyl, $C_{1-6}$haloalkyl, -$OR_a$, -OC(O)N $(R_a)_2$, -N $(R_a)$ $_2$, -$NR_aC(O)R_a$, -$NR_aC(O)N$ $(R_a)_2$, -$NR_aS(O)R_a$, -$NR_aS(O)_2R_a$, -S (=O)Ra, -S (= O)$_2R_a$, - $SR_a$, -$S(R_a)_5$, -C(=O)$R_a$, -C(=O)O$R_a$, -C(=O)N$(R_a)_2$, -$C_{3-14}$ cycloalkyl or -(3-14) heterocyclic, each $R_a$ is independently H, $C_{1-6}$ alkyl, $C_{1-6}$ haloalkyl, $C_{1-6}$ heteroalkyl, $C_{3-8}$ cycloalkyl, 3-8 heterocyclic, $C_{6-14}$ aryl or 5-14 heteroaryl; preferably $R_5$ is independently H, halogen, cyano, amino, hydroxyl, $C_{1-6}$ alkyl, $C_{1-6}$ alkoxy, $C_{1-6}$ haloalkyl, $C_{1-6}$ haloalkoxy, -S-$C_{1-6}$ alkyl, $C_{3-8}$ cycloalkyl or 3-8 heterocyclic.

**5.** The compound, the tautomeric, deuterated compound or pharmaceutically acceptable salt thereof of any one of claims 1-4, wherein; ring A is

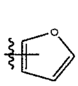, 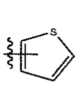, 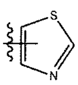, 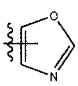, 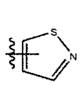, 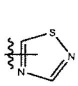, 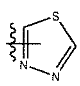, 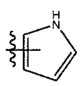, 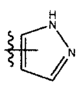, 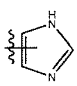, 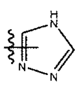,

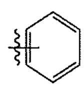, 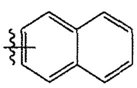, 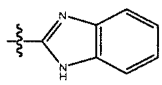, 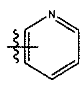, 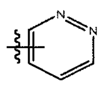, 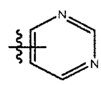 or 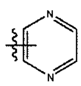,

ring A is optionally substituted by one or more $R_6$, preferably ring A is phenyl or pyridine.

**6.** The compound, the tautomeric, deuterated compound or pharmaceutically acceptable salt thereof of any one of claims 1-5, wherein;

$R_6$ is H, cyano, halogen, $C_{1-6}$ alkyl, $C_{1-6}$ halogen alkyl, -$OR_a$, -OC(O)N$(R_a)_2$, -N$(R_a)_2$, - $NR_aC(O)R_a$, -$NR_aC(O)N(R_a)_2$, -$NR_aS(O)R_a$, -$NR_aS(O)_2R_a$, -S(=O)$R_a$, -S(=O)$_2R_a$, -$SR_a$, -$S(R_a)_5$, - C(=O)$R_a$, -C(=O)O$R_a$, -C(=O)N$(R_a)_2$, $C_{2-6}$ alkenyl, $C_{2-6}$alkynyl, -$C_{3-14}$ cycloalkyl, 3-14 heterocyclic, -$C_{6-14}$ aryl or - 5-14 heteroaryl, each $R_a$ is independently H, $C_{1-6}$ alkyl, $C_{1-6}$ heteroalkyl, $C_{3-8}$ cycloalkyl, 3-8 heterocyclic, $C_{6-14}$ aryl or 5-14 heteraryl; preferably $R_6$ is H, halogen, cyano, $C_{1-3}$ alkyl, $C_{1-3}$ alkoxy, $C_{1-3}$ haloalkyl or -CHO, more preferably $R_6$ is H, fluorine, chlorine, cyano, methoxy or methyl.

**7.** The compound, the tautomeric, deuterated compound or pharmaceutically acceptable salt thereof of claim 1, having the structure:

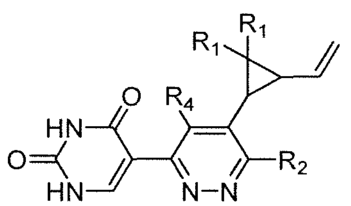

(IB)

or

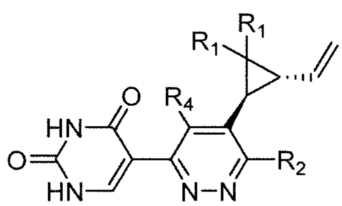

(IB-1)

wherein;

$R_1$, $R_2$ and $R_4$ are as defined in claim 1.

**8.** The compound, the tautomeric, deuterated compound or pharmaceutically acceptable salt thereof of any one of claims 1-7, wherein;
$R_2$ is H, halogen, cyano, $C_{1-3}$ alkyl, $C_{1-3}$ alkoxy or ethylene; preferably $R_2$ is H, chlorine, cyano, methyl or methoxy or ethylene; more preferably $R_2$ is chlorine, cyano, methyl or methoxy.

**9.** The compound, the tautomeric, deuterated compound or pharmaceutically acceptable salt thereof of any one of claims 1-8, wherein $R_4$ is H.

**10.** A compound, its tautomer, deuterated compound or pharmaceutically acceptable salt, wherein the compound is

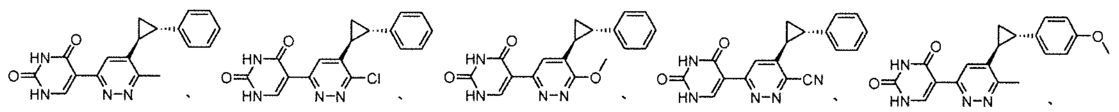
, , , , ,

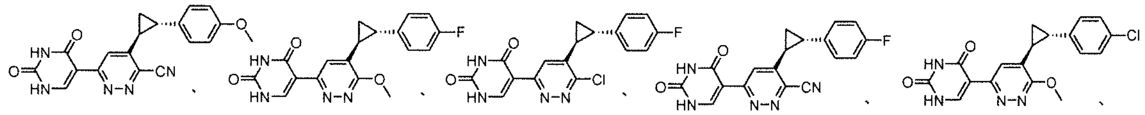
, , , , ,

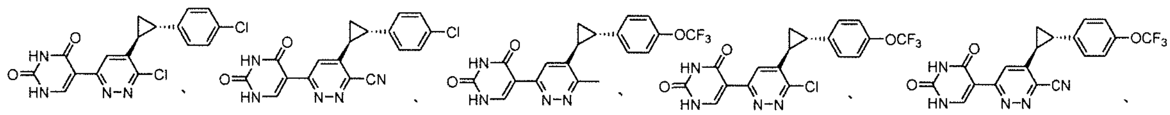
, , , , ,

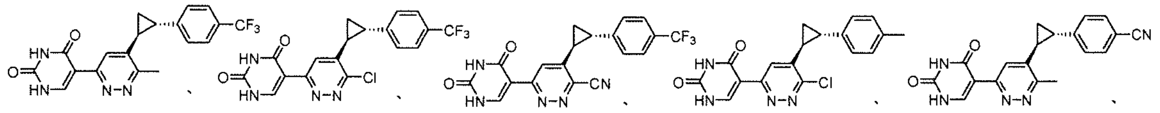
, , , , ,

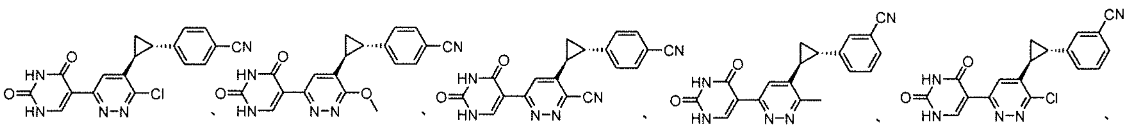
, , , , ,

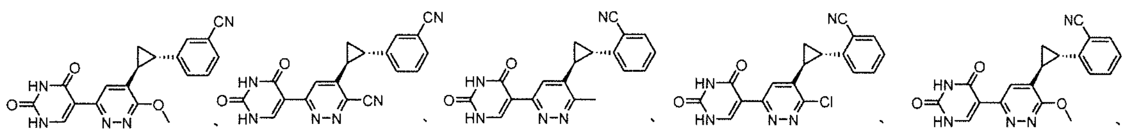
, , , , ,

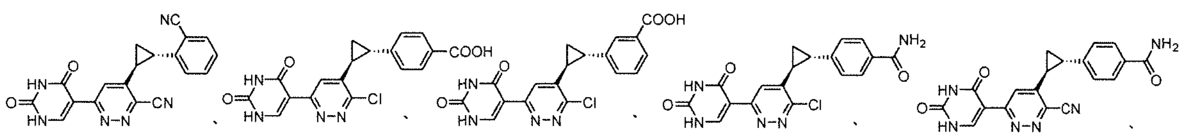
, , , , ,

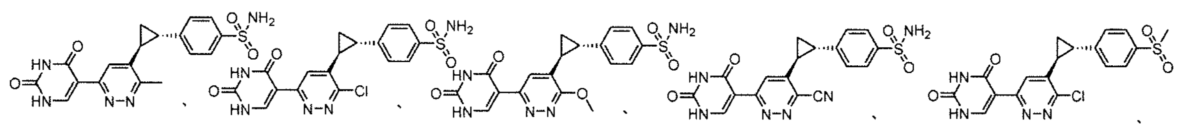
, , , , ,

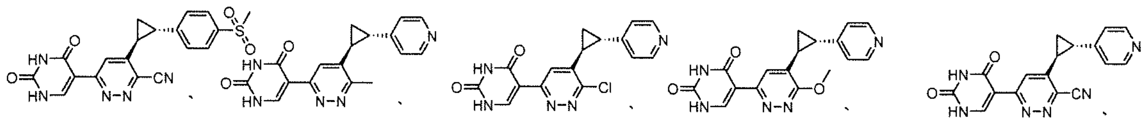
, , , , ,

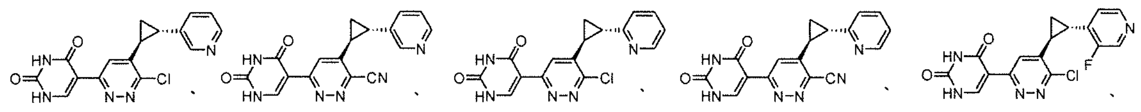
、 、 、 、 、

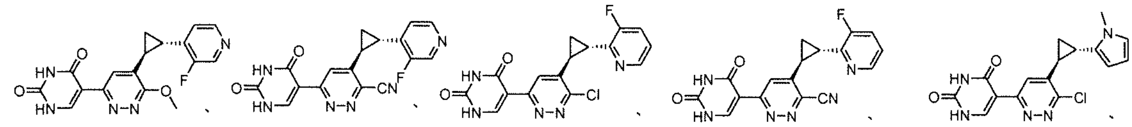
、 、 、 、 、

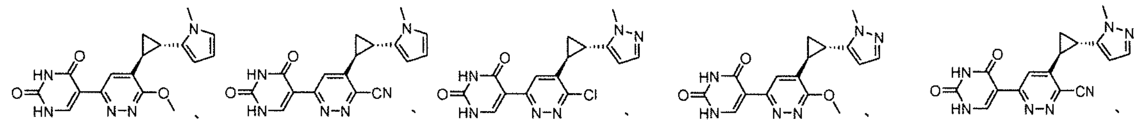
、 、 、 、 、

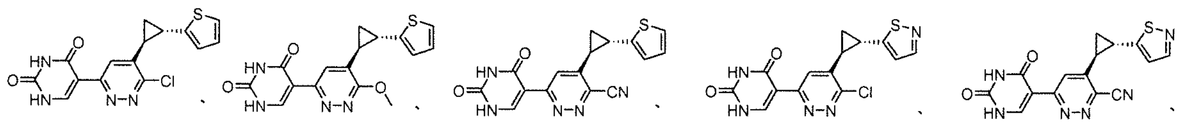
、 、 、 、 、

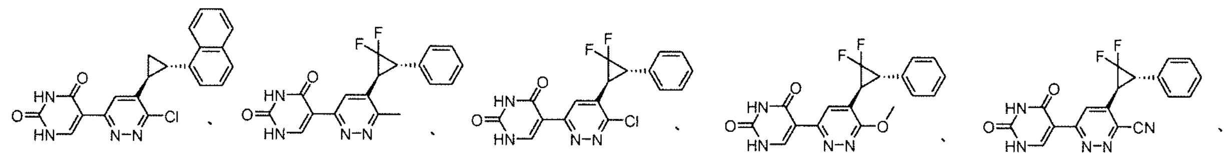
、 、 、 、 、

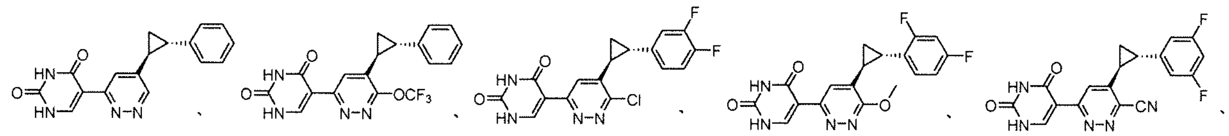
、 、 、 、 、

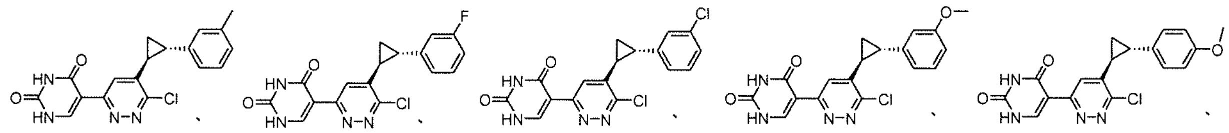
、 、 、 、 、

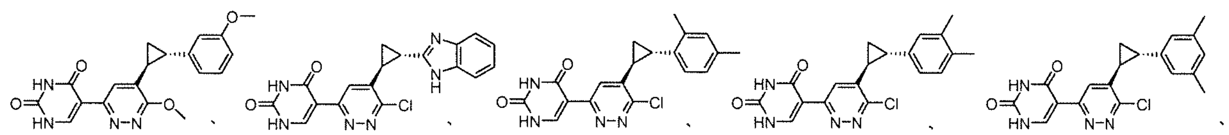
、 、 、 、 、

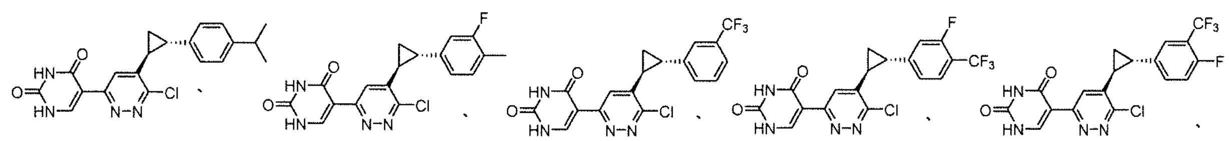
、 、 、 、 、

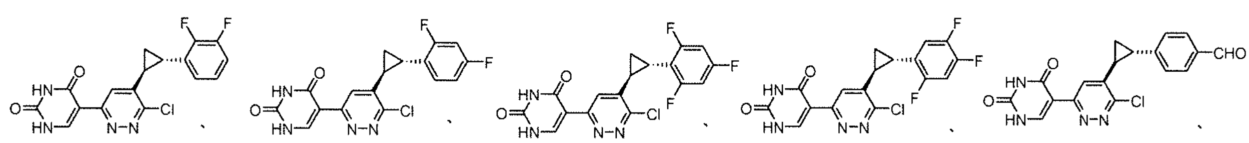
、 、 、 、 、

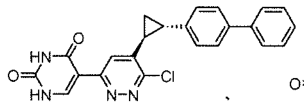、 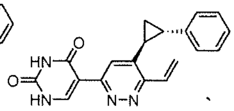、

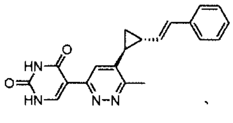、 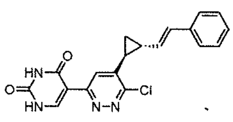、 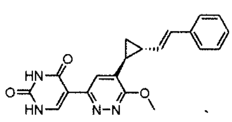、 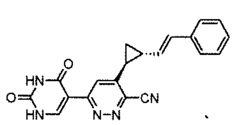、 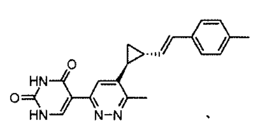、

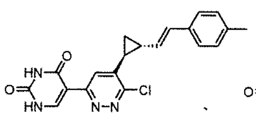、 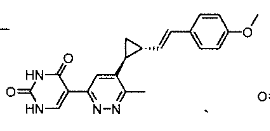、 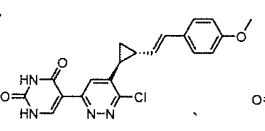、 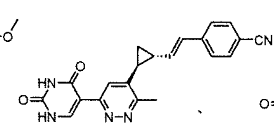、 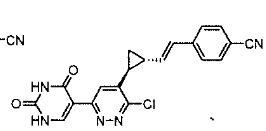、

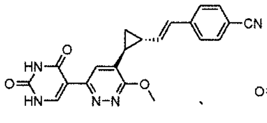、 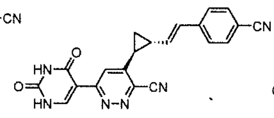、 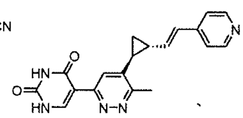、 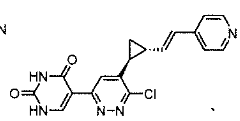、 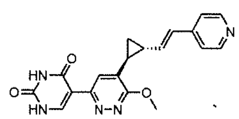、

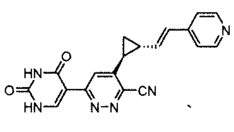、 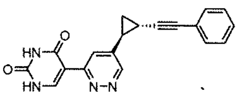、 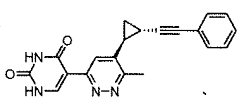、 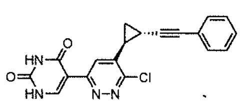、 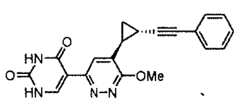、

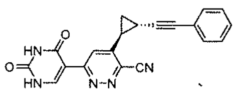、 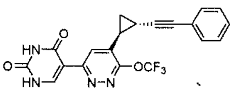、 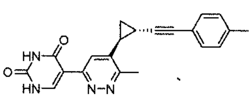、 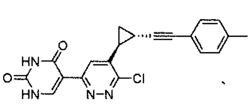、 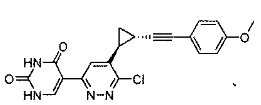、

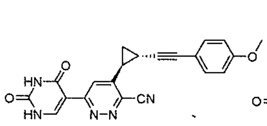、 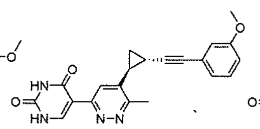、 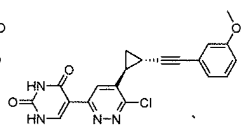、 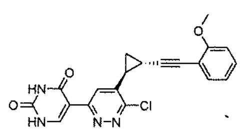、 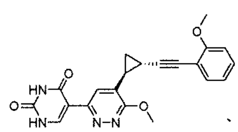、

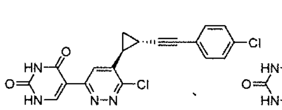、 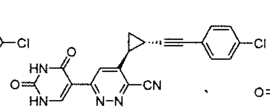、 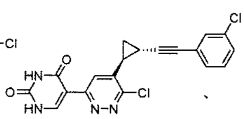、 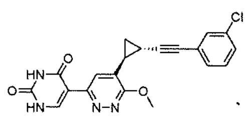、 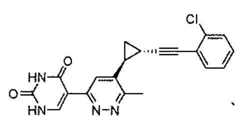、

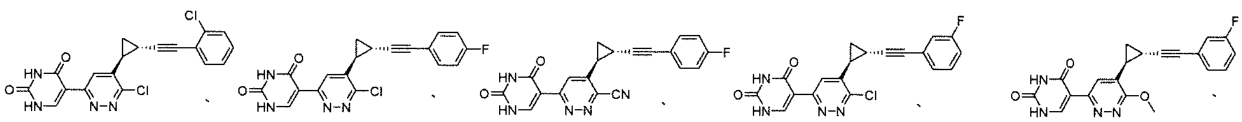、 、 、 、 、

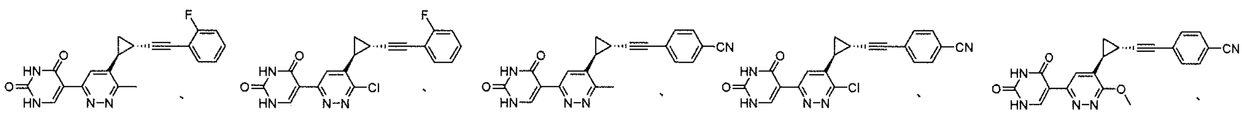、 、 、 、 、

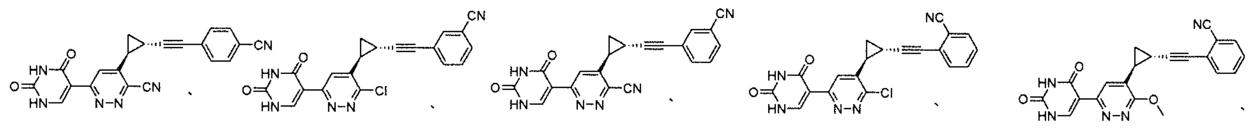

, , , ,

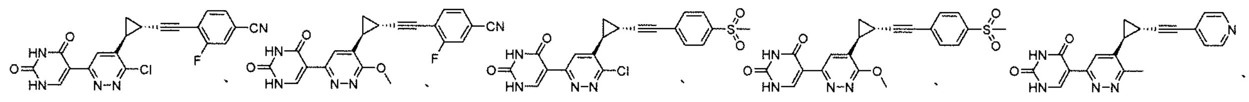

, , , ,

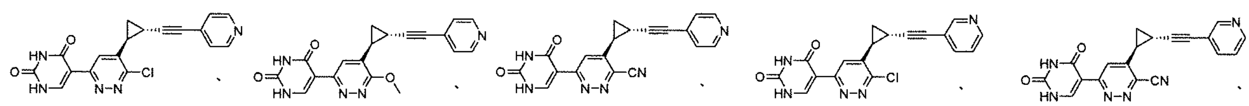

, , , ,

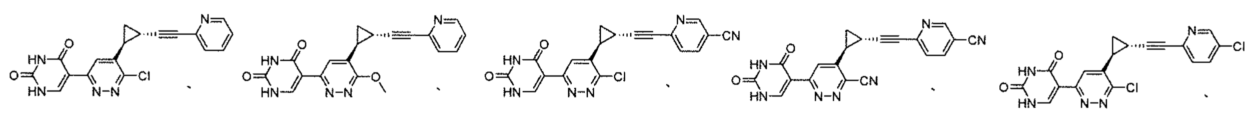

, , , ,

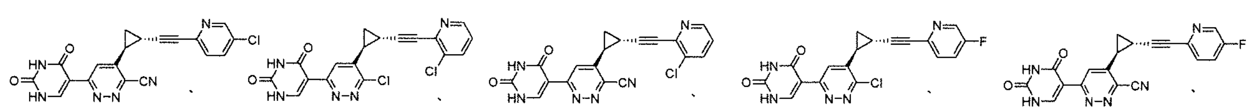

, , , ,

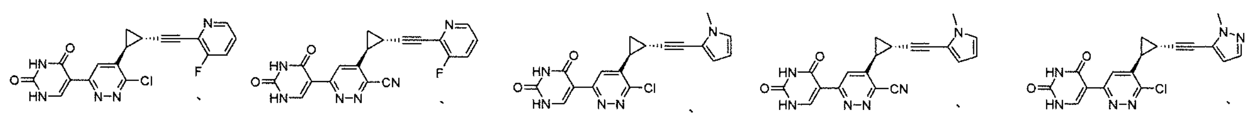

, , , ,

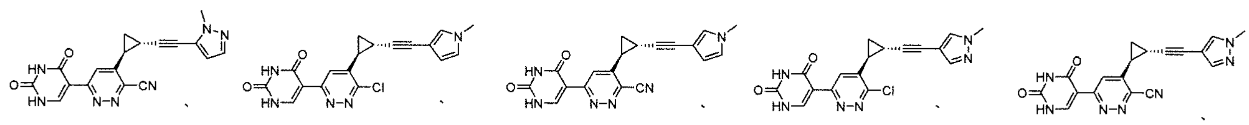

, , , ,

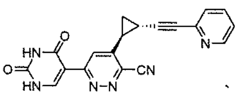

,

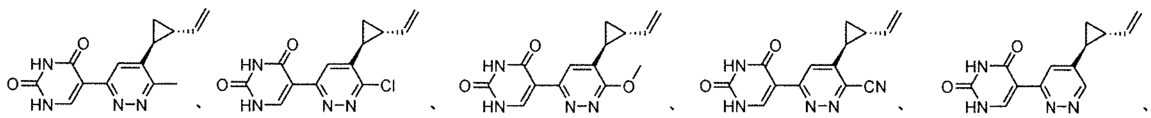

, , , ,

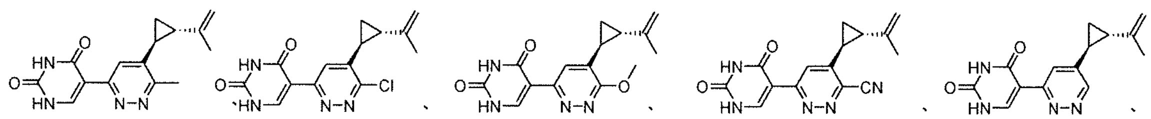

, , , ,

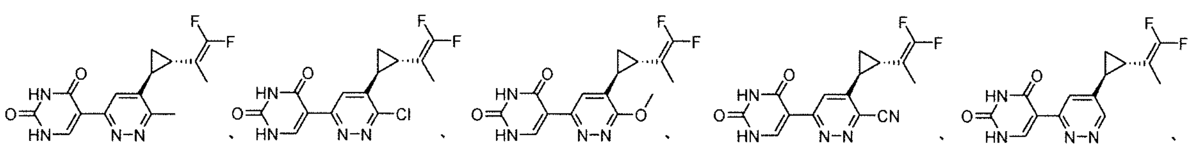

, , , ,

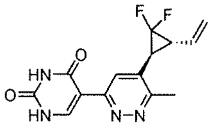 、 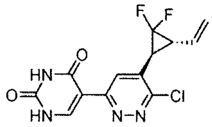 、 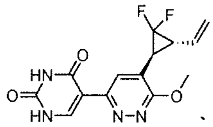 、 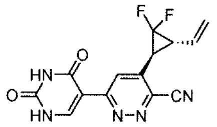 、 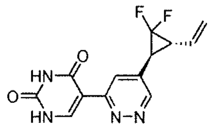 、

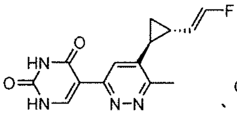 、 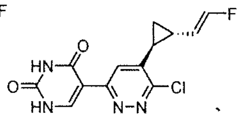 、 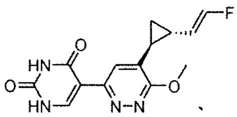 、 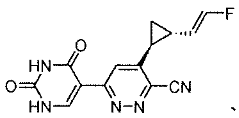 、 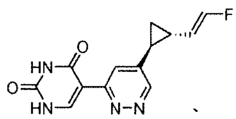 、

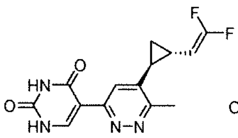 、 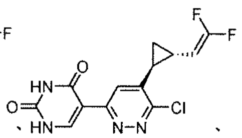 、 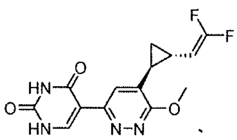 、 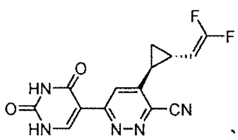 、 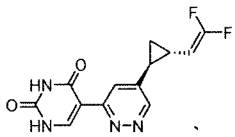 、

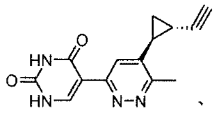 、 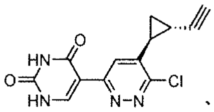 、 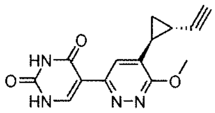 、 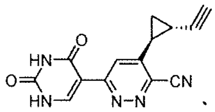 、 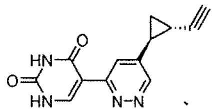 、

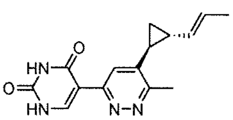 、 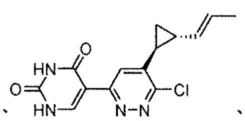 、 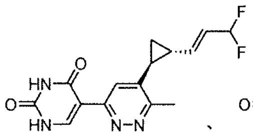 、 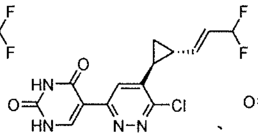 、 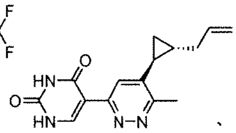 、

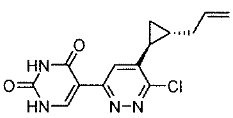 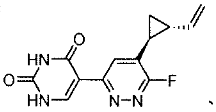 、 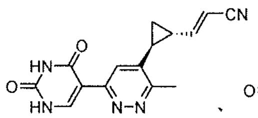 、 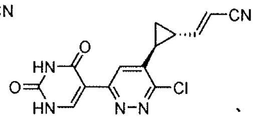 、 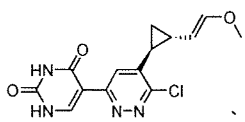 、

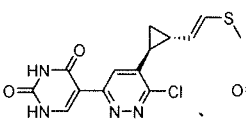 、 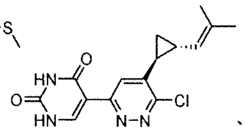 、 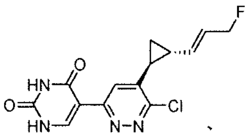 、 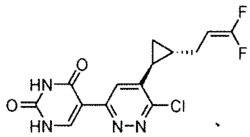 、 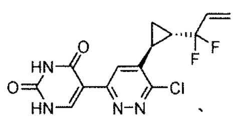 、

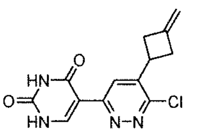 、 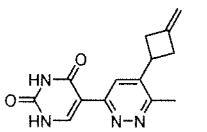 、 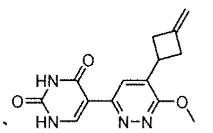 or 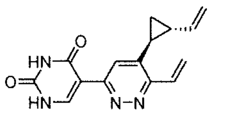 .

**11.** A pharmaceutical composition, wherein the pharmaceutical composition contains a therapeutically effective amount of the compound, the tautomeric, deuterated compound or pharmaceutically acceptable salt thereof of any one of claims 1-10 and at least one pharmaceutically acceptable excipient.

**12.** Use of the compound, the tautomeric, deuterated compound or pharmaceutically acceptable salt thereof of any one of claim 1-10 or the pharmaceutical compositions of claim 11 in the preparation of medicines; preferably in the preparation of medicines for treating cancer.

**13.** A method of treating and/or preventing a disease, comprising administering to a subject a therapeutically effective amount of the compound of any one of claims 1-10 or the pharmaceutical composition of claim 11.

**14.** A combination of drugs, the combination of drugs contains the compound, the tautomeric, deuterated compound or

pharmaceutically acceptable salt thereof of any one of claims 1-10, and anti-PD-L (1) antibodies.

**15.** A method for preparing a compound having formula (ID), its tautomeric, deuterated compound or pharmaceutically acceptable salt, which includes:

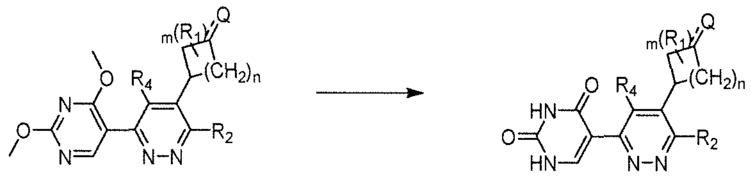

IC ID

obtaining the compound having formula (ID) by reacting a compound having formula (IC) under acidic conditions, wherein;

$R_1$-$R_2$, $R_4$, Q, m, n are as defined in claim 1.

**INTERNATIONAL SEARCH REPORT**

International application No.
**PCT/CN2021/116696**

**A. CLASSIFICATION OF SUBJECT MATTER**

C07D 403/04(2006.01)i； A61K 31/513(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D403/-， A61K31,-,A61P35/-

Documentation searched other than minimum documentation to the extent that such documents are included in the fields searched

Electronic data base consulted during the international search (name of data base and, where practicable, search terms used)

VEN, DWPI, CNKI, CNABS, STN(Reg, Caplus): 贝达, 吴颢 or 杨晓峰 or 刘奇声 or 韩晗 or 李金花 or 李扬 or 蒋枫 or 匡翠文 or 夏洪峰 or 张洪波 or 兰宏 or 王家炳 or 丁列明 or 苏艳桃, CD73, 核苷酸酶, 瘤, 癌, 嘧啶, 哒嗪, 环丙, 环丁, 环烷, nucleotidase, cancer, tumour, tumor, pyrimidin+, pyridazine? , structural formula search for claim 1

**C. DOCUMENTS CONSIDERED TO BE RELEVANT**

| Category* | Citation of document, with indication, where appropriate, of the relevant passages | Relevant to claim No. |
|---|---|---|
| X | WO 2019168744 A1 (LILLY CO ELI) 06 September 2019 (2019-09-06)<br>embodiments and claims | 1-12,14-15 |
| PX | WO 2021041319 A1 (LILLY CO ELI) 04 March 2021 (2021-03-04)<br>claims, embodiments | 1-12,14-15 |
| A | WO 2017120508 A8 (ARCUS BIOSCIENCES INC.) 05 July 2018 (2018-07-05)<br>entire document | 1-12,14-15 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **21 December 2021** | **30 December 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/116696**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13**
because they relate to subject matter not required to be searched by this Authority, namely:

[1] Claim 13 relates to a method for treating a disease, which belongs to methods for treating the human or animal body (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/116696**

| Patent document cited in search report | Publication date (day/month/year) | Patent family member(s) | Publication date (day/month/year) |
|---|---|---|---|
| WO 2019168744 A1 | 06 September 2019 | IL 277006 D0 | 29 October 2020 |
| | | US 11028074 B2 | 08 June 2021 |
| | | SG 11202008366 R A | 29 September 2020 |
| | | JO P20200209 A1 | 01 September 2019 |
| | | AU 2019228473 B2 | 25 February 2021 |
| | | KR 20200116965 A | 13 October 2020 |
| | | US 2021002257 A1 | 07 January 2021 |
| | | JP 2020517655 A | 18 June 2020 |
| | | CL 2020002158 A1 | 13 November 2020 |
| | | CO 2020010191 A2 | 31 August 2020 |
| | | CN 111819173 A | 23 October 2020 |
| | | TW 201945002 A | 01 December 2019 |
| | | PE 20210177 A1 | 29 January 2021 |
| | | CA 3092661 A1 | 06 September 2019 |
| | | EC SP20052897 A | 30 September 2020 |
| | | AU 2019228473 A1 | 20 August 2020 |
| | | EP 3759096 A1 | 06 January 2021 |
| | | TW I702954 B | 01 September 2020 |
| | | UA 123890 C2 | 16 June 2021 |
| | | CR 20200376 A | 23 October 2020 |
| | | BR 112020016066 A2 | 08 December 2020 |
| | | JP 6794560 B2 | 02 December 2020 |
| WO 2021041319 A1 | 04 March 2021 | None | |
| WO 2017120508 A8 | 05 July 2018 | CN 108697719 A | 23 October 2018 |
| | | BR 112018013827 A2 | 11 December 2018 |
| | | US 10239912 B2 | 26 March 2019 |
| | | MX 2018008350 A | 30 May 2019 |
| | | CL 2018001845 A1 | 07 December 2018 |
| | | PH 12018501361 A1 | 27 February 2019 |
| | | TW 201805006 A | 16 February 2018 |
| | | SG 11201805506 Y A | 30 July 2018 |
| | | US 11001603 B2 | 11 May 2021 |
| | | AU 2017206061 A8 | 26 July 2018 |
| | | WO 2017120508 A1 | 13 July 2017 |
| | | EP 3399984 A4 | 01 January 2020 |
| | | KR 20180100638 A | 11 September 2018 |
| | | US 10981944 B2 | 20 April 2021 |
| | | JP 2019501223 A | 17 January 2019 |
| | | US 2021002322 A1 | 07 January 2021 |
| | | US 2017267710 A1 | 21 September 2017 |
| | | US 2019309010 A1 | 10 October 2019 |
| | | EP 3399984 A1 | 14 November 2018 |
| | | CA 3009196 A1 | 13 July 2017 |
| | | AU 2017206061 A1 | 19 July 2018 |
| | | IL 260260 D0 | 31 July 2018 |
| | | EA 201891583 A1 | 31 January 2019 |

Form PCT/ISA/210 (patent family annex) (January 2015)